# EUROPEAN PATENT APPLICATION

(11) **EP 0 863 142 A1**
(43) Date of publication of application: **09.09.1998**
(21) Application number: 98301307.9
(22) Date of filing: 23.02.1998
(51) Int. Cl.: C07D 417/04, A01N 43/80, C07D 249/12, C07D 413/04

(54) **Heterocyclic herbicides**

(30) Priority: 05.03.1997 US 39872 P
(71) Applicant: E.I. DU PONT DE NEMOURS AND COMPANY, Wilmington Delaware 19898 (US)
(72) Inventor: Kamireddy, Balreddy, Hockessin, Delaware 19707 (US)
(74) Representative: Woodman, Derek

(57) **Abstract**

Compounds of Formula I, and their *N*-oxides and agriculturally suitable salts, are disclosed which are useful for controlling undesired vegetation 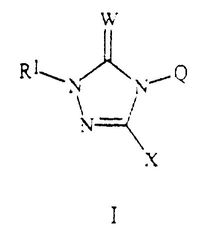 wherein
Q is 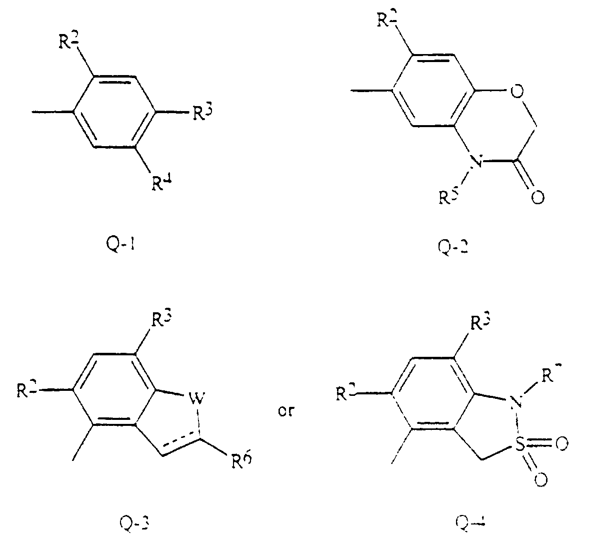 and W,X,R¹-R⁷ are as defined in the disclosure.

Also disclosed are compositions containing the compounds of Formula I and a method for controlling undesired vegetation which involves contacting the vegetation or its environment with an effective amount of a compound of Formula I.

## Description

### BACKGROUND OF THE INVENTION

This invention relates to certain triazolinones, their *N*-oxides, agriculturally suitable salts and compositions, and methods of their use for controlling undesirable vegetation.

The control of undesired vegetation is extremely important in achieving high crop efficiency. Achievement of selective control of the growth of weeds especially in such useful crops as rice, soybean, sugar beet, corn (maize), potato, wheat, barley, tomato and plantation crops, among others, is very desirable. Unchecked weed growth in such useful crops can cause significant reduction in productivity and thereby result in increased costs to the consumer. The control of undesired vegetation in noncrop areas is also important. Many products are commercially available for these purposes, but the need continues for new compounds which are more effective, less costly, less toxic, environmentally safer or have different modes of action.

United States patent 5,108,486 discloses compounds of Formula i as herbicides: 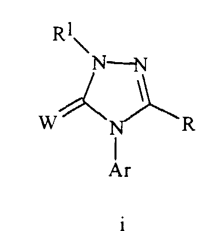 wherein, *inter alia,*
W is O or S;
R¹ is alkyl;
R is haloalkyl; and
Ar is substituted phenyl or pyridyl.

The triazinones of the present invention are not disclosed in this publication.

### SUMMARY OF THE INVENTION

This invention is directed to compounds of Formula I including all geometric and stereoisomers, *N*-oxides, and agriculturally suitable salts thereof, agricultural compositions containing them and their use for controlling undesirable vegetation: 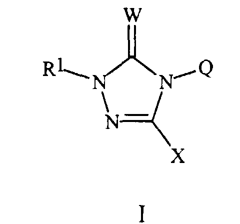 wherein
- Q is: 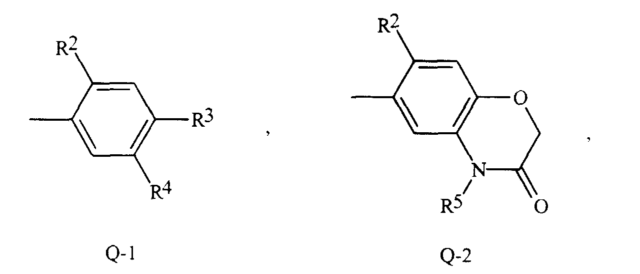 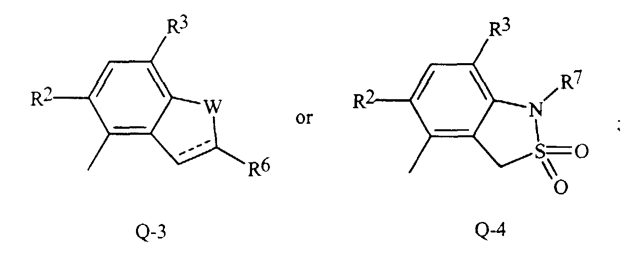
- each W is: independently O or S;
- X is: halogen, OR⁸, SR⁸ or S(O)ₙR⁹;
- R¹ is: C₁-C₄ alkyl, C₁-C₄ haloalkyl, C₂-C₄ alkoxyalkyl, C₃-C₄ alkenyl, C₃-C₄ haloalkenyl, C₃-C₄ alkynyl, CH₂CHO or CH₂CO₂R¹⁰;
- R² is: H or halogen;
- R³ is: C₁-C₂ alkyl, C₁-C₂ haloalkyl, OCH₃, SCH₃, OCHF₂, halogen, cyano or nitro;
- R⁴ is: OR¹¹, SR¹¹, CO₂R¹⁰, CH₂CO₂R¹⁰, WCH₂CO₂R^{10a}, OCH₂CN, CH₂CHCICO₂R¹⁰ or NR¹²SO₂R¹³;
- R⁵ is: C₁-C₄ alkyl, C₁-C₄ haloalkyl, C₂-C₄ cyanoalkyl, C₃-C₄ alkenyl, C₃-C₄ haloalkenyl, C₃-C₄ alkynyl, S(O)₂R⁹, CO₂R⁸ or C(=O)R⁸;
- R⁶ is: H, C₁-C₂ alkyl, C₁-C₂ haloalkyl, CO₂R¹⁰, CH₂OR¹⁰ or C(=O)R⁸;
- R⁷ is: H, C₁-C₄ alkyl, C₁-C₄ haloalkyl, C₃-C₄ alkenyl, C₃-C₄ haloalkenyl, C₃-C₄ alkynyl, S(O)₂R⁹, CO₂R⁸, CH₂CO₂R¹⁰, C(=O)R⁸ or CH₂C(=O)R⁸; or R⁷ is benzyl optionally substituted on the phenyl ring with C₁-C₂ alkyl, C₁-C₂ haloalkyl, C₁-C₂ alkoxy, halogen, nitro or cyano;
- each R⁸ is: independently C₁-C₄ alkyl, C₁-C₄ haloalkyl, C₃-C₄ alkenyl, C₃-C₄ haloalkenyl or C₃-C₄ alkynyl;
- each R⁹ is: independently C₁-C₄ alkyl or C₁-C₄ haloalkyl;
- each R¹⁰ is: independently H, C₁-C₄ alkyl, C₁-C₄ haloalkyl, C₃-C₄ alkenyl, C₃-C₄ haloalkenyl or C₃-C₄ alkynyl;
- R^{10a} is: H, C₁-C₄ alkyl, C₁-C₄ haloalkyl, C₃-C₄ alkenyl, C₃-C₄ haloalkenyl, C₃-C₄ alkynyl or 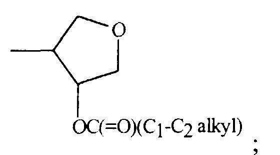
- R¹¹ is: C₁-C₄ alkyl, C₁-C₄ haloalkyl, C₃-C₇ cycloalkyl, C₃-C₇ halocycloalkyl, C₂-C₆ alkoxyalkyl, C₃-C₄ alkenyl, C₃-C₄ haloalkenyl or C₃-C₄ alkynyl;
- R¹² is: H, C₁-C₄ alkyl, C₁-C₄ haloalkyl, C₃-C₆ cycloalkyl, C₃-C₆ halocycloalkyl, C₃-C₄ alkenyl, C₃-C₄ haloalkenyl, C₃-C₄ alkynyl, C₂-C₆ alkoxyalkyl, C₂-C₆ haloalkoxyalkyl, CO₂R⁸, C(=O)R⁸ or S(O)₂R⁹;
- R¹³ is: C₁-C₄ alkyl, C₁-C₄ haloalkyl, C₁-C₄ alkoxy, C₁-C₄ haloalkoxy, C₂-C₆ alkoxyalkyl, C₂-C₄ cyanoalkyl, C₂-C₆ alkenyl, C₂-C₆ haloalkenyl or C₂-C₆ alkynyl; and
- n is: 1 or 2; and
wherein the dashed line in Q-3 indicates that the bond may be a carbon-carbon single bond or a carbon-carbon double bond.

In the above recitations, the term "alkyl", used either alone or in compound words such as "alkylthio" or "haloalkyl" includes straight-chain or branched alkyl, such as, methyl, ethyl, *n*-propyl, i-propyl, or the different butyl, pentyl or hexyl isomers. "Alkenyl" includes straight-chain or branched alkenes such as ethenyl, 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain or branched alkynes such as ethynyl, 1-propynyl, 2-propynyl and the different butynyl, pentynyl and hexynyl isomers. "Alkynyl" can also include moieties comprised of multiple triple bonds such as 2,5-hexadiynyl. "Alkoxy" includes, for example, methoxy, ethoxy, n-propyloxy, isopropyloxy and the different butoxy, pentoxy and hexyloxy isomers. "Alkoxyalkyl" denotes alkoxy substitution on alkyl. Examples of "alkoxyalkyl" include CH₃OCH₂, CH₃OCH₂CH₂, CH₃CH₂OCH₂, CH₃CH₂CH₂CH₂OCH₂ and CH₃CH₂OCH₂CH₂. "Cyanoalkyl" denotes an alkyl group substituted with one cyano group. Examples of "cyanoalkyl" include NCCH₂, NCCH₂CH₂ and CH₃CH(CN)CH₂. "Alkylamino", "dialkylamino", "alkenylthio", "alkenylsulfinyl", "alkenylsulfonyl", "alkynylthio", "alkynylsulfinyl", "alkynylsulfonyl", and the like, are defined analogously to the above examples. "Cycloalkyl" includes, for example, cyclopropyl, cyclobutyl, cyclopentyl, and cyclohexyl.

One skilled in the art will appreciate that not all nitrogen containing heterocycles can form *N*-oxides since the nitrogen requires an available lone pair for oxidation to the oxide; one skilled in the art will recognize those nitrogen containing heterocycles which can form *N*-oxides. One skilled in the art will also recognize that tertiary amines can form *N*-oxides. Synthetic methods for the preparation of *N*-oxides of heterocycles and tertiary amines are very well known by one skilled in the art including the oxidation of heterocycles and tertiary amines with peroxy acids such as peracetic and *m*-chloroperbenzoic acid (MCPBA), hydrogen peroxide, alkyl hydroperoxides such as *t*-butyl hydroperoxide, sodium perborate, and dioxiranes such as dimethyldioxirane. These methods for the preparation of *N*-oxides have been extensively described and reviewed in the literature, see for example: T.L. Gilchrist in *Comprehensive Organic Synthesis,* vol. 7, pp 748-750, S. V. Ley, Ed., Pergamon Press; M. Tisler and B. Stanovnik in *Comprehensive Heterocyclic Chemistry,* vol. 3, pp 18-20, A. J. Boulton and A. McKillop, Eds., Pergamon Press; M. R. Grimmett and B. R. T. Keene in *Advances in Heterocyclic Chemistry,* vol. 43, pp 149-161, A. R. Katritzky, Ed., Academic Press; M. Tisler and B. Stanovnik in *Advances in Heterocyclic Chemistry,* vol. 9, pp 285-291, A. R. Katritzky and A. J. Boulton, Eds., Academic Press; and G. W. H. Cheeseman and E. S. G. Werstiuk in *Advances in Heterocyclic Chemistry,* vol. 22, pp 390-392, A. R. Katritzky and A. J. Boulton, Eds., Academic Press.

The term "halogen", either alone or in compound words such as "haloalkyl", includes fluorine, chlorine, bromine or iodine. The term "1-2 halogen" indicates that one or two of the available positions for that substituent may be halogen which are independently selected. Further, when used in compound words such as "haloalkyl", said alkyl may be partially or fully substituted with halogen atoms which may be the same or different. Examples of "haloalkyl" include F₃C, CICH₂, CF₃CH₂ and CF₃CCI₂. The terms "haloalkenyl", "haloalkynyl", "haloalkoxy", "haloalkylthio", and the like, are defined analogously to the term "haloalkyl". Examples of "haloalkenyl" include (Cl)₂C=CHCH₂ and CF₃CH₂CH=CHCH₂. Examples of "haloalkynyl" include HC≡CCHCl, CF₃C≡C, CCI₃C≡C and FCH₂C≡CCH₂. Examples of "haloalkoxy" include CF₃O, CCI₃CH₂O, HCF₂CH₂CH₂O and CF₃CH₂O.

The total number of carbon atoms in a substituent group is indicated by the "Cᵢ-Cⱼ" prefix where i and j are numbers from 1 to 6. For example, C₁-C₃ alkylsulfonyl designates methylsulfonyl through propylsulfonyl; C₂ alkoxyalkyl designates CH₃OCH₂; C₃ alkoxyalkyl designates, for example, CH₃CH(OCH₃), CH₃OCH₂CH₂ or CH₃CH₂OCH₂; and C₄ alkoxyalkyl designates the various isomers of an alkyl group substituted with an alkoxy group containing a total of four carbon atoms, examples including CH₃CH₂CH₂OCH₂ and CH₃CH₂OCH₂CH₂. Examples of "alkylcarbonyl" include C(O)CH₃, C(O)CH₂CH₂CH₃ and C(O)CH(CH₃)₂. Examples of"alkoxycarbonyl" include CH₃OC(=O), CH₃CH₂OC(=O), CH₃CH₂CH₂OC(=O), (CH₃)₂CHOC(=O) and the different butoxy- or pentoxycarbonyl isomers. In the above recitations, when a compound of Formula I is comprised of one or more heterocyclic rings, all substituents are attached to these rings through any available carbon or nitrogen by replacement of a hydrogen on said carbon or nitrogen.

When a compound is substituted with a substituent bearing a subscript that indicates the number of said substituents can exceed 1, said substituents (when they exceed 1) are independently selected from the group of defined substituents. Further, when the subscript indicates a range, e.g. (R)ᵢ₋ⱼ, then the number of substituents may be selected from the integers between i and j inclusive.

When a group contains a substituent which can be hydrogen, for example R⁷, then, when this substituent is taken as hydrogen, it is recognized that this is equivalent to said group being unsubstituted.

Compounds of this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers. Accordingly, the present invention comprises compounds selected from Formula I, *N*-oxides and agriculturally suitable salts thereof. The compounds of the invention may be present as a mixture of stereoisomers, individual stereoisomers, or as an optically active form.

The salts of the compounds of the invention include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. The salts of the compounds of the invention also include those formed with organic bases (e.g., pyridine, ammonia, or triethylamine) or inorganic bases (e.g., hydrides, hydroxides, or carbonates of sodium, potassium, lithium, calcium, magnesium or barium) when the compound contains an acidic group such as a carboxylic acid or phenol.

Preferred compounds for reasons of better activity and/or ease of synthesis are:
Preferred 1. Compounds of Formula I above, and *N*-oxides and agriculturally-suitable salts thereof, wherein:
   Q is Q-1.
Preferred 2. Compounds of Preferred 1 wherein:
   R¹ is C₁-C₄ alkyl or C₁-C₄ haloalkyl;
   X is F, Cl, Br, OR⁸ or SR⁸; and
   R⁸ is C₁-C₂ alkyl or C₁-C₂ haloalkyl.
Preferred 3. Compounds of Preferred 2 wherein:
   R² is F or Cl;
   R³ is halogen, OCH₃ or cyano;
   R⁴ is OR¹¹, NR¹²SO₂R¹³ or CO₂R¹⁰;
   R⁸ is C₁-C₂ alkyl;
   R¹⁰ is C₁-C₃ alkyl or C₁-C₃ haloalkyl;
   R¹¹ is C₁-C₃ alkyl, C₃-C₄ alkenyl or C₃-C₄ alkynyl;
   R¹² is H, CO₂R⁸ or C(=O)R⁸; and
   R¹³ is C₁-C₃ alkyl or C₁-C₃ haloalkyl.
Preferred 4. Compounds of Formula I above, and *N*-oxides and agriculturally-suitable salts thereof, wherein:
   Q is Q-2.
Preferred 5. Compounds of Preferred 4 wherein:
   R¹¹ is C₁-C₄ alkyl or C₁-C₄ haloalkyl;
   X is F, Cl, Br, OR⁸ or SR⁸; and
   R⁸ is C₁-C₂ alkyl or C₁-C₂ haloalkyl.
Preferred 6. Compounds of Preferred 5 wherein:
   R² is F or Cl; and
   R⁵ is C₁-C₂ alkyl or C₃-C₄ alkynyl.
Preferred 7. Compounds of Formula I above, and *N*-oxides and agriculturally-suitable salts thereof, wherein:
   Q is Q-3.
Preferred 8. Compounds of Preferred 7 wherein:
   R¹ is C₁-C₄ alkyl or C₁-C₄ haloalkyl;
   X is F, Cl, Br, OR⁸ or SR⁸; and
   R⁸ is C₁-C₂ alkyl or C₁-C₂ haloalkyl.
Preferred 9. Compounds of Preferred 8 wherein:
   R² is F or Cl;
   R³ is halogen, OCH₃ or cyano;
   W is O; and
   R⁶ is C₁-C₂ alkyl or C₁-C₂ haloalkyl.
Preferred 10. Compounds of Formula I above, and *N*-oxides and agriculturally-suitable salts thereof, wherein:
   Q is Q-4.
Preferred 11. Compounds of Preferred 10 wherein:
   R¹¹ is C₁-C₄ alkyl or C₁-C₄ haloalkyl;
   X is F, Cl, Br, OR⁸ or SR⁸; and
   R⁸ is C₁-C₃ alkyl or C₁-C₂ haloalkyl.
Preferred 12. Compounds of Preferred 11 wherein:
   R² is F or Cl;
   R³ is halogen, OCH₃ or cyano;
   R⁷ is H, C₁-C₃ alkyl, C₃-C₄ alkynyl, CO₂R¹⁰ or C(=O)R⁸;
   R⁸ is C₁-C₃ alkyl; and
   R¹⁰ is C₁-C₂ alkyl.
Most preferred are compounds of Formula I above selected from the group: 1-acetyl-7-chloro-4-(3-chloro- 1,5-dihydro-1-methyl-5-oxo-4*H*-1,2,4-triazol-4-yl)-5-fluoro-1,3-dihydro-2,1-benzisothiazole 2,2-dioxide; and
1-chloro-*N*-[2-chloro-5-(3-chloro-1,5-dihydro-1-methyl-5-oxo-4*H*-1,2,4-triazol-4-yl)-4-fluorophenyl]methanesulfonamide.

### DETAILS OF THE INVENTION

The compounds of Formula I can be prepared by one or more of the following methods and variations as described in Schemes 1-10. The definitions of X, W, R¹-R¹³, and n in the compounds of Formulae 1-33 below are as defined above in the Summary of the Invention. Compounds of Formulae 1a-1g are various subsets of the compounds of Formula 1, and all substituents for Formulae 1a-1g are as defined for Formula 1.

Compounds of Formula I are prepared from the corresponding anilines as shown in Scheme 1. The anilines of Formula 1 are first converted to isocyanates of Formula 2 by reaction with phosgene or a phosgene equivalent. Isocyanates of Formula 2 are condensed with appropriate hydrazines to give compounds of Formula 3, which on subsequently heating with either phosgene or phosgene followed by POCl₃ afford the heterocycles of Formula I wherein X is Cl. The compounds of Formula I wherein X is Cl can be further converted to compounds of Formula I wherein X is OR⁸ or SR⁸ by reaction with appropriate alkoxides or thioakloxides. The thioalkoxides of Formula I can be further oxidized to sulfoxides and sulfones by methods well known in the art.

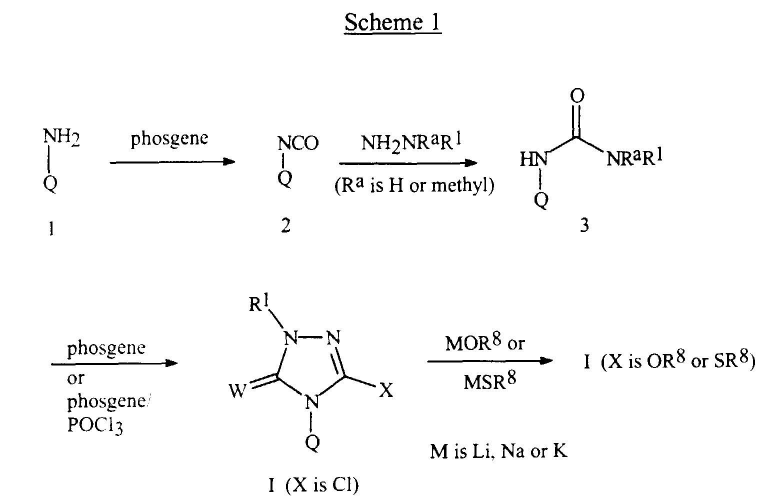

Anilines of Formula la (corresponding to compounds of Formula 1 wherein Q is Q-1 and R⁴ is NR¹²SO₂R¹³) are prepared by the method illustrated in Scheme 2. Nitration of the acetanilide of Formula 4 using a nitric acid/sulfuric acid mixture affords the nitro compound of Formula 5. Reduction of the nitro group to the aniline of Formula 6 can be achieved by catalytic hydrogenation over a metal catalyst such as palladium, iridium or Raney® nickel. The preferred catalyst is 5% iridium adsorbed onto charcoal. The aniline of Formula 6 is contacted with a sulfonyl chloride to give the corresponding sulfonamide which on hydrolysis with 6*N* HCI in ethanol provides the compound of Formula 7. For compounds wherein R¹² is other than H, the sulfonamide nitrogen can be alkylated, acylated or sulfonylated to give the R^{12a}-substituted compounds of Formula 1a. The alkylation is performed using an alkyl halide or alkyl sulfonate in the presence of a base such as potassium carbonate, sodium methoxide, potassium *t*-butoxide (*t*-BuOK) or sodium hydride in an anhydrous solvent such as dimethylformamide (DMF), tetrahydrofuran or acetonitrile at ambient temperature to 80 °C. Acylations to form the carbonyl-substituted sulfonamides are accomplished by condensing the sulfonamide of Formula 7 with the appropriate acylating agent, for example an acyl chloride, isocyanate or carbamoyl chloride. Sulfonylations to form the sulfonyl-substituted sulfonamides are accomplished in an analogous manner by reacting the sulfonamide of Formula 7 with the appropriate sulfonylating agent, for example a sulfonyl chloride.

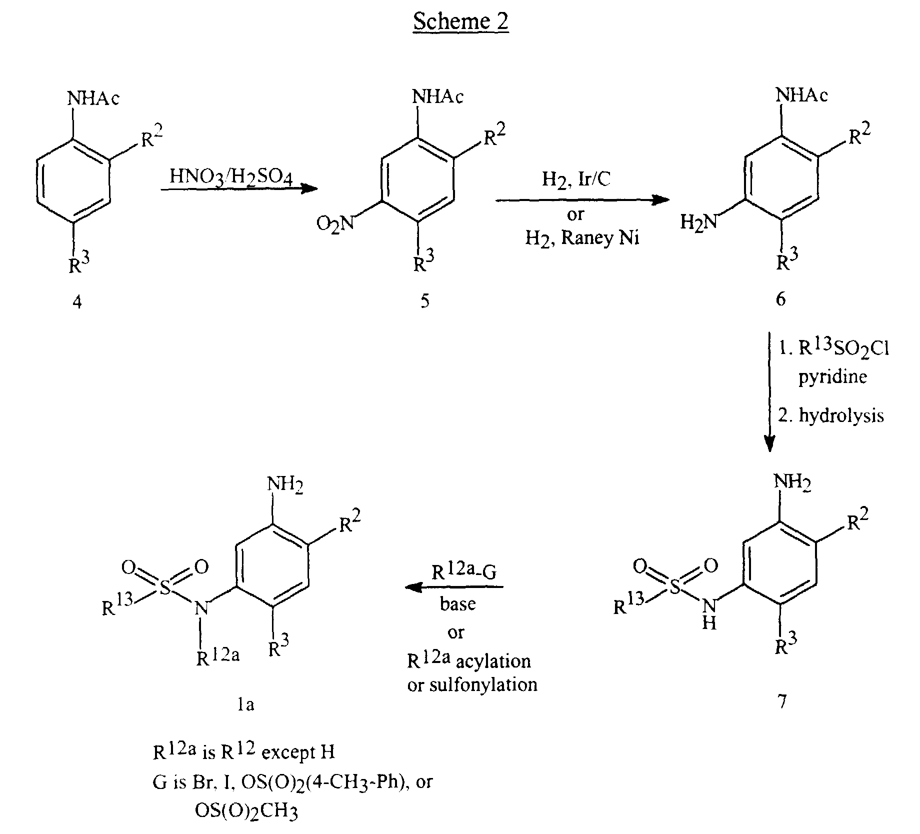

Some anilines of Formula 1a can also be prepared by the method illustrated in Scheme 3. The nitro aniline of Formula 8 is sulfonylated to afford the compound of Formula 9. Further alkylation, acylation or sulfonylation gives the nitro compound of Formula 10. Reduction of the nitro group to the aniline of Formula 11 can be achieved by iron in acetic acid or by catalytic hydrogenation over a metal catalyst such as palladium or iridium. The preferred method is by hydrogenation over 5% iridium adsorbed onto charcoal. Chlorination of the phenyl ring provides the compound of Formula la wherein R^{12a} is R¹² as defined in the Summary of the Invention except H and R³ is Cl.

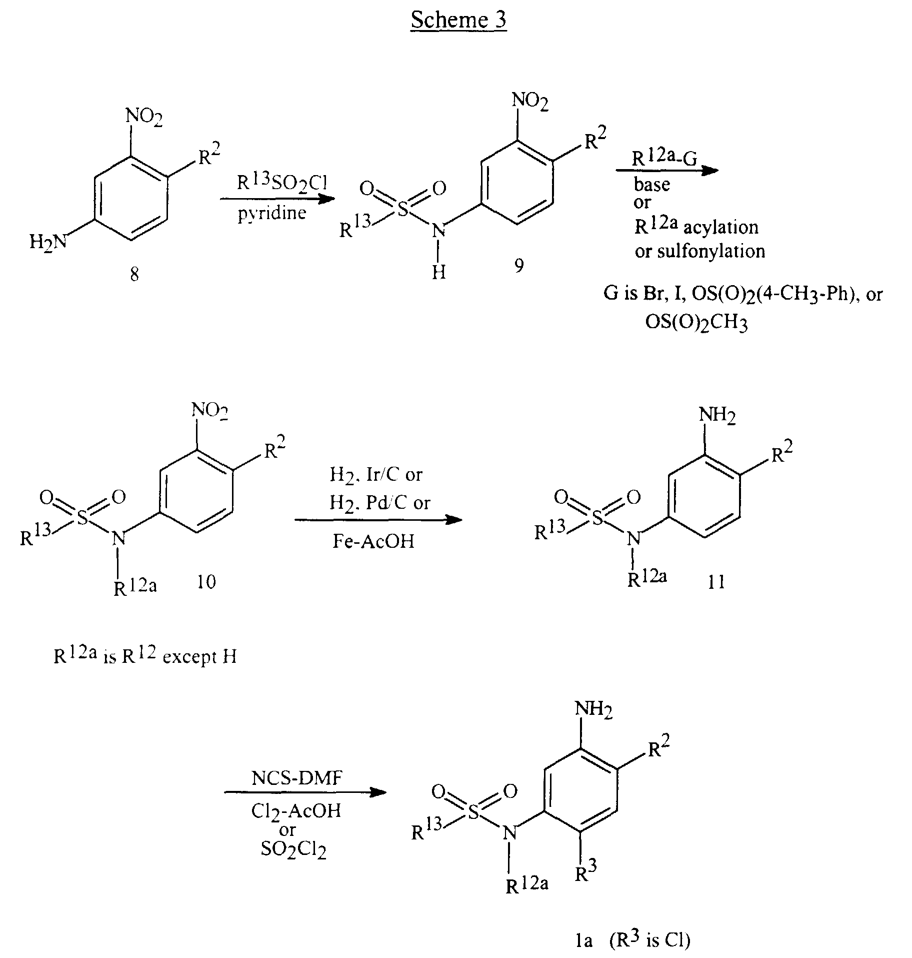

Anilines of Formula 1b (corresponding to compounds of Formula 1 wherein Q is Q-1 and R⁴ is alkoxy) are prepared by the method illustrated in Scheme 4. Alkylation of a nitrophenol of Formula 12 using an alkyl halide or alkyl sulfonate gives the corresponding ether of Formula 13. Reduction of the nitro group to the aniline of Formula 1b can be achieved by catalytic hydrogenation over a metal catalyst such as iridium or Raney® nickel, or by reduction with iron-acetic acid or stannous chloride.

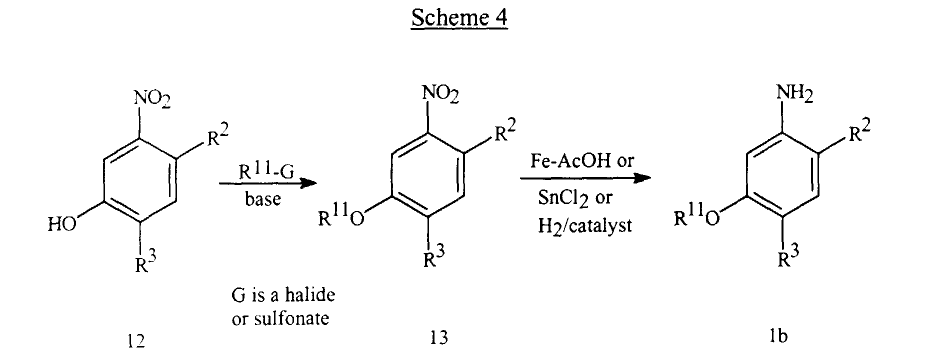

Anilines of Formula 1c (corresponding to compounds of Formula 1 wherein Q is Q-1 and R⁴ is thioalkoxy) are prepared by the method illustrated in Scheme 5. Alkylation of a nitrothiophenol of Formula 14 using an alkyl halide or alkyl sulfonate gives the corresponding thioether of Formula 15. Reduction of the nitro group to the aniline of Formula 1c can be achieved by iron-acetic acid or stannous chloride.

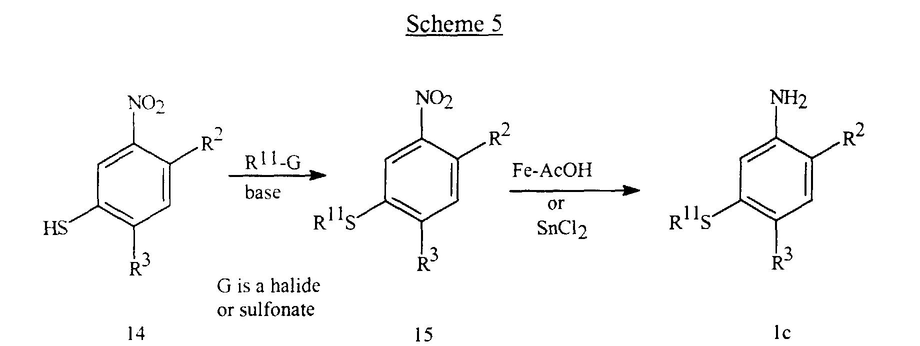

Anilines of Formula 1d (corresponding to compounds of Formula 1 wherein Q is Q-1 and R⁴ is an ester) are prepared by the method illustrated in Scheme 6. Nitration oi a carboxylic acid of Formula 16 using a nitric acid/sulfuric acid mixture affords the corresponding nitro compound of Formula 17. Esterification using an alkyl halide in the presence of a base, followed by reduction of the nitro group using iron-acetic or stannous chloride, gives the aniline of Formula 1d.

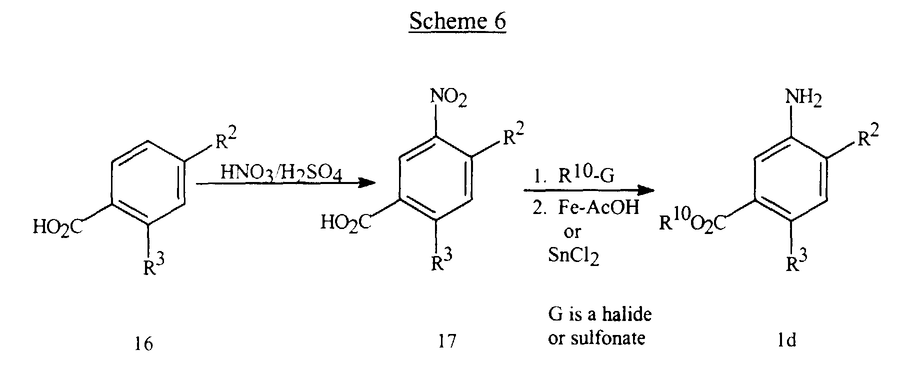

Anilines of Formula 1e (corresponding to compounds of Formula 1 wherein Q is Q-2) are prepared by the method illustrated in Scheme 7. Alkylation of a nitrophenol of Formula 18 using ethyl bromoacetate in the presence of base such as potassium carbonate or sodium hydride in dimethylformamide (DMF), tetrahydrofuran (THF), acetone or acetonitrile gives the corresponding alkyl ether, which on hydrogenation over a metal catalyst such as palladium adsorbed onto charcoal affords the benzoxazinone of Formula 19. Nitration of the compound of Formula 19 using a nitric acid/sulfuric acid mixture affords the nitro compound of Formula 20. Alkylation of the nitro compound of Formula 20 using either an alkyl halide or an alkyl sulfonate in the presence of base such as potassium carbonate or sodium hydride in DMF or THF gives the *N*-alkyl compound of Formula 21. Reduction of the nitro group to the aniline provides the compound of Formula le; this reduction can be achieved by catalytic hydrogenation over a metal catalyst such as palladium or iridium or by reduction with Raney® nickel, iron-acetic acid or stannous chloride.

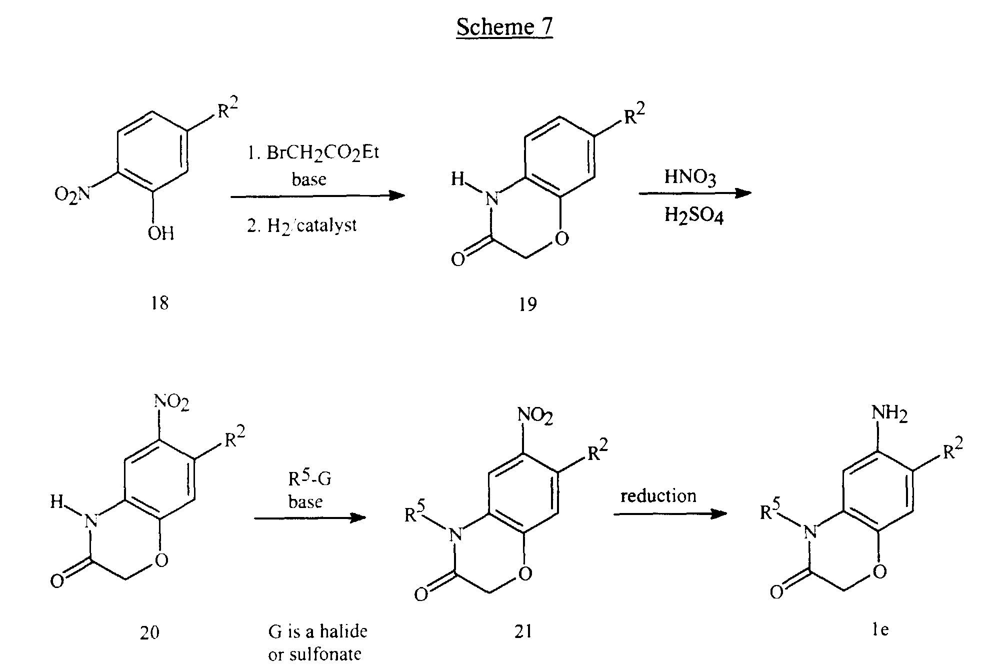

Anilines of Formula 1f (corresponding to compounds of Formula 1 wherein Q is Q-3) are prepared by the method illustrated in Scheme 8. Alkylation of a nitrophenol of Formula 12 with, for example, allyl bromide, in the presence of a base such as potassium carbonate or sodium hydride in *N,N*-dimethylformamide, tetrahydrofuran, acetone or acetonitrile provides the compound of Formula 22. The aniline of Formula 23 can be obtained by reduction of the nitro compound of Formula 22 by iron in acetic acid or stannous chloride in an appropriate inert solvent such as ethanol or ethyl acetate. Condensation of phthalic anhydride with the aniline of Formula 23 in acetic acid or xylene or toluene provides the compound of Formula 24. The compound of Formula 24 will undergo a Claisen rearrangement upon heating to 150 °C to provide the alcohol of Formula 25. Cyclization of the compound of Formula 25 can be accomplished by various methods known in the art to yield the saturated and unsaturated cyclic ethers. These methods include treatment with p-toluenesulfonic acid (PTSA) to yield compounds wherein R⁶ is methyl, and epoxidation followed by cyclization with PTSA or hydroboration followed by cyclization with PTSA to yield compounds wherein R⁶ is hydroxymethyl; see, for example, Brown, H. et al. *J. Org. Chem.* (1973), *38,* 4092. Deprotection of the phthalimide group with hydrazine provides the compound of Formula 1f.

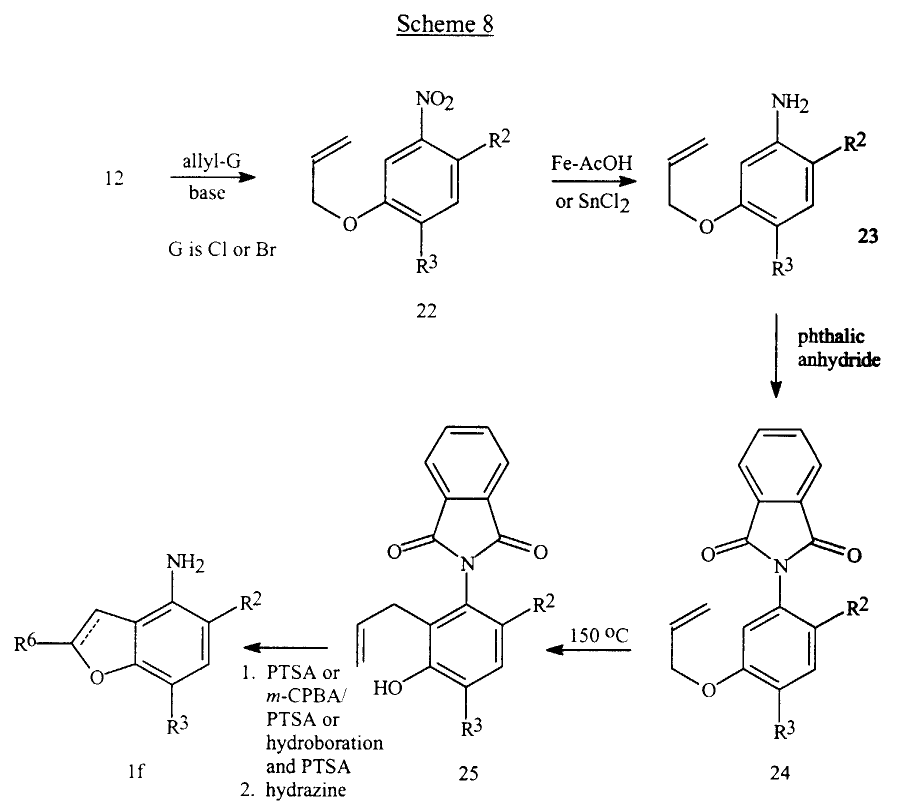

Anilines of Formula 1g (corresponding to compounds of Formula 1 wherein Q is Q-4) are prepared by the method illustrated in Scheme 9. The nitro aniline of Formula 26 is treated with a sulfonyl chloride in the presence of a base such as pyridine, *N,N*-dimethylaminopyridine (DMAP), or triethylamine at -20 °C to 25 °C to obtain the sulfonamide of Formula 27. For compounds wherein R⁷ is other than H, the sulfonamide nitrogen can be alkylated or acylated to give the R⁷-substituted compound of Formula 28. The alkylation is performed using an alkyl halide or alkyl sulfonate in the presence of a base such as potassium carbonate, sodium methoxide, potassium t-butoxide (*t*-BuOK), or sodium hydride in an anhydrous solvent such as dimethylformamide (DMF), tetrahydrofuran, or acetonitrile at ambient temperature to 80 °C. Acylations to form compounds wherein R⁷ is C(O)R⁸ are accomplished by condensing the sulfonamide of Formula 27 with the appropriate acylating agent, for example an acyl chloride, isocyanate or carbamoyl chloride.

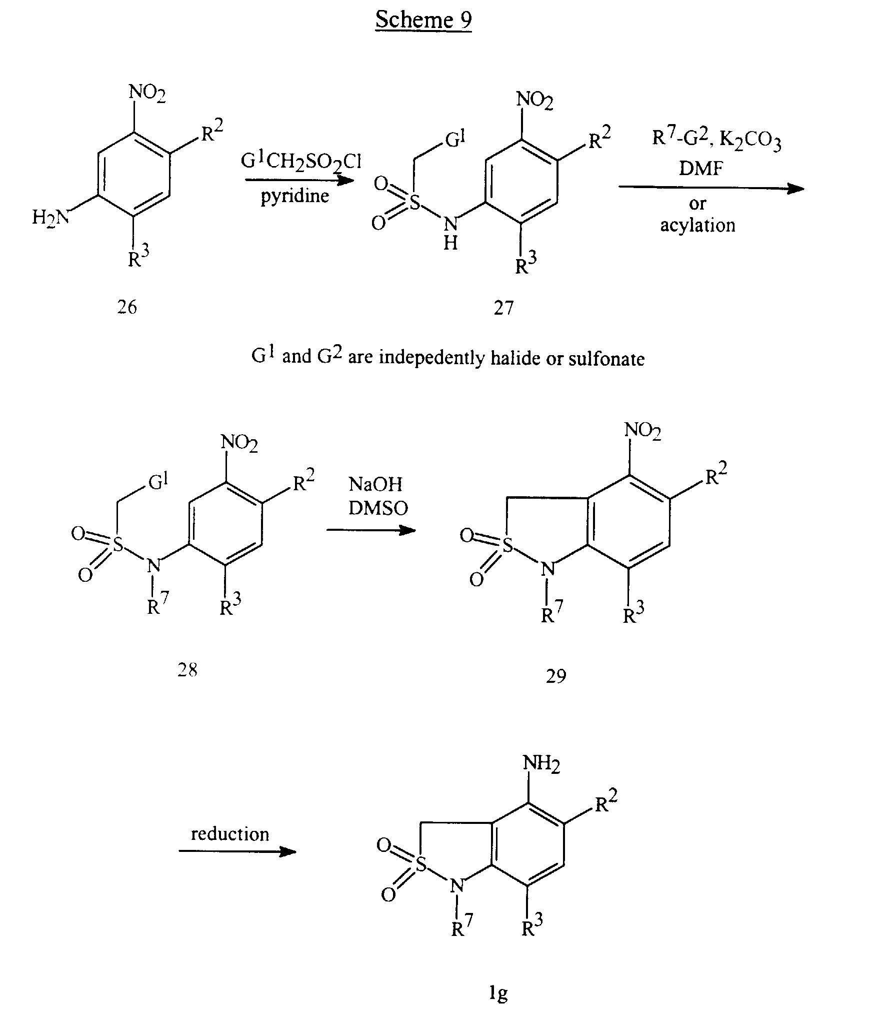

The cyclization to form the cyclic sulfonamide functionality is achieved via intramolecular vicarious nucleophilic substitution of hydrogen as described by K. Wojciechowski and M. Makosza in *Synthesis* (1992), 571. Treatment of the sulfonamide of Formula 28 with a base such as sodium hydroxide, sodium methoxide, or potassium *t*-butoxide in an inert solvent such as dimethyl sulfoxide (Me₂SO), dimethylformamide or tetrahydrofuran at a temperature between -60 °C and ambient temperature affords the cyclic sulfonamide of Formula 29. The nitro group in the compound of Formula 29 can be reduced to the corresponding aniline of Formula 1g using iron-HCI, SnCl₂·H₂O or by hydrogenation over palladium on carbon.

An alternate synthetic route to compounds of Formula 1g wherein R³ is Cl or Br involves the introduction of the R³ substituent at a later stage of the synthesis (Scheme 10). The cyclization of the compound of Formula 30 affords the sulfonamides of Formulae 31 and 32. The ratio of products depends on the nature of R², and the base and solvent used in the cyclization. The isomers can be separated by chromatography or crystallization techniques well known to one skilled in the art.

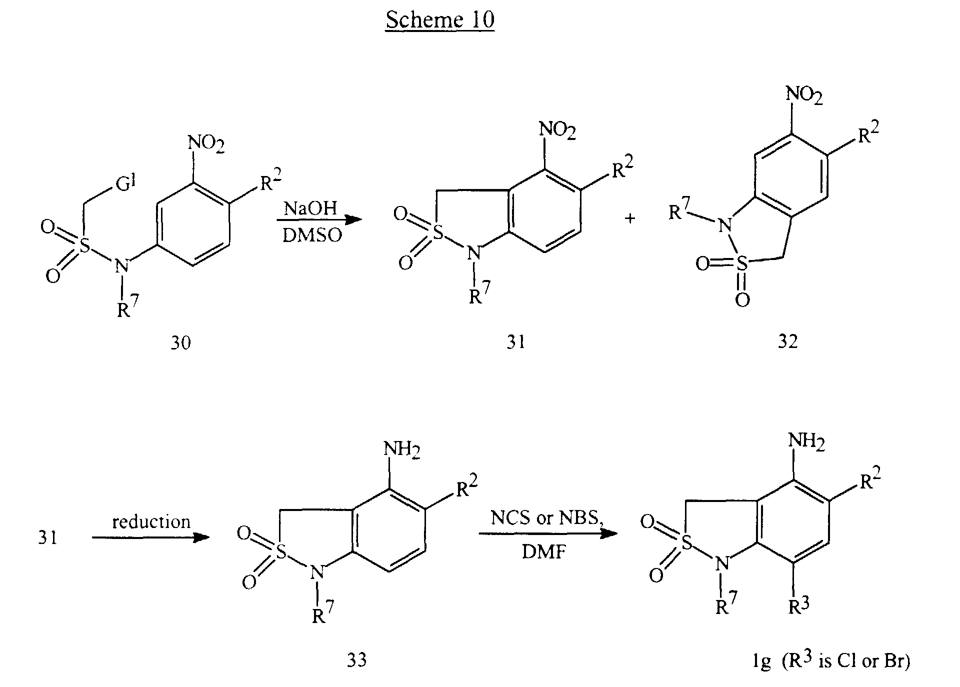

Reduction of the nitro compound of Formula 31 provides the aniline of Formula 33. Introduction of a chlorine or bromine atom to give the aniline of Formula 1g wherein R³ is Cl or Br can be accomplished using *N*-chlorosuccinimide (NCS) or *N*-bromosuccinimide (NBS), respectively, in an inert solvent such as dimethylformamide (DMF) at a temperature between ambient temperature and 80 °C.

Compounds of Formulae 4, 8, 12, 16, 18, and 26 are either commercially available or can be prepared by methods known to one skilled in the art. Compounds of Formula 30 can be prepared by the method described in Scheme 9 by starting from the appropriate nitro aniline of Formula 26 wherein R³ is H.

It is recognized that some reagents and reaction conditions described above for preparing compounds of Formula I may not be compatible with certain functionalities present in the intermediates. In these instances, the incorporation of protection/deprotection sequences or functional group interconversions into the synthesis will aid in obtaining the desired products. The use and choice of the protecting groups will be apparent to one skilled in chemical synthesis (see, for example, Greene, T. W.; Wuts, P. G. M. *Protective Groups in Organic Synthesis,* 2nd ed.; Wiley: New York, 1991). One skilled in the art will recognize that, in some cases, after the introduction of a given reagent as it is depicted in any individual scheme, it may be necessary to perform additional routine synthetic steps not described in detail to complete the synthesis of compounds of Formula I. One skilled in the art will also recognize that it may be necessary to perform a combination of the steps illustrated in the above schemes in an order other than that implied by the particular sequence presented to prepare the compounds of Formula I.

One skilled in the art will also recognize that compounds of Formula I and the intermediates described herein can be subjected to various electrophilic, nucleophilic, radical, organometallic, oxidation, and reduction reactions to add substituents or modify existing substituents.

Without further elaboration, it is believed that one skilled in the art using the preceding description can utilize the present invention to its fullest extent. The following Examples are, therefore, to be construed as merely illustrative, and not limiting of the disclosure in any way whatsoever. Percentages are by weight except for chromatographic solvent mixtures or where otherwise indicated. Parts and percentages for chromatographic solvent mixtures are by volume unless otherwise indicated. ¹H NMR spectra are reported in ppm downfield from tetramethylsilane; s = singlet, d = doublet, t = triplet, q = quartet, m = multiplet, dd = doublet of doublets, dt = doublet of triplets, br s = broad singlet.

### EXAMPLE 1

### Step A: Preparation of N-(4-chloro-2-fluoro-5-nitrophenyl)acetamide

To a stirred solution of *N*-(4-chloro-2-fluorophenyl)acetamide (180.6 g, 0.96 mol) in concentrated sulfuric acid (1L) was added a mixture of 175 mL of concentrated HNO₃ and 175 mL of concentrated sulfuric acid at 0 to 5 °C in 1.5 h. After the addition was finished, the reaction mixture was stirred for another 0.5 h. The solution was poured into 5 L of ice water. After the product precipitated, it was isolated by filtration and then was dissolved in 2.5 L of ethyl acetate. After separation of the water layer, the organic layer was dried over sodium sulfate and then the solvent was evaporated under reduced pressure. The crude product was triturated in diisopropyl ether (1.5 L), isolated by filtration, and dried under reduced pressure to afford the title compound of Step A (196.2 g, 87.6%) melting at 145-146 °C. ¹H NMR ((CD₃)₂SO): δ 10.3-10.2 (s, 1H), 8.9 (d, 1H), 7.9-7.8 (d, 1H), 2.2-2.1 (s, 3H).

### Step B: Preparation of N-(5-amino-4-chloro-2-fluorophenyl)acetamide

To a suspension of the title compound of Step A (116 g, 500 mmol) in 500 mL of ethyl acetate was added 3.63 g of iridium (5%) on charcoal. The hydrogenation was performed at 4 bar of hydrogen pressure in an autoclave for 4.5 h at 60 °C. The reaction was then filtered and the solvent removed under reduced pressure. Drying of the crystalline product yielded the title compound of Step B (99.4 g, 98%) melting at 142-143 °C. ¹H NMR (CDCl₃): δ 7.9 (d, 1H), 7.3-7.2 (br s, 1H), 7.1-7.0 (d, 1H), 4.1-4.0 (s, 2H), 2.2-2. (s, 3H).

### Step C: Preparation of N-[4-chloro-5-[[(chloromethyl)sulfonyl]amino]-2-fluorophenyl]acetamide

To a stirred solution of the title compound of Step B (8.10 g, 40 mmol) in pyridine (50 mL) was added chloromethylsulfonyl chloride (3.84 mL, 40.45 mmol) over 30 minutes at 0 °C. After stirring at room temperature for 14 h, chloromethylsulfonyl chloride (0.38 mL, 4.05 mmol) was added to complete the reaction. Water was added to precipitate the product. After filtration and washing with water and petroleum ether, the title compound of Step C was isolated as a bright powder (11.17 g, 89%) melting at 208-210 °C. ¹H NMR ((CD₃)₂SO): δ 10.2-10.1 (s, 1H), 9.9 (s, 1H), 8.1 (d, 1H), 7.6-7.5 (d, 1H), 5.0-4.9 (s, 2H), 2.1 (s, 3H).

### Step D: Preparation of N-(5-amino-2-chloro-4-fluorophenyl)-1-chloromethanesulfonamide

The title compound of Step C (10.3g, 32.6 mmol) was suspended in 450 mL of 2M HCl. The temperature was raised to the boiling point of the reaction mixture under an argon atmosphere. After 45 minutes of stirring at this temperature, the reaction was cooled to room temperature, neutralized with NaHCO₃, and the product was extracted into ethyl acetate. After drying of the solution over sodium sulfate, the solvent was evaporated under reduced pressure yielding 8.9 g (98%) of the title compound of Step D as bright crystals melting at 105-107 °C. ¹H NMR ((CD₃)₂SO): δ 9.9-9.8 (br s, 1H), 7.2 (d, 1H), 6.9 (d, 1H), 5.5-5.4 (s, 2H), 4.9 (s, 2H).

### Step E: Preparation of 1-chloro-N-(2-chloro-4-fluoro-5-isocyanatophenyl)methanesulfonamide

To a stirred solution of phosgene (68 mmol) in 35 mL toluene was added the title compound of Step D (4.2 g, 15.4 mmol) in 80 mL toluene at 0 °C and the reaction was stirred at room temperature overnight. The temperature was then raised to 70 °C for 4 h. After cooling to room temperature, the reaction mixture was filtered and the solvent was removed under reduced pressure. Drying of the white precipitate under reduced pressure yielded the title compound of Step E (4.4 g, 95%) melting at 101.5-102.5 °C. ¹H NMR (CDCl₃): δ 7.5 (d, 1H), 7.3 (d, 1H), 7.0-6.9 (br s, 1H), 4.6-4.5 (s, 2H).

### Step F: Preparation of N'-[4-chloro-5-[[(chloromethyl)sulfonyl]amino]-2-fluorophenyl]-N,N-dimethylurea

To a solution of the title compound of Step E (2.99 g, 10 mmol) in 50 mL of ethyl acetate was added *N,N*-dimethyl hydrazine (0.72 g, 12 mmol) dropwise at room temperature with stirring. The reaction mixture was stirred at room temperature for an additional 2 h under an argon atmosphere. The solvent was then removed under reduced pressure, and the crude product was purified by flash chromatography to yield the title compound of Step F as a white solid melting at 175-177 °C (3.26 g, 91%). ¹H NMR ((CD₃)₂SO): δ 10.25 (s, 1H), 8.52 (s, 1H), 8.23 (d, 1H), 8.02 (s, 1H), 7.56 (d, 1H), 4.95 (s, 2H), 2.55 (s, 3H) 2.50 (s, 3H).

### Step G: Preparation of 1-chloro-N-[2-chloro-5-(3-chloro-4,5-dihydro-1-methyl-5-oxo-1H-1,2,4-triazol-4-yl)-4-fluorophenyl]methanesulfonamide

A mixture of the title compound of Step F (2.8 g, 7.79 mmol) in 50 mL of ethyl acetate and diphosgene (4.43 g, 22 mmol) was heated at 70 °C for 4 h under an argon atmosphere. After completion of the reaction (TLC, 2:1 hexane:ethyl acetate), the solvent was removed under reduced pressure, and the title compound of Step G, a compound of this invention, was isolated and purified by flash chromatography to yield a white solid melting at 151-153 °C (2.53 g, 83%). ¹H NMR (CDCl₃): δ 7.74 (d, 1H), 7.40 (d, 1H), 7.11 (s, 1H), 4.59 (s, 2H), 3.53 (s, 3H).

### EXAMPLE 2

### Step A: Preparation of 1-chloro-5-fluoro-4-nitro-2-(2-propynyloxy)benzene

To a solution of 2-chloro-4-fluoro-5-nitrophenol (10 g, 52.5 mmol) in 250 mL of *N,N*-dimethylformamide containing potassium carbonate (14.5 g) was added propargyl bromide (7.5 g) at room temperature. The reaction mixture was stirred at room temperature under an argon atmosphere for 12 h. After completion of the reaction, the potassium carbonate was filtered off and the reaction mixture was diluted with ethyl acetate (500 mL) and washed with water (twice with 100 mL). The organic phase was separated, dried over MgSO₄, and the solvent was removed under reduced pressure. The title compound of Step A was isolated as an oil by flash chromatography. ¹H NMR (CDCl₃): δ 7.80 (d, 1H), 7. 38 (d, 1H), 4.85 (d, 2H), 2.63 (t, 1H).

### Step B: Preparation of 4-chloro-2-fluoro-5-(2-propynyloxy)benzenamine

To a mixture of iron powder (26.55 g) and 35 mL of an aqueous solution of acetic acid (5%) at 80 °C was added slowly dropwise a solution of the title compound of Step A (12.5 g) in 45 mL acetic acid and 50 mL of ethyl acetate. After completion of the addition, heating was continued for an additional 2 h. After completion of the reaction, the reaction mixture was filtered through a pad of Celite®. The filtrate was diluted with ethyl acetate (1 L), washed with water (twice with 200 mL) and with aqueous sodium bicarbonate (200 mL). The organic phase was separated, dried, and concentrated under reduced pressure. Purification by flash chromatography yielded the title compound of Step B as a solid melting at 90-91 °C. ¹H NMR (CDCl₃): δ 7.02 (d, 1H), 6. 54 (d, 1H), 4.93 (d, 2H), 3.36 (br s, 2H), 2.54 (t, 1H).

### Step C: Preparation of 1-chloro-3-fluoro-4-isocyanato-6-(2-propynyloxy)benzene

To a stirred solution of diphosgene (4 g, 20.3 mmol) in 75 mL of *p*-dioxane was added the title compound of Step B (3.98 g, 20 mm) in 10 mL of *p*-dioxane at room temperature. The reaction mixture was cooled to 10 °C and a solution of 5 mL of triethylamine in 25 mL of *p*-dioxane was added dropwise at that temperature. The temperature was then raised to 70 °C for 6 h. After cooling to room temperature, the reaction mixture was filtered and the solvent was removed under reduced pressure to give the title compound of Step C as an oil which was used directly as a starting material for Step D.

### Step D: Preparation of N'-[4-chloro-2-fluoro-5-(2-propynyloxy)phenyl]-N,N-dimethylurea

To a solution of title compound of Step C (3.0 g, 13.2 mmol) in 50 mL of ethyl acetate was added *N,N*-dimethyl hydrazine (0.96 g, 16 mmol) dropwise at room temperature with stirring. The reaction mixture was stirred at room temperature for an additional 2 h under an argon atmosphere. After 2 h, the solvent was removed under reduced pressure, and the title compound of Step D was purified by flash chromatography to yield a solid melting at 147-148 °C (3.42 g, 91%). ¹H NMR (CDCl₃): δ 8.41 (s, 1H), 8.22 (d, 1H), 7. 12 (d, 1H), 5.96 (s, 1H), 4.75 (d, 2H), 2.58 (s, 6H), 2.53 (t, 1H).

### Step E: Preparation of 5-chloro-4-[4-chloro-2-fluoro-5-(2-propynyloxy)phenyl]-2,4-dihydro-2-methyl-3H-1,2,4-triazol-3-one

A mixture of the title compound of Step D (2.28 g, 8 mmol) in 30 mL of ethyl acetate and diphosgene (4.5 g, 22.8 mmol) was heated at 70 °C for 4 h under an argon atmosphere. After completion of the reaction (TLC, 2:1 hexane:ethyl acetate), the solvent was removed under reduced pressure, and the title compound of Step E, a compound of this invention, was isolated by flash chromatography as a white solid melting at 120-122 °C (2.1 g, 86%).): ¹H NMR (CDCl₃): δ 7.32 (d, 1H), 7. 04 (d, 1H), 4.77 (s, 2H), 3.51 (s, 3H), 2.59 (t, 1H).

### EXAMPLE 3

### Step A: Preparation of 2-chloro-4-fluoro-5-nitrobenzoic acid

To a stirred solution of 2-chloro-4-fluorobenzoic acid (50 g, 0.286 mol) in concentrated sulfuric acid (250 mL) was added a mixture of concentrated nitric acid (50 mL) and concentrated sulfuric acid (50 mL) at 0 to 5 °C over a period of 30 minutes. After the addition was finished, the reaction mixture was stirred for another 30 minutes. The solution was then poured into 2 L of ice water. After the product precipitated, it was isolated by filtration and was then dissolved in ethyl acetate (1 L). The organic layer was separated, dried, and concentrated under reduced pressure to yield the title product of Step A as a colorless solid melting at 138-140 °C (57.6 g, 92%). ¹H NMR (CDCl₃): δ 8.80 (d, 1H), 7. 42 (d, 1H).

### Step B: Preparation of phenylmethyl 2-chloro-4-fluoro-5-nitrobenzoate

A mixture of the title compound of Step A (47.3 g, 0.21 mol), benzyl bromide (44.2 g, 0.25 mol) and potassium carbonate (75 g) in anhydrous acetone (400 mL) was stirred at room temperature under an argon atmosphere for 12 h. After completion of the reaction, the potassium carbonate was removed by filtration and the solvent was removed under reduced pressure. The title compound of Step B was purified by flash chromatography to afford a solid melting at 86-87 °C (60.9 g, 94%). ¹H NMR (CDCl₃): δ 8.62 (d, 1H), 7. 42 (m, 6H), 5.36 (s, 2H).

### Step C: Preparation of phenylmethyl 5-amino-2-chloro-4-fluorobenzoate

A mixture of stannous chloride (57.1 g) and the title compound of Step B (25 g, 84 mmol) in 300 mL of ethyl acetate was heated at reflux for 4 h. After completion of the reaction, the reaction mixture was cooled and sodium carbonate (27 g) was added and the mixture stirred at room temperature for 10 h. The precipitated inorganic salts were removed by filtration through a pad of Celite®. The filtrate was concentrated under reduced pressure and the title compound of Step C was purified by flash chromatography to yield a solid melting at 66-68 °C (19.2 g, 82%). ¹H NMR (CDCl₃): δ 7.40 (m, 6H), 7. 08 (d, 1H), 5.34 (s, 2H), 3.82 (s, 2H).

### Step D: Preparation of phenylmethyl 2-chloro-4-fluoro-5-isocyanatobenzoate

To a stirred solution of diphosgene (2 g, 10.15 mmol) in 45 mL of *p*-dioxane was added the title compound of Step C (2.8 g, 10 mm) in 10 mL p-dioxane at room temperature. The reaction mixture was cooled to 10 °C and a solution of 4 mL of triethylamine in 20 mL of *p*-dioxane was added dropwise at that temperature. The temperature was then raised to 70 °C for 6 h. After cooling to room temperature, the reaction mixture was filtered and the solvent was removed under reduced pressure to give the title compound of Step D as an oil which was used directly as a starting material in Step E.

### Step E: Preparation of phenylmethyl 2-chloro-5-[[(dimethylamino)carbonyl]amino]-4-fluorobenzoate

To a solution of title compound of Step D (3.06 g, 10 mmol) in 50 mL of ethyl acetate was added *N,N*-dimethyl hydrazine (0.90 g, 15 mmol) dropwise at room temperature with stirring. The reaction mixture was stirred at room temperature for an additional 2 h under an argon temperature. After 2 h, solvent was removed under reduced pressure, and the title compound of Step E was purified by flash chromatography to yield a solid melting at 144-145 °C (3.35 g, 92%). ¹H NMR (CDCl₃): δ 8.82 (d, 1H), 8. 40 (s, 1H), 7.38 (m, 5H), 7.20 (d, 1H), 6.10 (s, 1H), 5.35 (s, 2H), 2.58 (s, 6H).

### Step F: Preparation of phenylmethyl 2-chloro-5-(3-chloro-4,5-dihydro-1-methyl-5-oxo-1H-1,2,4-triazol-4-yl)-4-fluorobenzoate

A mixture of the title compound of Step E (1.83 g, 5 mmol) in 30 mL of ethyl acetate and diphosgene (2.0 g, 10 mmol) was heated at 70 °C for 4 h under an argon atmosphere. After completion of the reaction (TLC, 2:1 hexane:ethyl acetate), the solvent was removed under reduced pressure, and the title compound of Step F was isolated by flash chromatography to yield a solid melting at 134-136 °C (1.7 g, 88%). ¹H NMR (CDCl₃): δ 7.96 (d, 1H), 7. 40 (m, 6H), 5.36 (s, 2H), 3.51 (s, 3H).

### Step G: Preparation of 2-chloro-5-(3-chloro-4,5-dihydro-1-methyl-5-oxo-1H-1,2,4-triazol-4-yl)-4-fluorobenzoic acid

A suspension of the title compound of Step F (1.54 g, 4 mmol) and palladium adsorbed on charcoal (5%) in ethyl acetate was stirred under a hydrogen atmosphere for 2 h. The catalyst was removed by filtration and the solvent was removed under reduced pressure to give the title compound of Step G as a solid melting at 197-198 °C (1.17 g, 96%). ¹H NMR (CDCl₃): δ 13.60 (br s, 1H), 8. 20 (d, 1H), 7.94 (d 1H), 3.42 (s, 3H).

### Step H: Preparation of 1-methylethyl 2-chloro-5-(3-chloro-4,5-dihydro-1-methyl-5-oxo-1H-1,2,4-triazol-4-yl)-4-fluorobenzoate

A mixture of the title compound of Step G (0.6 g, 1.96 mmol), 2-bromopropane (0.37 g, mmol), and potassium carbonate (0.5 g) in anhydrous dimethylformamide (10 mL) was stirred at room temperature under an argon atmosphere for 6 h. The reaction mixture was then diluted with ethyl acetate (50 mL) and washed with water (twice with 20 mL). The organic phase was dried, concentrated under reduced pressure, and the title compound of Step H, a compound of this invention, was purified by flash chromatography to yield a white solid melting at 80-82 °C (0.605 g, 89%). ¹H NMR (CDCl₃): δ 7.90 (d, 1H), 7. 40 (d, 1H), 5.27 (m, 1H), 3.52 (s, 3H). 1.39 (d, 6H).

### EXAMPLE 4

### Step A: Preparation of 7-fluoro-2H-1,4-benzoxazin-3(4H)-one

A mixture of 3-fluoro-5-nitrophenol (50 g, 0.318 mol), ethyl bromoacetate (63.78 g) and potassium carbonate (44 g) in anhydrous dimethylformamide (350 mL) was stirred at room temperature under an argon atmosphere for 12 h. The reaction mixture was diluted with ethyl acetate (1 L), washed with water (twice with 300 mL) and brine (twice with 300 mL). The organic phase was separated, dried and the solvent was removed under reduced pressure to give the corresponding ether compound (74 g, 96%).
A suspension of the above compound (27.3 g, 0.112 mol) in tetrahydrofuran (110 mL) containing palladium adsorbed on charcoal (10%, 1.5 g ) was hydrogenated using a Parr shaker at 48 psi of hydrogen. After completion of the reaction the catalyst was removed by filtration through a pad of Celite®, and the solvent was removed under reduced pressure to give the title compound of Step A as a solid melting at 209-210 °C (18 g, 96%). ¹H NMR (CDCl₃): δ 6.94 (m, 1H), 6. 72 (m, 2H), 4.59 (s, 2H), 3.52 (s, 3H). 1.39 (d, 6H).

### Step B: Preparation of 7-fluoro-6-nitro-2H-1,4-benzoxazin-3(4H)-one

To a stirred solution of the title compound of Step A (10 g, 59 mmol) in concentrated sulfuric acid (35 mL) was added a mixture of concentrated nitric acid (7 mL) and concentrated sulfuric acid (7 mL) at 0 to 5 °C over a period of 30 minutes. After the addition was finished, the reaction mixture was stirred for another 30 minutes. The solution was poured into 1 L of ice water. After the product precipitated, it was isolated by filtration and was then dissolved in ethyl acetate (500 mL). The organic layer was separated, dried and concentrated under reduced pressure to afford the title compound of Step B as a colorless solid melting at 185-189 °C (10.5 g, 84%). ¹H NMR ((CD₃)₂SO): δ 7.62 (d, 1H), 7.24 (d, 1H), 4.78 (s, 2H), 3.52 (s, 3H). 1.39 (d, 6H).

### Step C: Preparation of 7-fluoro-6-nitro-4-(2-propynyl)-2H-1,4-benzoxazin-3(4H)-one

A mixture of the title compound of Step B (3.17 g, 14.9 mmol), propargyl bromide (2.67 g), and potassium carbonate (4.2 g) in anhydrous dimethylformamide (30 mL) was stirred at room temperature under an argon atmosphere for 12 h. After completion of the reaction, the potassium carbonate was removed by filtration and the reaction mixture was diluted with ethyl acetate (200 mL), and washed with water (twice with 100 mL). The solvent was removed under reduced pressure to yield the title compound of Step C, which was purified by flash chromatography (3.0 g, 81%). ¹H NMR ((CD₃)₂SO): δ 7.98 (d, 1H), 7.34 (d, 1H), 4.94 (s, 2H), 4.82 (d, 2H), 2.48 (t, 1H).

### Step D: Preparation of 6-amino-7-fluoro-4-(2-propynyl)-2H-1,4-benzoxazin-3(4H)-one

To a mixture of iron powder (6.7 g) and 15 mL of an aqueous solution of acetic acid (5%) at 80 °C was added slowly dropwise a solution of the title compound of Step C (3.0 g, 12 mmol) in 12 mL acetic acid and 12 mL of ethyl acetate. After completion of the addition, heating was continued for an additional 2 h. After completion of the reaction, the reaction mixture was filtered through a pad of Celite®. The filtrate was diluted with ethyl acetate (200 mL) and washed with water (twice with 25 mL) and with aqueous sodium bicarbonate (25 mL). The organic phase was separated, dried, and concentrated under reduced pressure. Purification by flash chromatography yielded the title compound of Step D as a solid melting at 146-148 °C. ¹H NMR (CDCl₃): δ 6.72 (d, 1H), 6.64 (d, 1H), 4.62 (d, 2H), 4.55 (s, 2H), 3.64 (br s, 2H), 2.28 (t, 1H).

### Step E: Preparation of 7-fluoro-6-isocyanato-4-(2-propynyl)-2H-1,4-benzoxazin-3(4H)-one

To a stirred solution of diphosgene (1.97 g, 10 mmol) in 45 mL of *p*-dioxane was added the title compound of Step D (2.1 g, 9.5 mmol) in 10 mL of *p*-dioxane at room temperature. The reaction mixture was cooled to 10 °C and a solution of 4 mL of triethylamine in 20 mL of *p*-dioxane was added dropwise at that temperature. The reaction mixture was then filtered and the solvent was removed under reduced pressure to give the title compound of Step E as an oil (2.3 g) which was used directly as a starting material in Step F.

### Step F: Preparation of N'-[3,4-dihydro-7-fluoro-3-oxo-4-(2-propynyl)-2H-1,4-benzoxazin-6-yl]-N,N-dimethylurea

To a solution of title compound of Step E (2.1 g, 8.5 mmol) in 50 mL of ethyl acetate was added *N,N*-dimethyl hydrazine (0.6 g, 10 mmol) dropwise at room temperature with stirring. The reaction mixture was stirred at room temperature for an additional 2 h under an argon atmosphere. After 2 h, the solvent was removed under reduced pressure, and the title compound of Step F was purified by flash chromatography to afford a solid melting at 182-183 °C (2.8 g, 92%). ¹H NMR ((CD₃)₂SO): δ 8.37 (s, 1H), 7. 96 (d, 1H), 7.85 (s, 1H), 7.06 (d, 1H), 4.67 (s, 2H), 4.60 (d, 2H), 3.28 (t, 1H), 2.52 (s, 6H).

### Step G: Preparation of 6-(3-chloro-4,5-dihydro-1-methyl-5-oxo-1H-1,2,4-triazol-4-yl)-7-fluoro-4-(2-propynyl)-2H-1,4-benzoxazin-3(4H)-one

A mixture if the title compound of Step F (1.53 g, 5 mmol) in 30 mL of ethyl acetate and diphosgene (2.0 g, 10 mmol) was heated at 70 °C for 4 h under an argon atmosphere. After completion of the reaction (TLC, 2:1 hexane:ethyl acetate), the solvent was removed under reduced pressure, and the title compound of Step G, a compound of this invention, was isolated by flash chromatography as a white solid melting at 193-195 °C (1.5 g, 89%). ¹H NMR (CDCl₃): δ 7.16 (d, 1H), 6.96 (d, 1H), 4.18 (s, 2H), 4.12 (m, 2H), 3.53 (s, 3H), 2.30 (t,lH).

### EXAMPLE 5

### Step A: Preparation of 1-chloro-N-(4-fluoro-3-nitrophenyl)methanesulfonamide

To a solution of 4-fluoro-3-nitroaniline (23.4 g, 0.15 mol) in anhydrous pyridine (120 mL) was added dropwise a solution of chloromethylsulfonyl chloride (22.35 g, 0.15 mol) at 0 °C under a nitrogen atmosphere. The mixture was then stirred at 0-5 °C for 4 h and then the mixture was poured onto ice (1500 g) containing concentrated aqueous hydrochloric acid (300 mL). The mixture was filtered, and the isolated solid was dissolved in methylene chloride (1200 mL). After drying (MgSO₄) and concentration under reduced pressure, the crude product was dissolved in 1-chlorobutane:ether 9:1 (v/v) and passed through silica gel. The eluent was concentrated under reduced pressure to give the title compound of Step A as a solid melting at 118-119 °C.

### Step B: Preparation of 1-chloro-N-(4-fluoro-3-nitrophenyl)-N-[(4-methoxyphenvl)methyl]methanesulfonamide

A solution of the title compound of Step A (3 g, 11.16 mmol), 4-methoxybenzyl chloride (2.1 g, 13.4 mmol), tetrabutylammonium bromide (0.36 g, 1.1 mmol) and anhydrous potassium carbonate (4.5 g, 13.8 mmol) in anhydrous dimethylformamide (20 mL) was stirred at room temperature for 6 h. When the starting material had been consumed based on TLC (silica gel), the reaction mixture was poured into water (100 mL) and extracted with ethyl acetate (250 mL). The organic phase was washed with brine (100 mL), dried (MgSO₄), and the solvent was removed under reduced pressure. Flash chromatography of the residue afforded 2.5 g of the title compound of Step A as a solid melting at 106-107 °C. ¹H NMR (CDCI₃, 300 MHz): δ 8.01 (dd, 1H), 7.48 (m,1H), 7.21 (dd, 1H), 6.80-7.12 (m, 4H), 4.93 (s, 2H), 4.57 (s, 2H), 3.77 (s, 3H).

### Step C: Preparation of 5-fluoro-1,3-dihydro-1-[(4-methoxyphenyl)methyl]-4-nitro-2,1-benzisothiazole 2,2-dioxide and 5-fluoro-1,3-dihydro-1-[(4-methoxyphenyl)methyl]-6-nitro-2,1-benzisothiazole 2,2-dioxide

The title compound of Step B (1.66 g, 4.26 mmol) in dimethyl sulfoxide (8 mL) was added dropwise to a vigorously stirred suspension of powdered NaOH (3.32 g) in dimethyl sulfoxide (15 mL) while maintaining the temperature at 25-27 °C. After completion of the addition, the reaction mixture was stirred at 25-27 °C for an additional 45 min. The reaction mixture was then poured into a mixture of ice (600 g) and concentrated HCI (40 mL) and extracted with ethyl acetate (300 mL). The organic extract was dried (MgSO₄) and the solvent was removed under reduced pressure. Flash chromatography of the resulting residue afforded two products. The first compound to elute was 5-fluoro-1,3-dihydro-1-[(4-methoxyphenyl)methyl]-6-nitro-2,1-benzisothiazole 2,2-dioxide (Compound A) as a solid melting at 104-105 °C. ¹H NMR (CDCl₃, 400 MHz): δ 7.26 (m, 3H), 6.88 (m, 3H), 4.68 (s, 2H), 4.46 (s, 2H), 3.82 (s, 3H). The second compound to elute was 5-fluoro-1,3-dihydro-1-[(4-methoxyphenyl)methyl]-4-nitro-2,1-benzisothiazole 2,2-dioxide (Compound B) as a solid melting at 182-183 °C. ¹H NMR (CDCl₃, 400 MHz): δ 7.30 (d, 2H), 7.16 (t, 1H), 6.90 (d, 2H), 6.74 (dd, 1H), 4.73 (d, 2H), 3.80 (s, 3H).

### Step D: Preparation of 5-fluoro-1,3-dihydro-1-[(4-methoxyphenyl)methyl]-2,1-benzisothiazol-4-amine 2,2-dioxide

A mixture of 5-fluoro-1,3-dihydro-1-[(4-methoxyphenyl)methyl]-4-nitro-2,1-benzisothiazole 2,2-dioxide (1.54 g, 4.37 mmol) and SnCl₂·H₂O (4.93 g, 21.9 mmol) in ethyl acetate (200 mL) was heated at reflux for 3 h. Then the reaction mixture was cooled, diluted with ethyl acetate (200 mL) and sodium carbonate (15 g) was added. The resulting mixture was vigorously stirred for 12 h and filtered through a pad of Celite®. The filtrate was concentrated under reduced pressure and the residue was subjected to flash chromatography to give 1.35 g of the title compound of Step D as brown solid melting at 154-157 °C. ¹H NMR (CDCl₃, 400 MHz): δ 7.33 (d, 2H), 6.87 (d, 2H), 6.82 (dd, 1H), 5.91 (dd, 1H), 4.65 (s, 2H), 4.22 (s, 2H), 3.79 (s, 3H), 3.75 (br s, 2H).

### Step E: Preparation of 7-chloro-5-fluoro-1,3-dihydro-1-[(4-methoxyphenyl)methyl]-2,1-benzisothiazol-4-amine 2,2-dioxide

A mixture of the title compound of Step D (0.387 g, 1.2 mmol) and *N*-chlorosuccinimide (0.168 g, 1.26 mmol) in dimethylformamide (6 mL) was heated at 80 °C for 4 h. After completion of the reaction, the mixture was poured into a mixture of ether (25 mL) and water (10 mL). The organic phase was separated, dried (MgSO₄) and the solvent was removed under reduced pressure. Flash chromatography of the resulting residue afforded 0.4 g of the title compound of Step D as a solid melting at 126-127 °C. ¹H NMR (CDCl₃, 400 MHz): δ 7.23 (d, 2H), 7.03 (d, 1H), 6.74 (d, 2H), 4.93 (s, 2H), 3.84 (s, 2H), 3.75 (s, 3H).

### Step F: Preparation of 7-chloro-5-fluoro-1,3-dihydro-4-isocyanato-1-[(4-methoxyphenyl)methyl]-2,1-benzisothiazole 2,2-dioxide

Following the procedure described in Step C in Example 2, reaction of the title compound of Step E (0.68 g, 2 mmol), diphosgene (2.2 mmol) and triethylamine (3 mmol) afforded the title compound of Step F as a an oil which was used directly as a starting material for Step G.

### Step G: Preparation of N'-[7-chloro-5-fluoro-1,3-dihydro-1-[(4-methoxyphenyl)methyl]-2,2-dioxido-2,1-benzisothiazol-4-yl]-N,N-dimethylurea

To a solution of title compound of Step F (7.7 g, 20 mmol) in 50 mL of ethyl acetate was added *N,N*-dimethyl hydrazine (1.32 g, 22 mmol) dropwise at room temperature with stirring. The reaction mixture was stirred at that temperature for an additional 2 h under an argon atmosphere. After 2 h, the solvent was removed under reduced pressure, and the title compound of Step G was purified by flash chromatography (8 g, 90%) as a solid melting at 99-100 °C. ¹H NMR (CDCl₃): δ 8.06 (s, 1H), 7.28 (d, 2H), 7.12 (d, 1H), 6.78 (d, 2H), 5.71 (s, 1H), 4.96 (s, 2H), 4.24 (s, 2H), 3.74 (s, 3H), 2.60 (s,6H).

### Step H: Preparation of 5-chloro-4-[7-chloro-5-fluoro-1,3-dihydro-1-[(4-methoxyphenyl)methyl]-2,2-dioxido-2,1-benzisothiazol-4-yl]-2,4-dihydro-2-methyl-3H-1,2,4-triazol-3-one

A mixture of the title compound of Step G (3.96 g, 9 mmol) in 30 mL of ethyl acetate and diphosgene (4.0 g) was heated at 70 °C for 4 h under an argon atmosphere. After completion of the reaction (TLC, 2:1 hexane:ethyl acetate), the solvent was removed under reduced pressure, and the title compound of Step H was isolated by flash chromatography as a white solid melting at 182-184 °C (3.8 g, 89%).¹H NMR (CDCl₃): δ 7.21 (d, 1H), 7.24 (d, 2H), 6.78 (d, 2H), 4.99 (s, 2H), 4.04 (q, 2H), 3.74 (s, 3H), 3.48 (s,3H).

### Step I: Preparation of 5-chloro-4-(7-chloro-5-fluoro-1,3-dihydro-2,2-dioxido-2,1-benzisothiazol-4-yl)-2,4-dihydro-2-methyl-3H-1,2,4-triazol-3-one

A mixture of the title compound of Step H (0.5 g, 1.05 mmol) and concentrated sulfuric acid (1.5 mL) in ethyl acetate (10 mL) was stirred at room temperature under an argon atmosphere for 12 h. The reaction mixture was then diluted with ethyl acetate (30 mL), washed with water (three times 15 mL) and sodium bicarbonate solution (5%, twice with 10 mL). The organic phase was separated, dried, and concentrated under reduced pressure to afford the title product of Step I (0.275 g, 74%) as a solid melting at 249-250 °C. ¹H NMR ((CD₃)₂SO): δ 7.70(s, 1H), 7.38 (d, 1H), 4.62 (q, 2H), 4.04 (q, 2H), 3.48 (s,3H).

### Step J: Preparation of 1-acetyl-7-chloro-4-(3-chloro-4,5-dihydro-1-methyl-5-oxo-1H-1,2,4-triazol-4-yl)-5-fluoro-1,3-dihydro-2,2-dioxido-2,1-benzisothiazole

To a solution of the title compound of Step (0.3 g, 0.8 mmol), pyridine (0.5 mL) and dimethylaminopyridine (50 mg) in anhydrous methylene chloride (5 mL) was added acetyl chloride (90 mg) at room temperature. The reaction mixture was stirred under an argon atmosphere for 2 h. After completion of the reaction, the solvent was removed under reduced pressure and the title product of Step J, a compound of this invention, was isolated by flash chromatography as a colorless solid melting at 200-203 °C (0.255 g, 81%). ¹H NMR (CDCl₃): δ 7.46 (d, 1H), 4.62 (q, 2H), 4.54 (q, 2H), 3.54 (s,3H), 2.60 (s, 3H).

By the procedures described herein together with methods known in the art, the following compounds of Tables 1 to 4 can be prepared. The following abbreviation is used in the Tables which follow: CN = cyano.

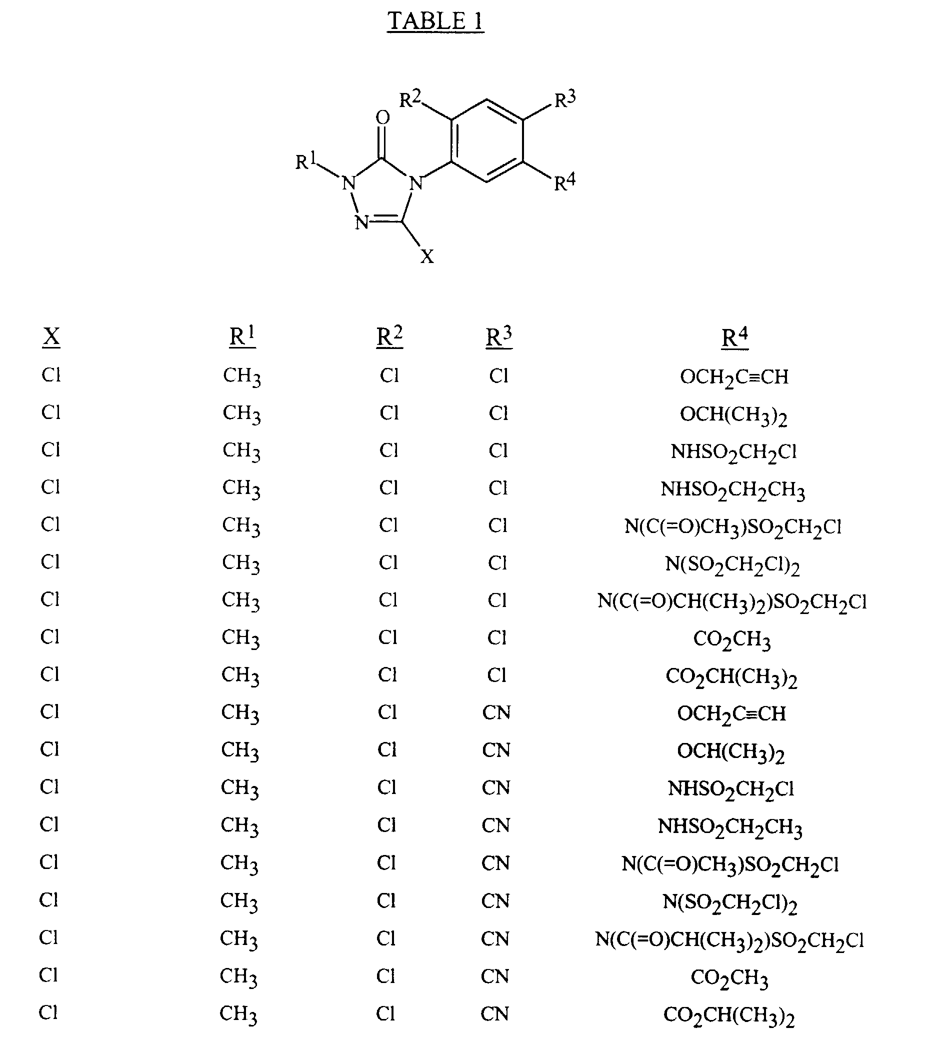

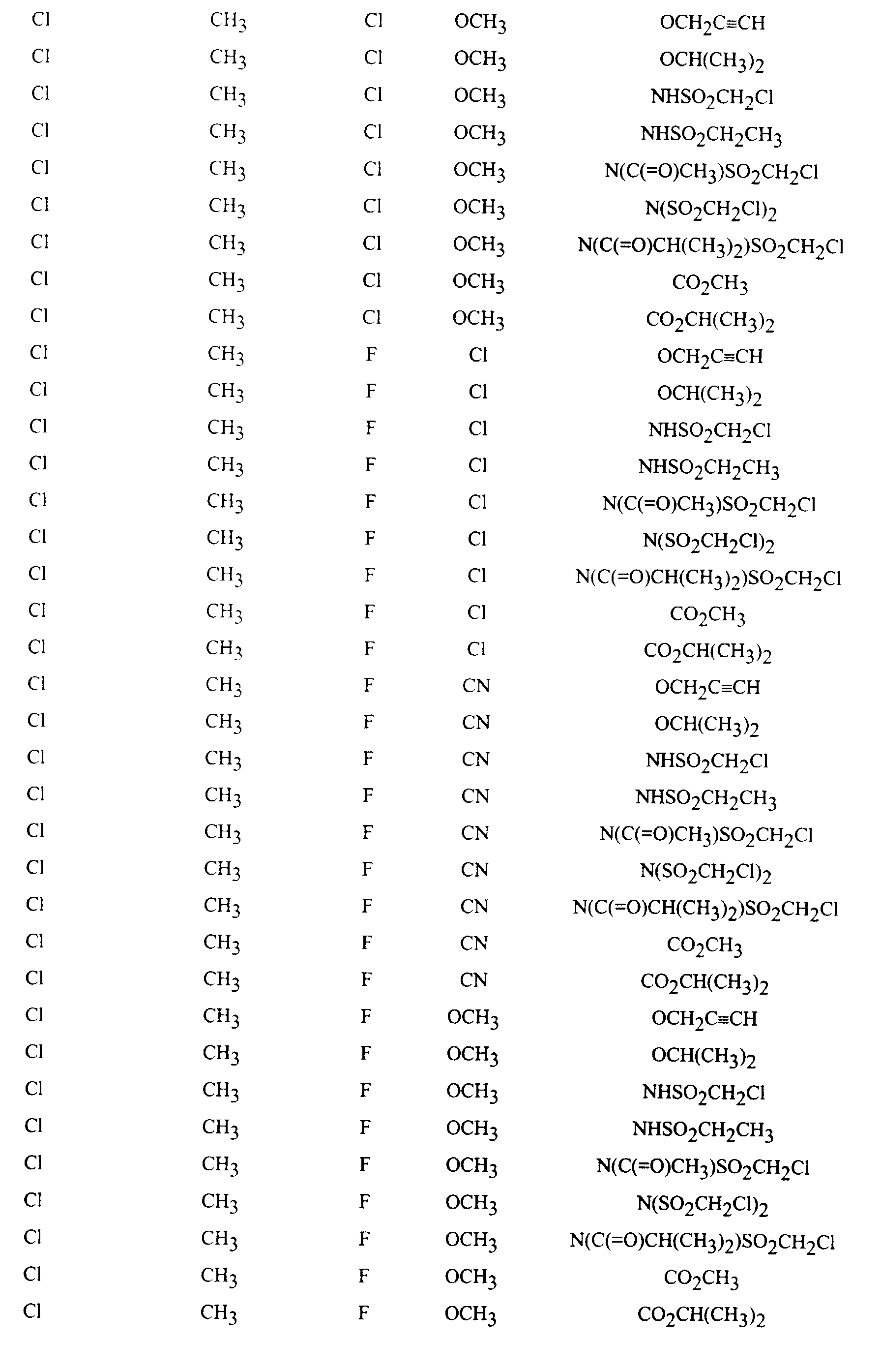

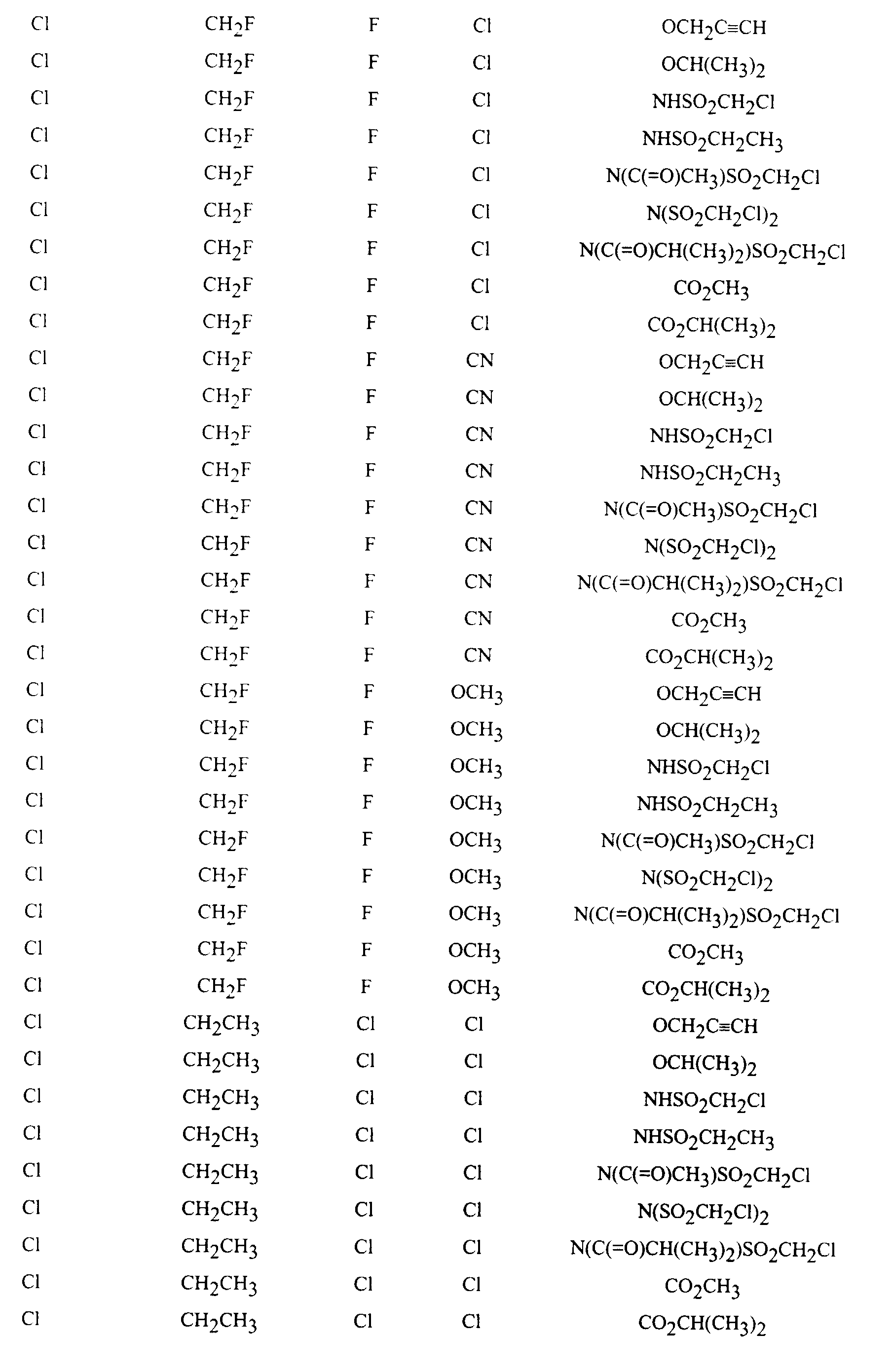

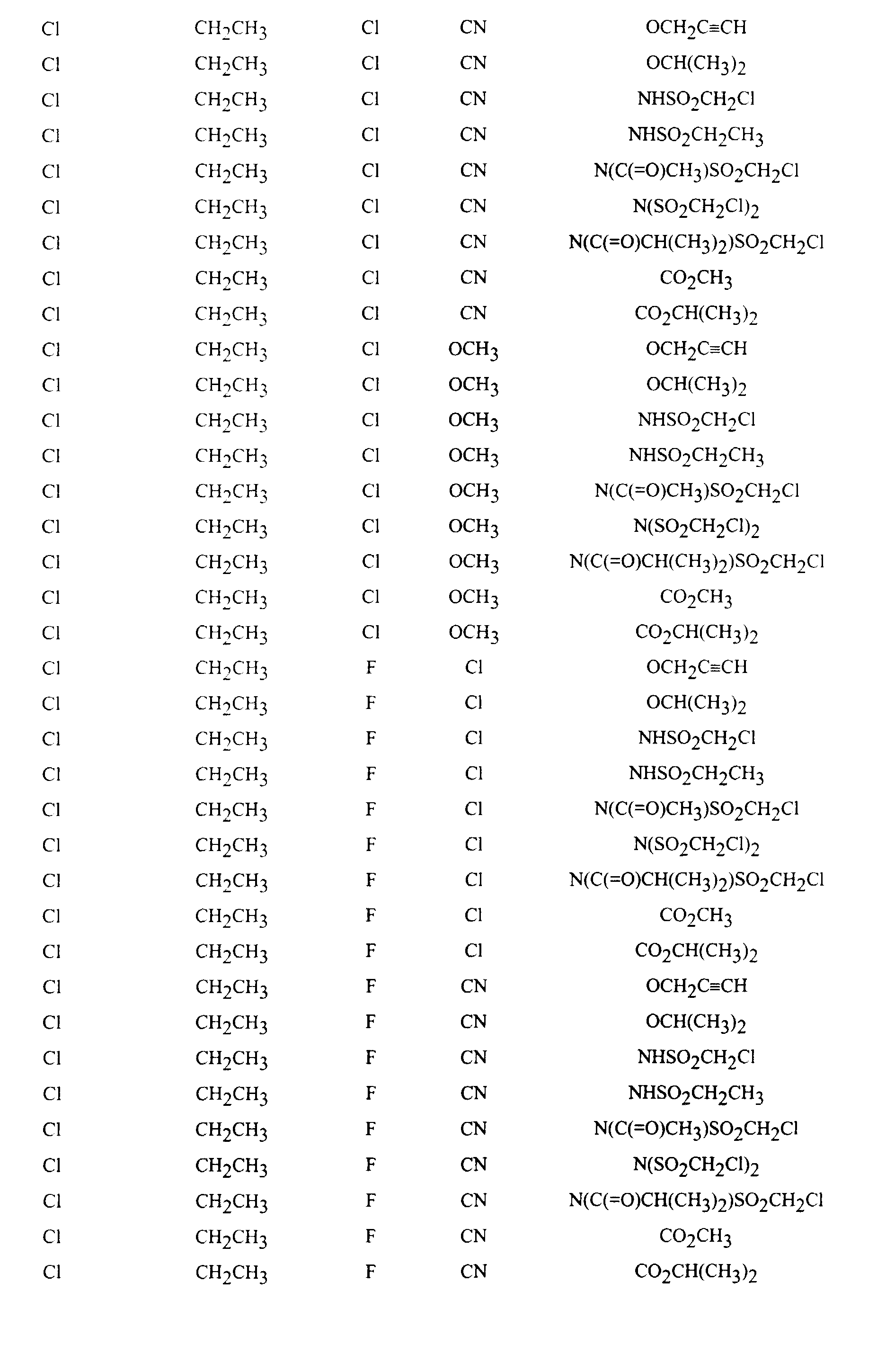

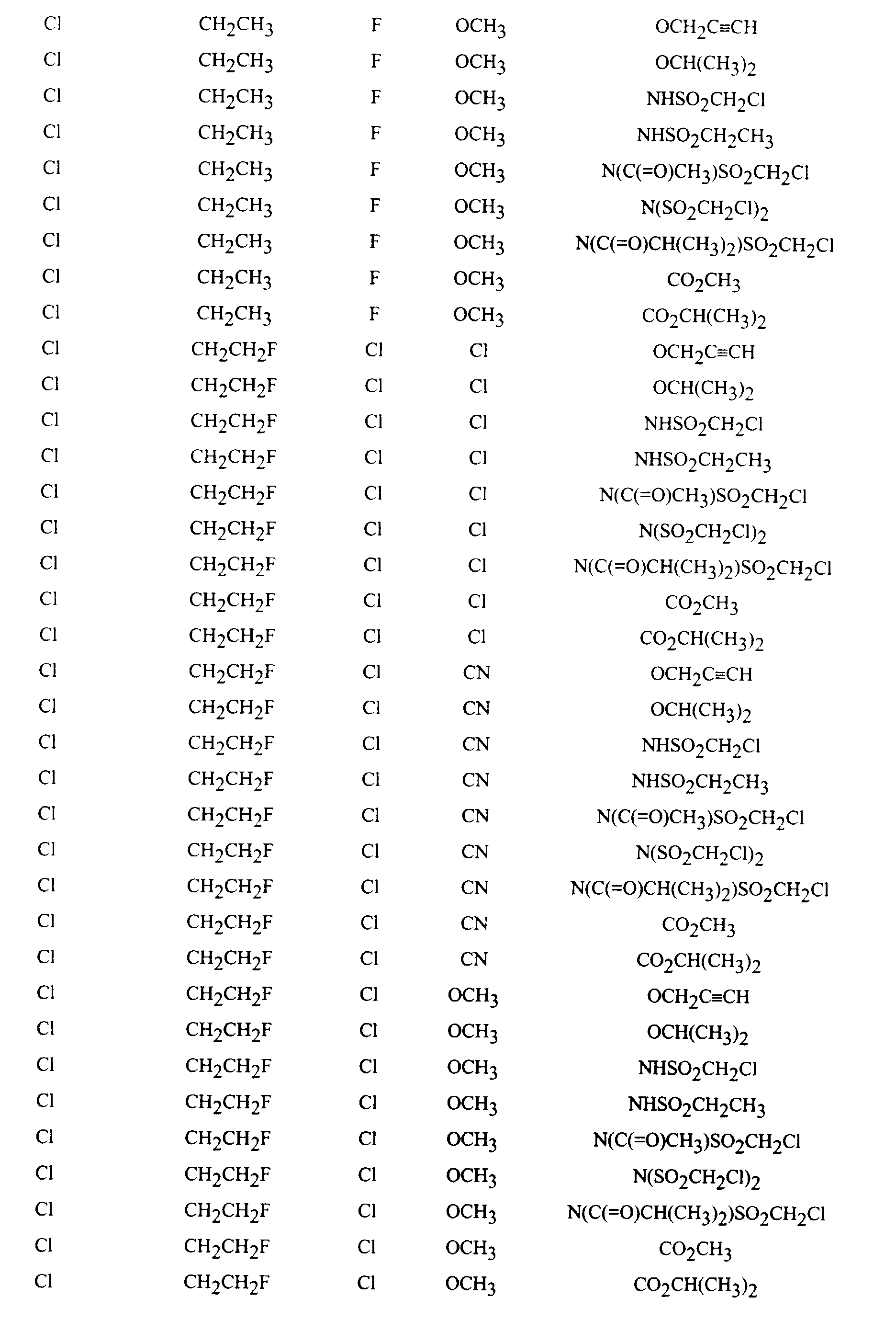

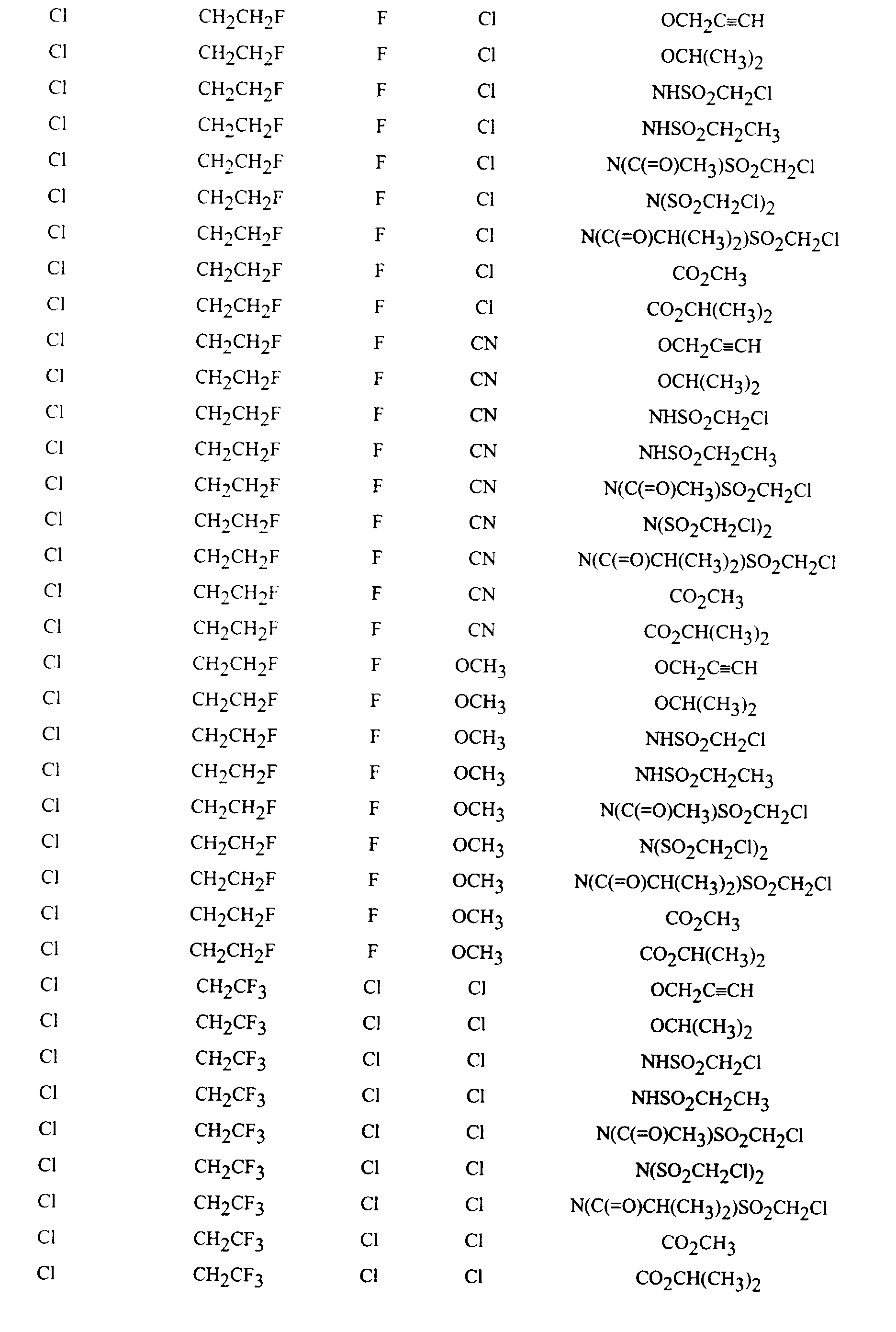

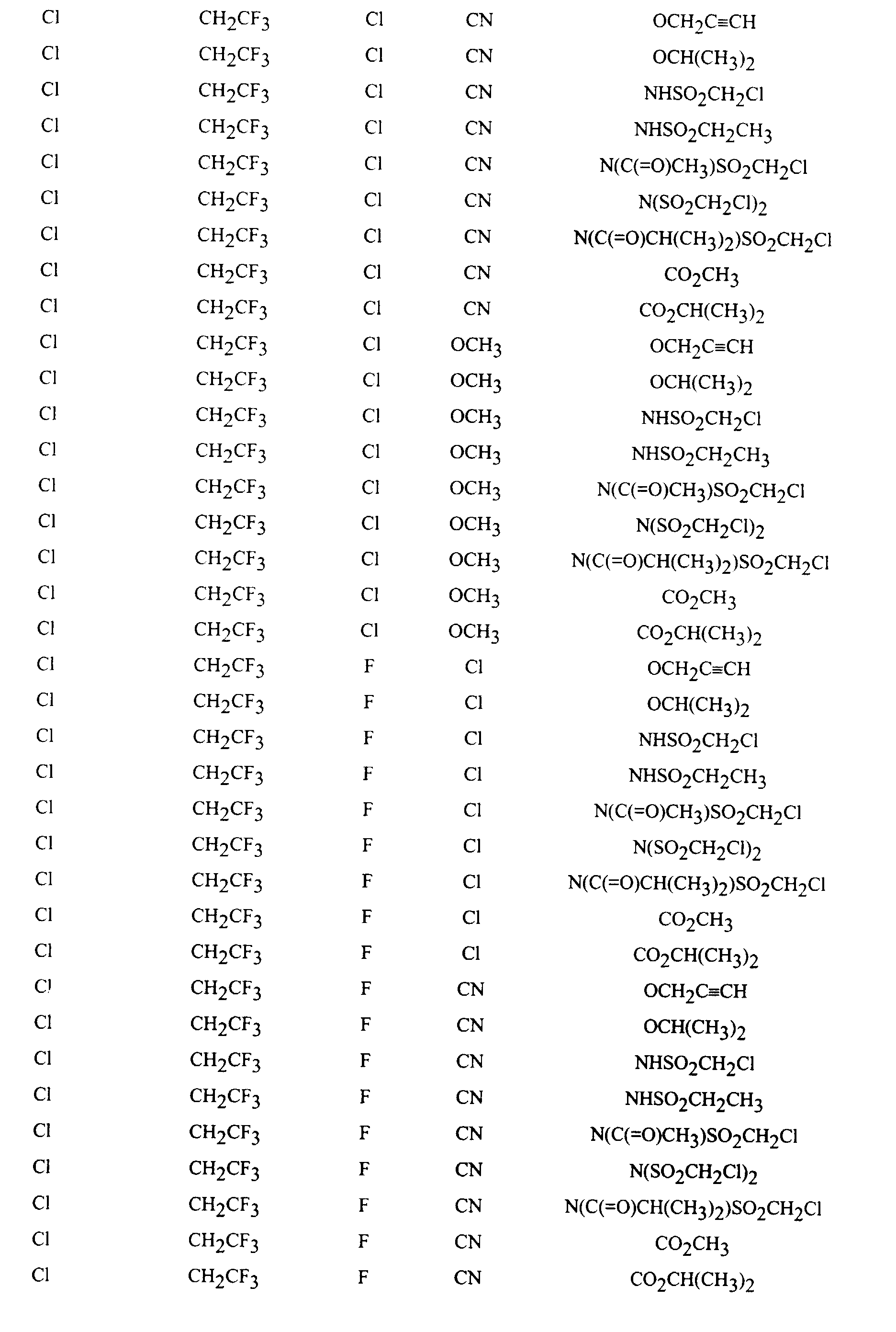

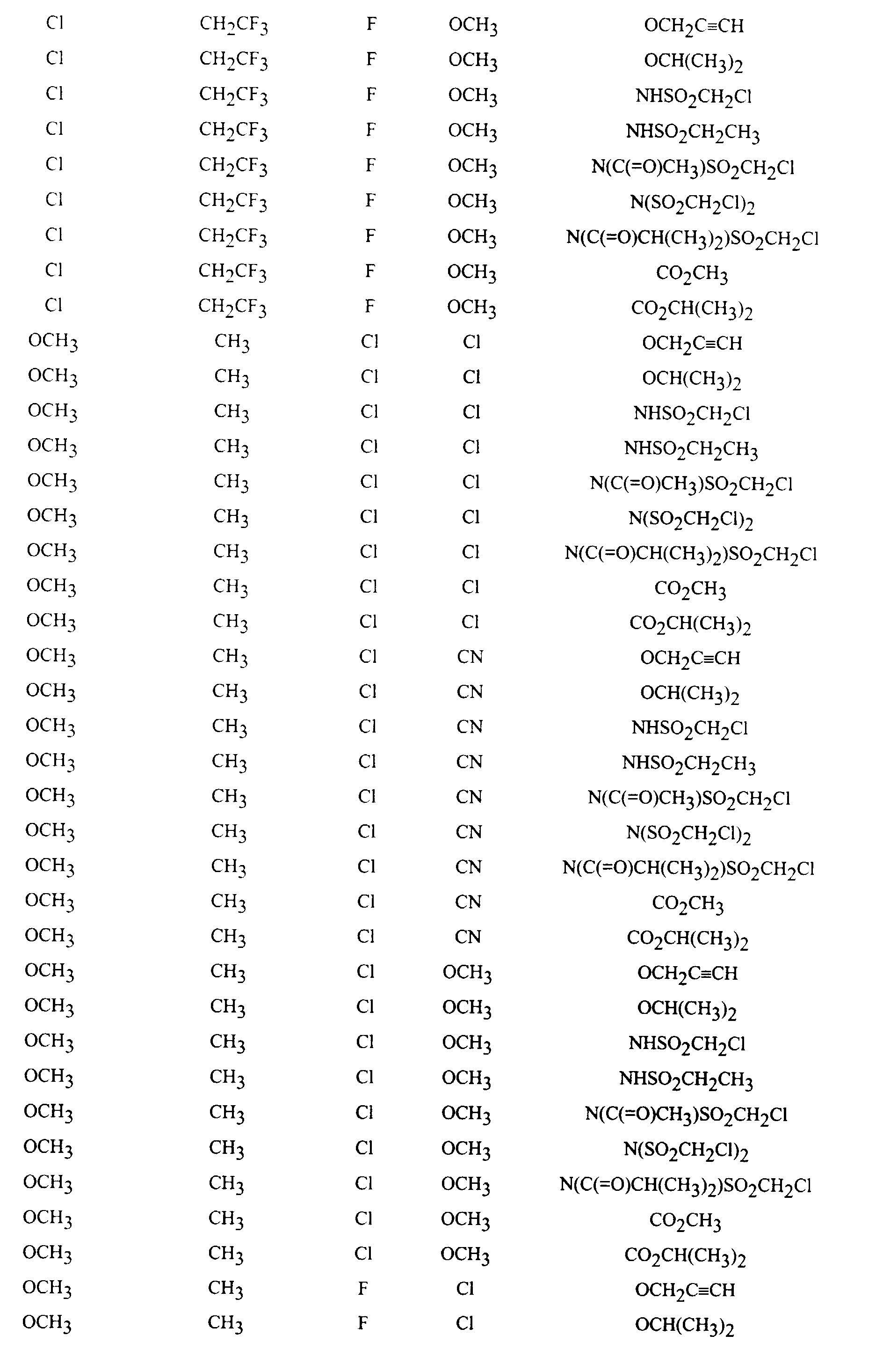

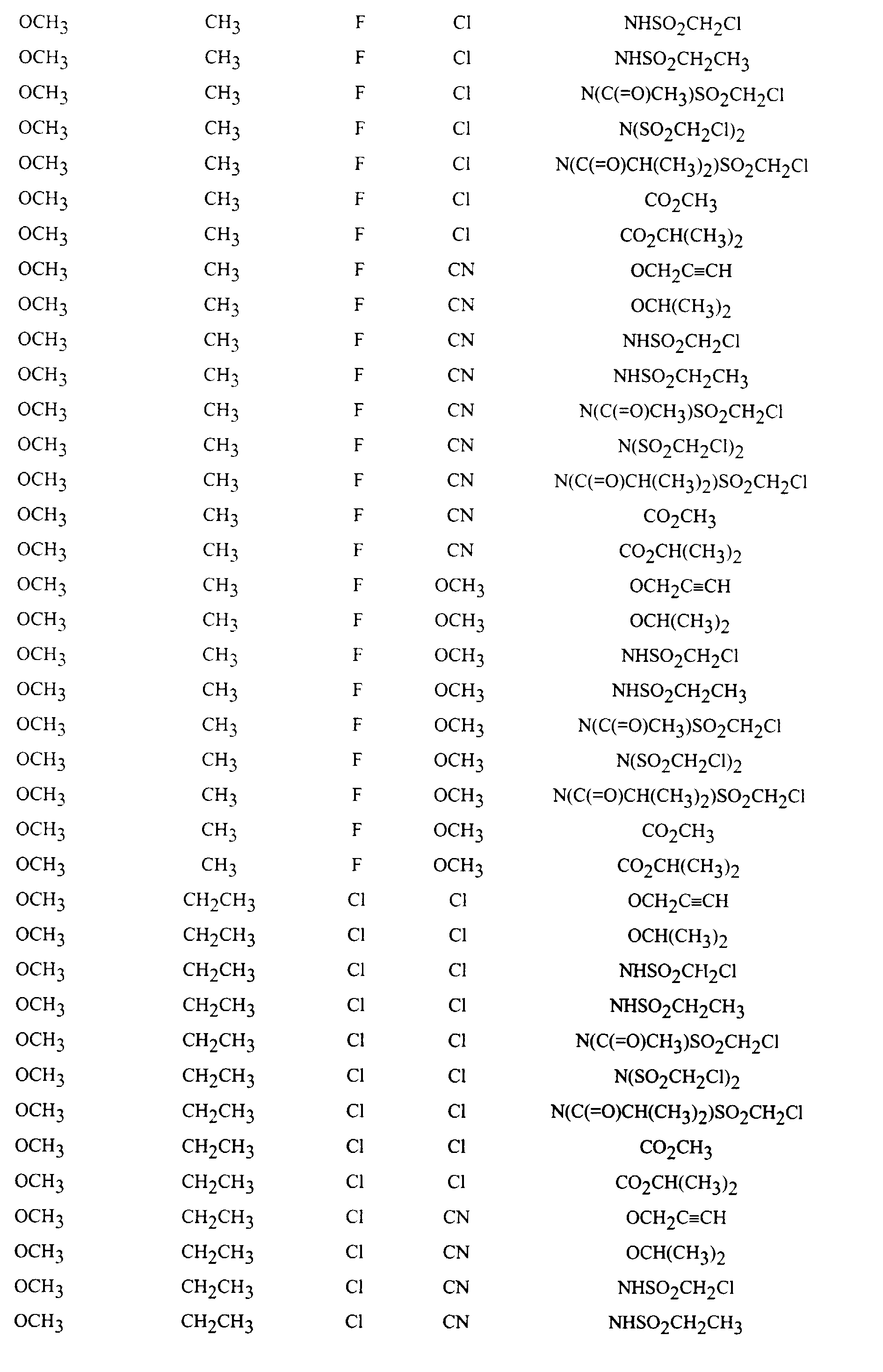

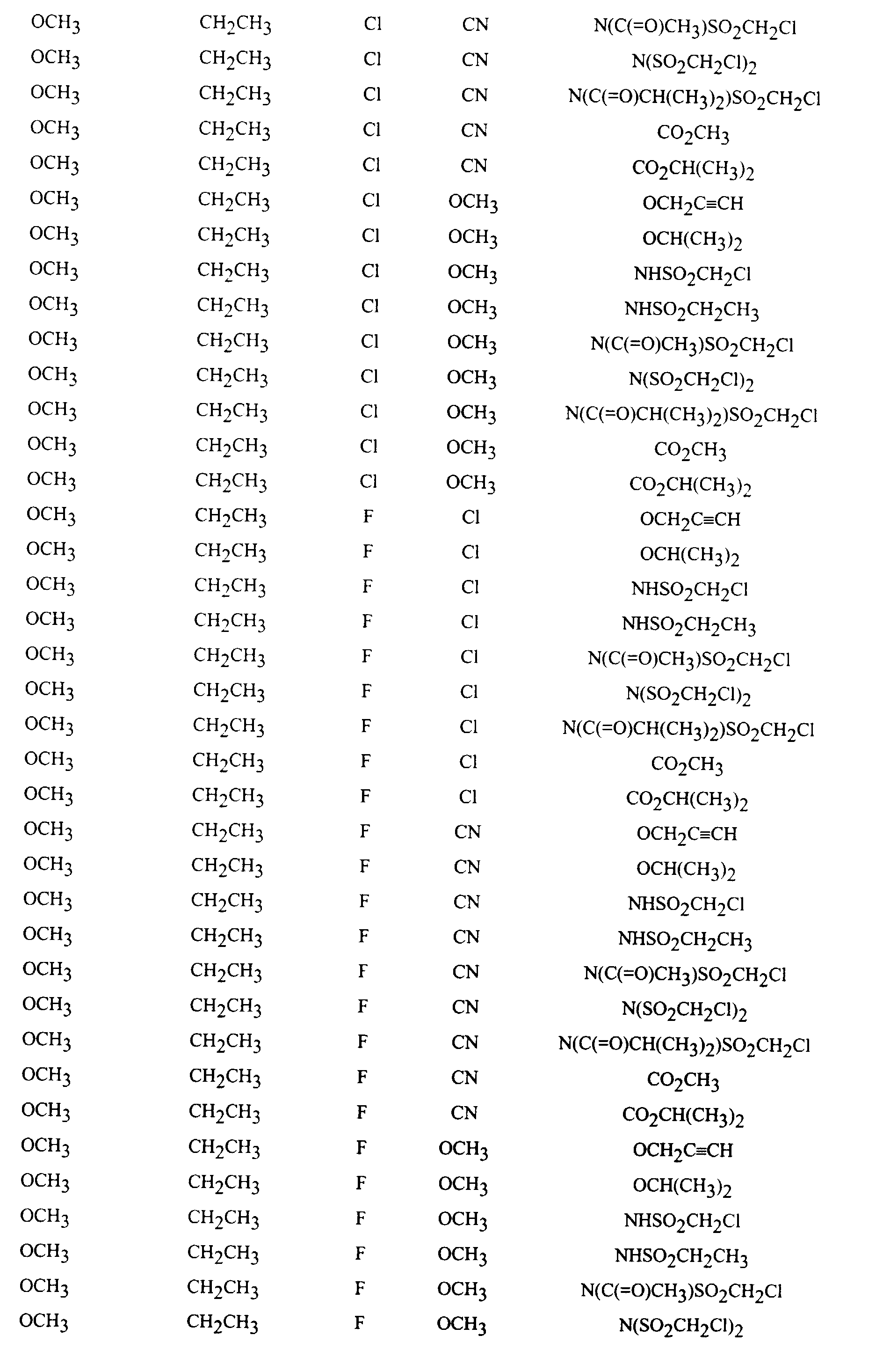

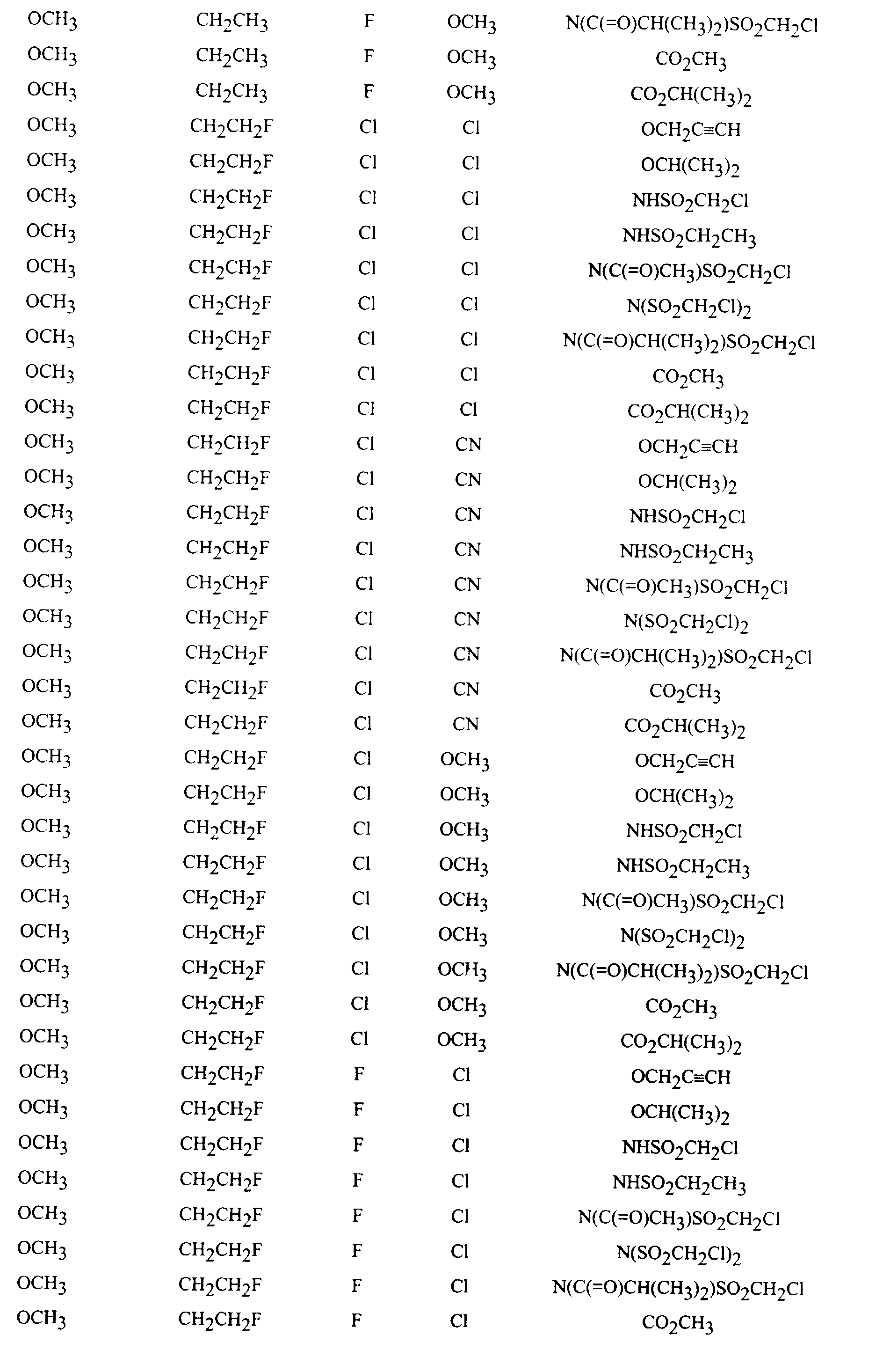

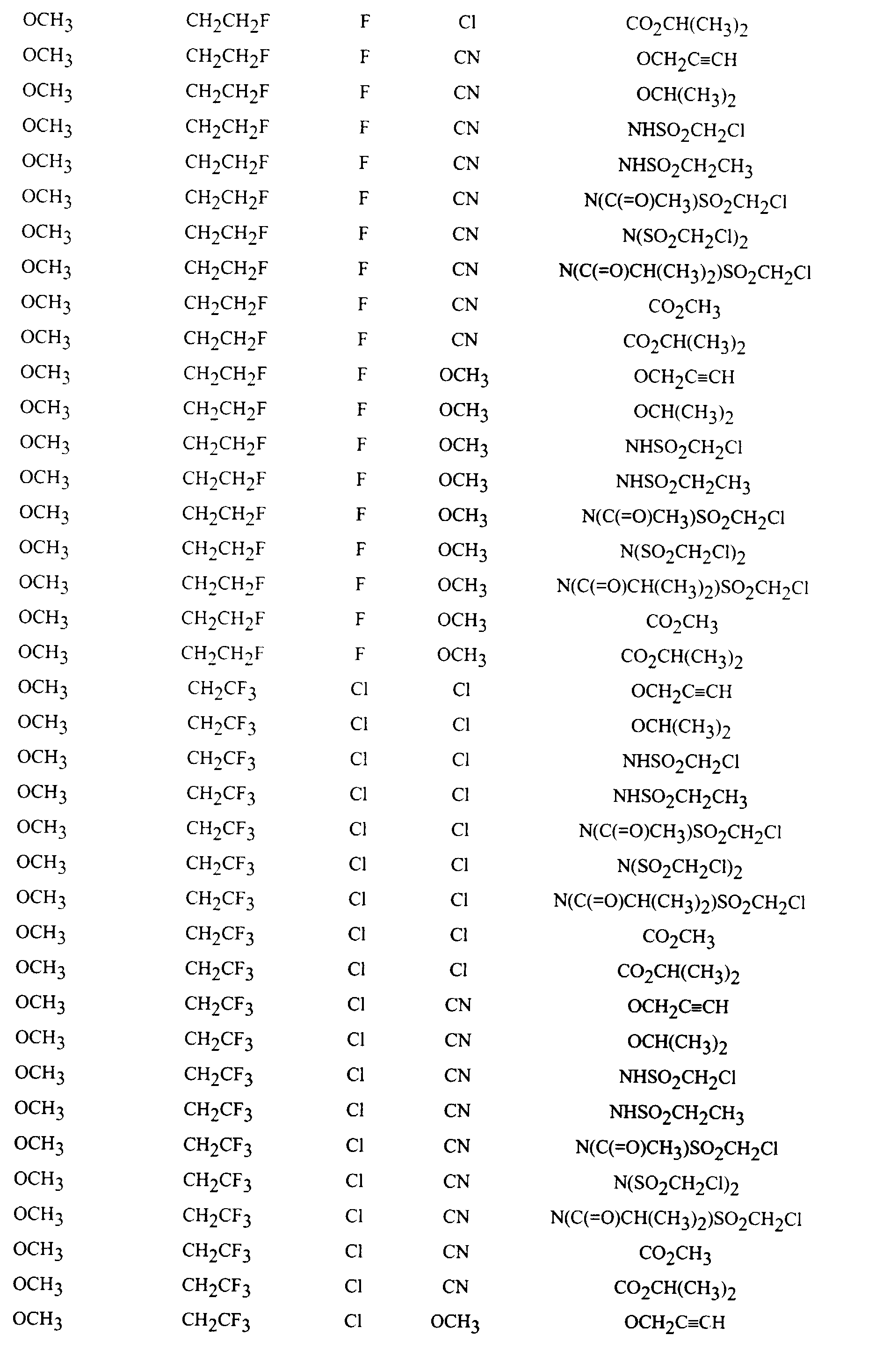

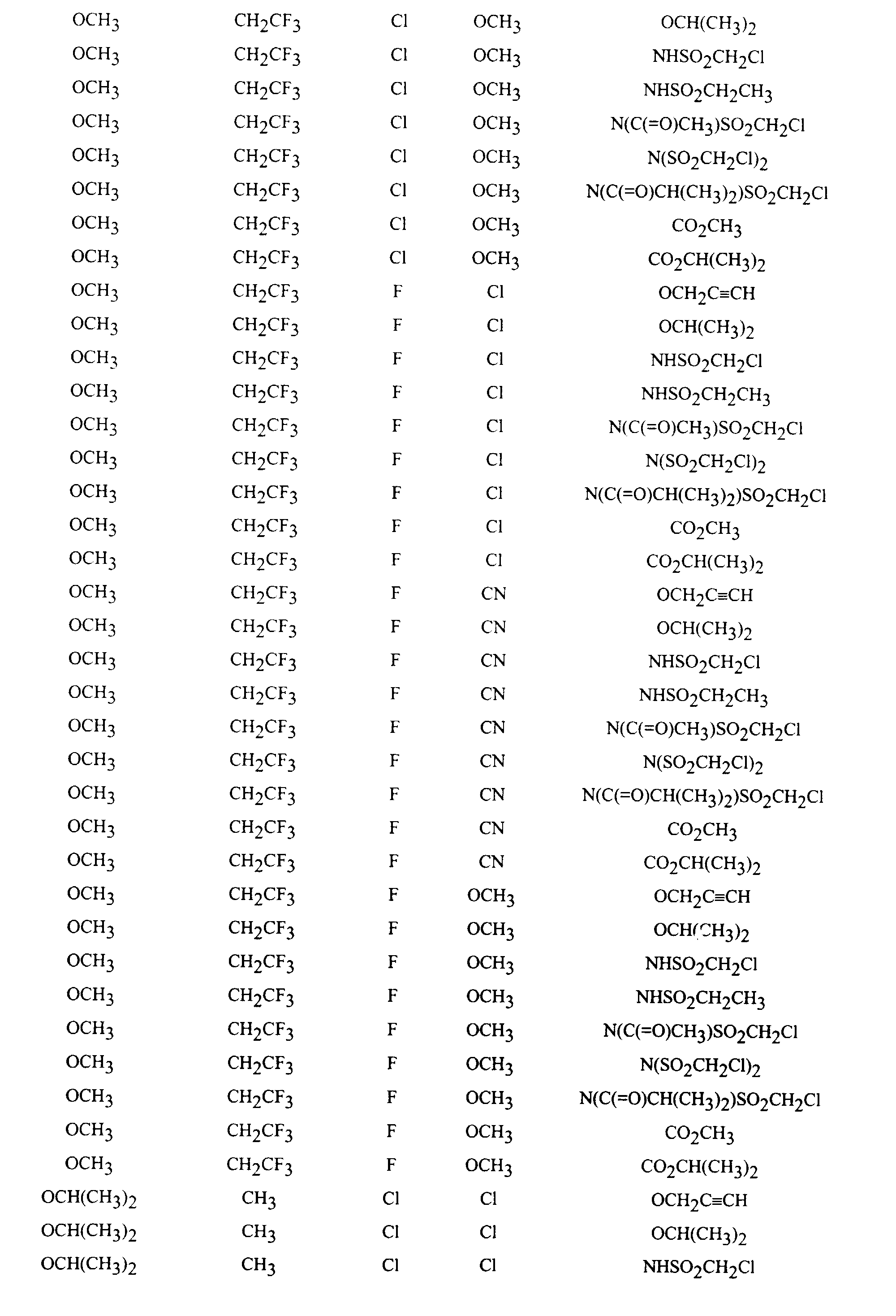

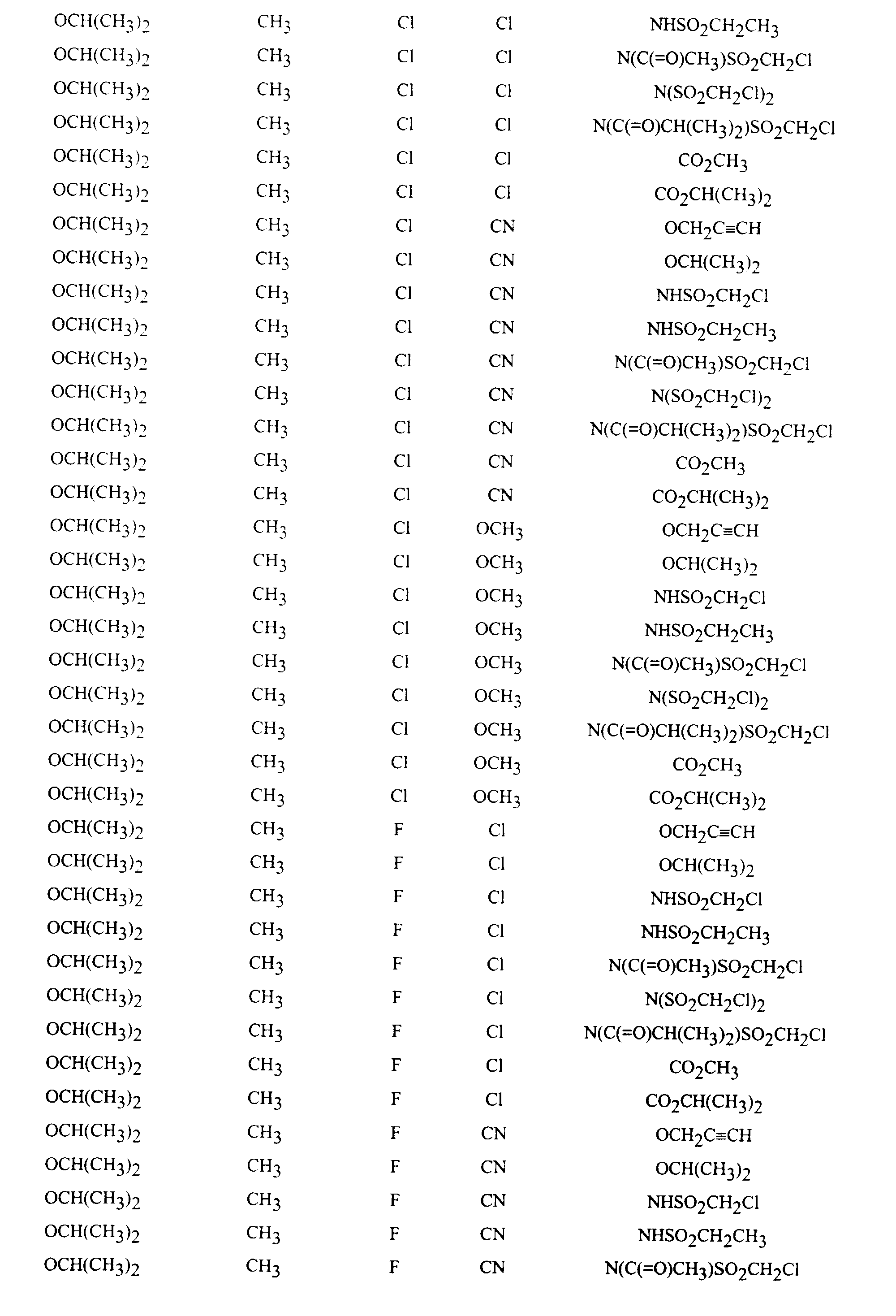

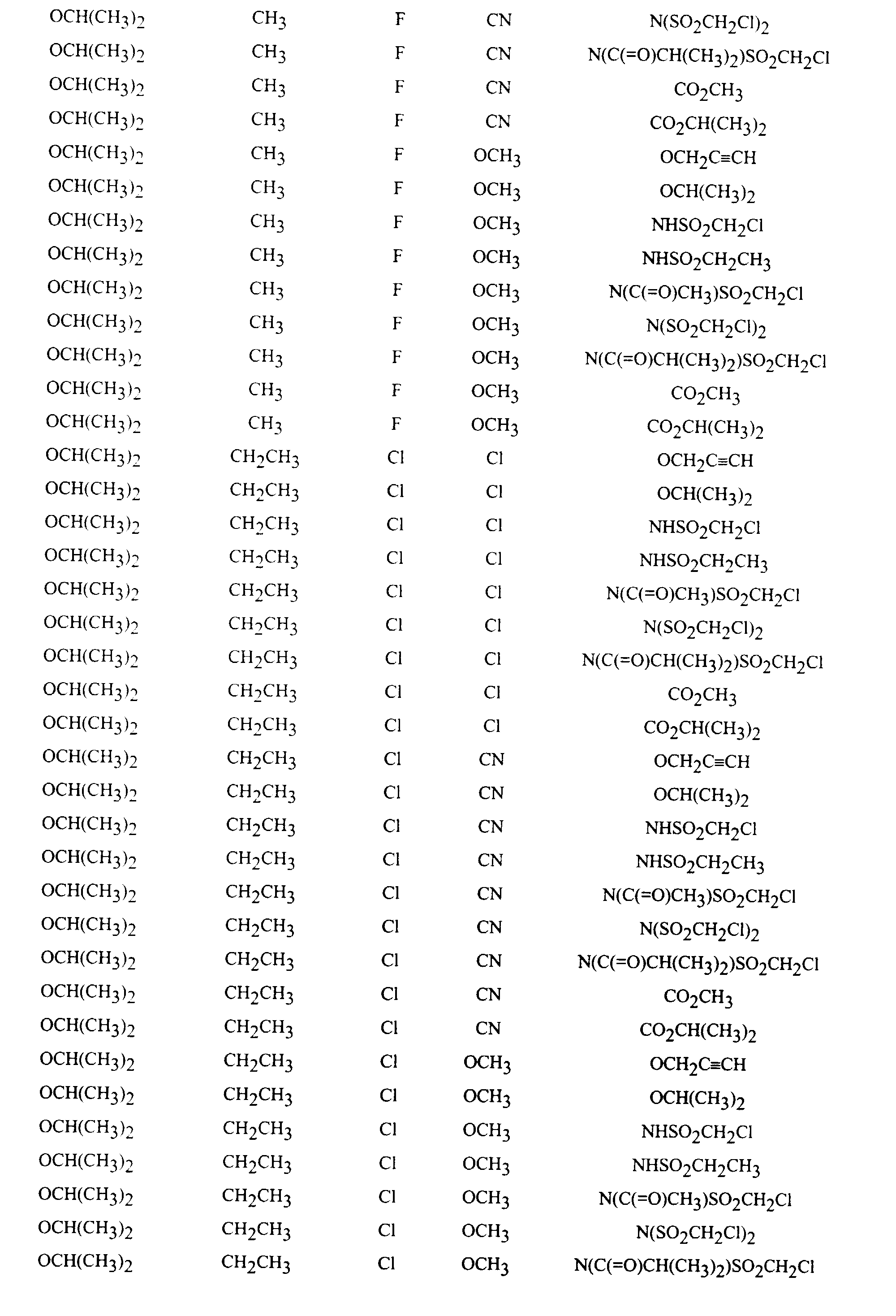

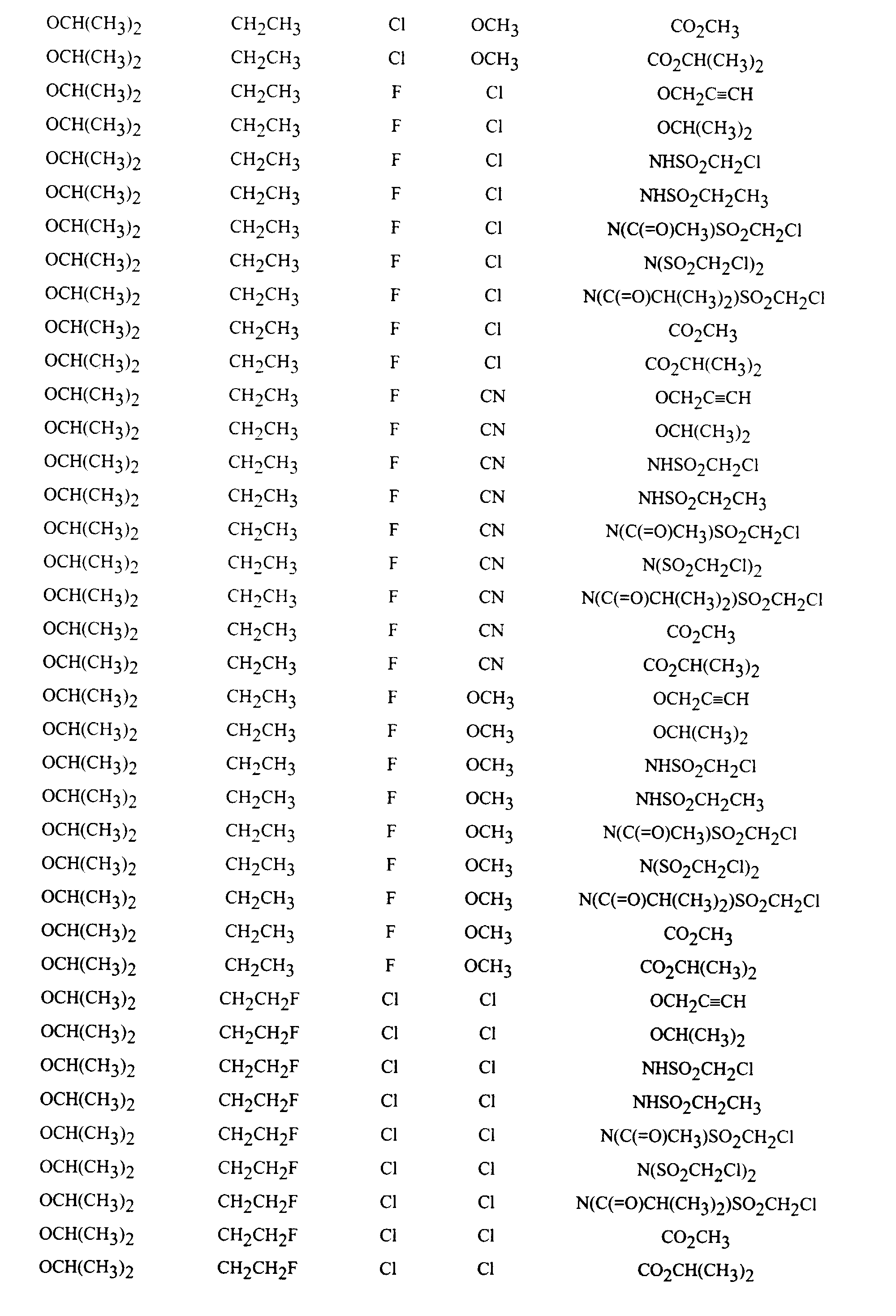

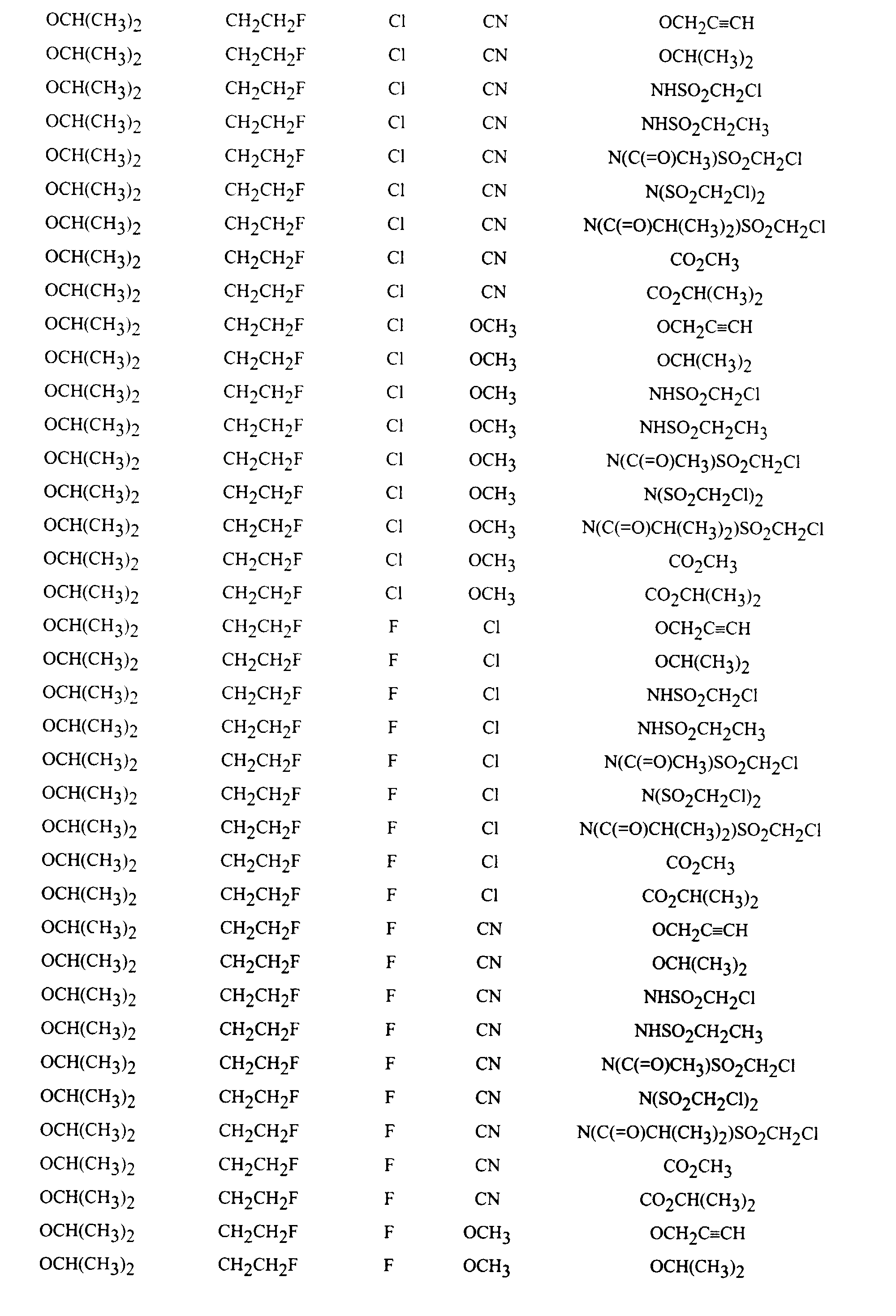

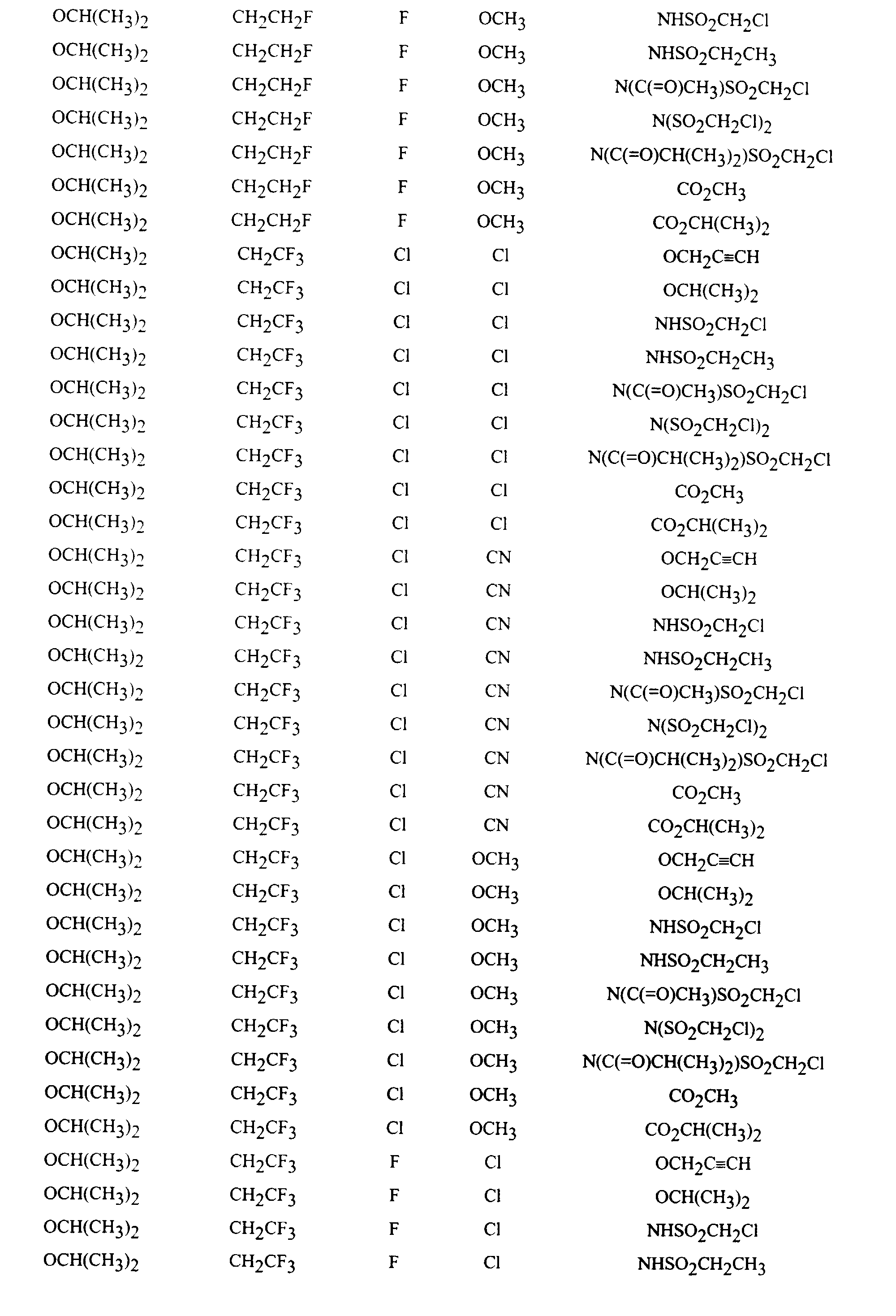

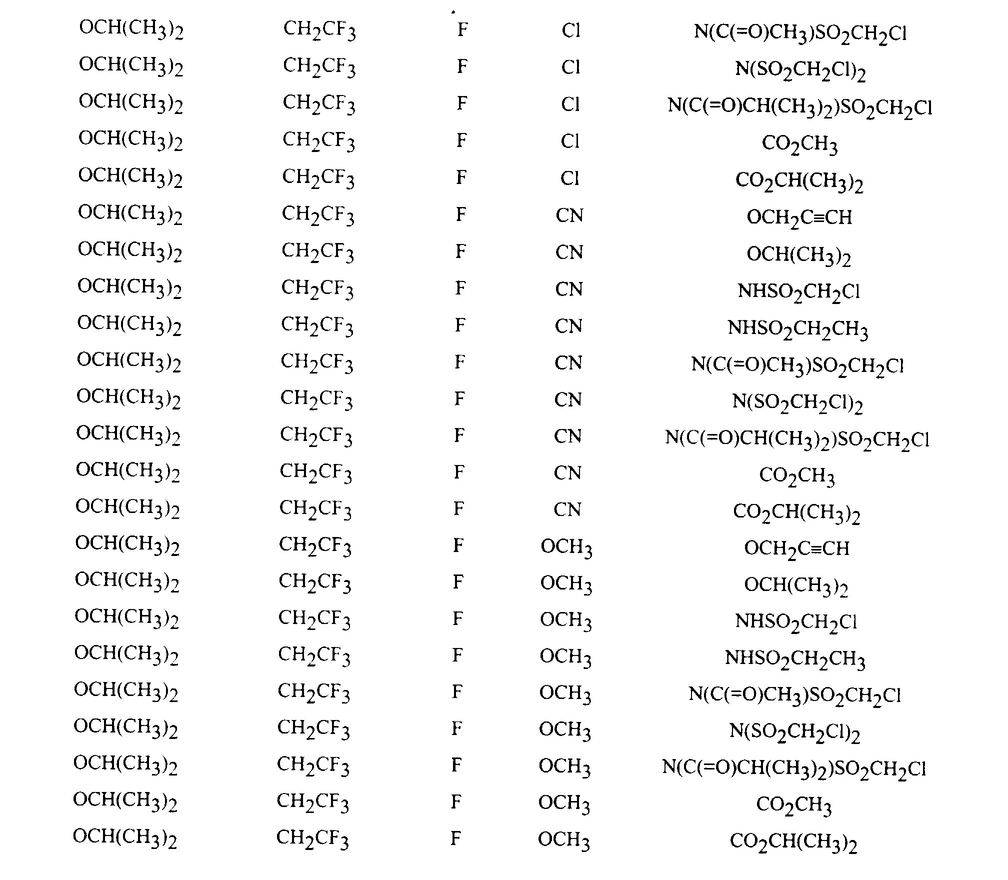

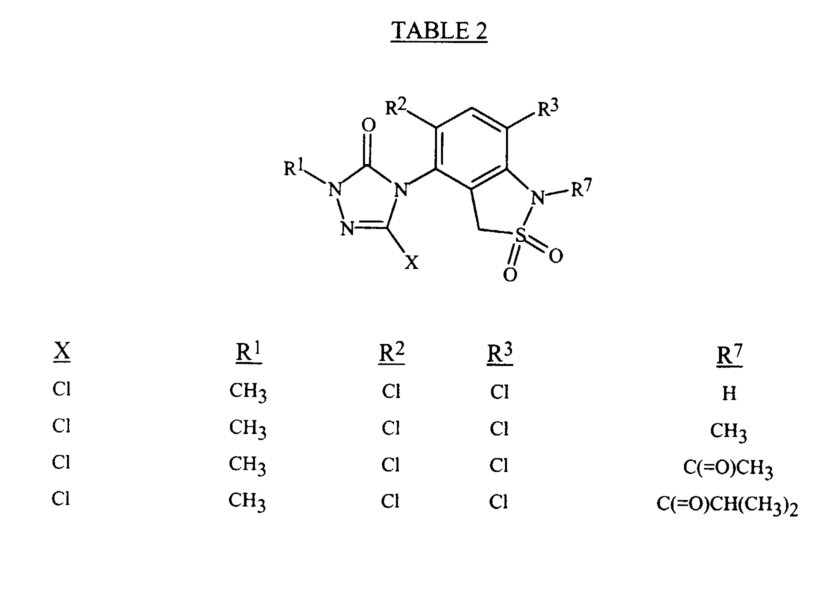

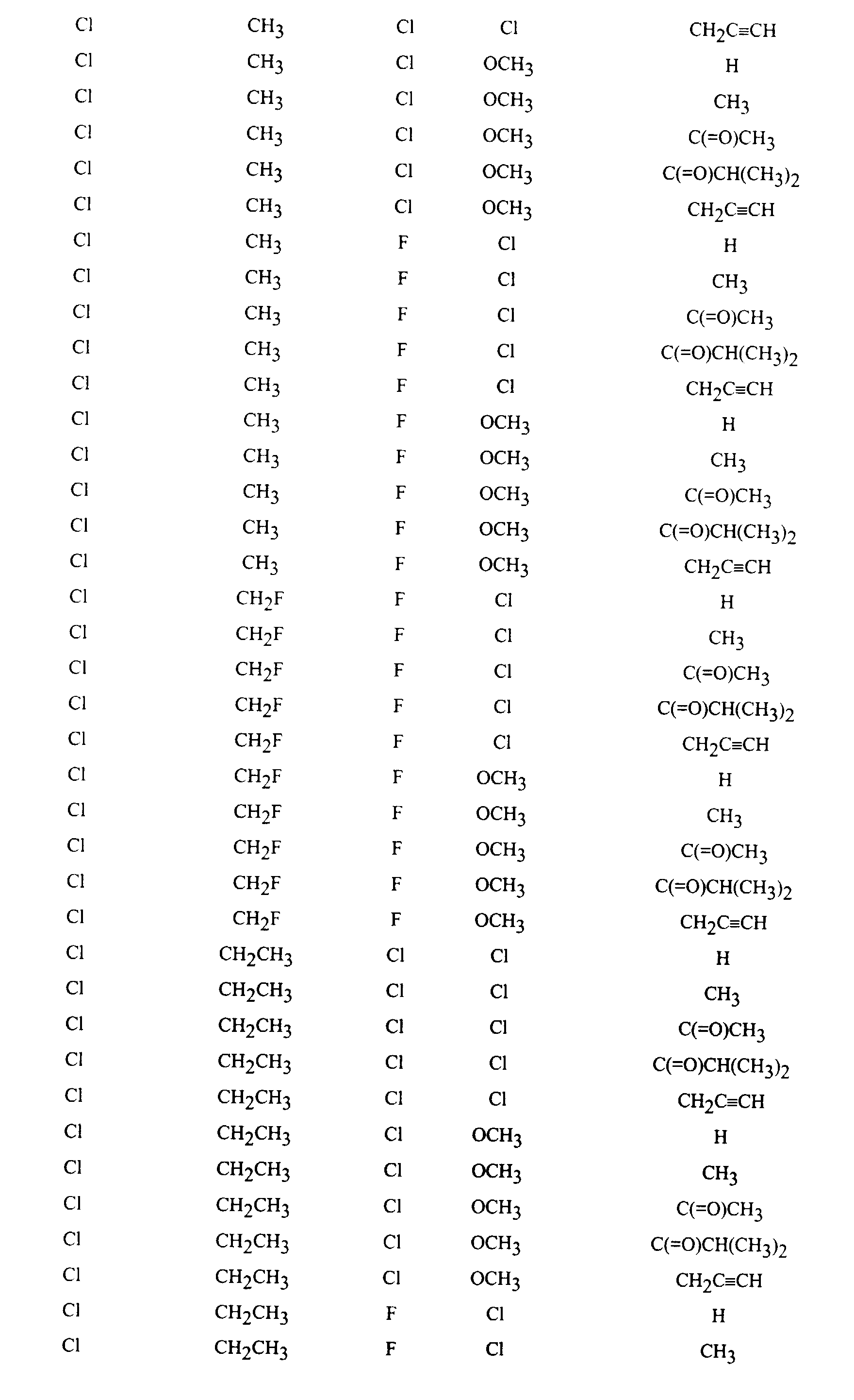

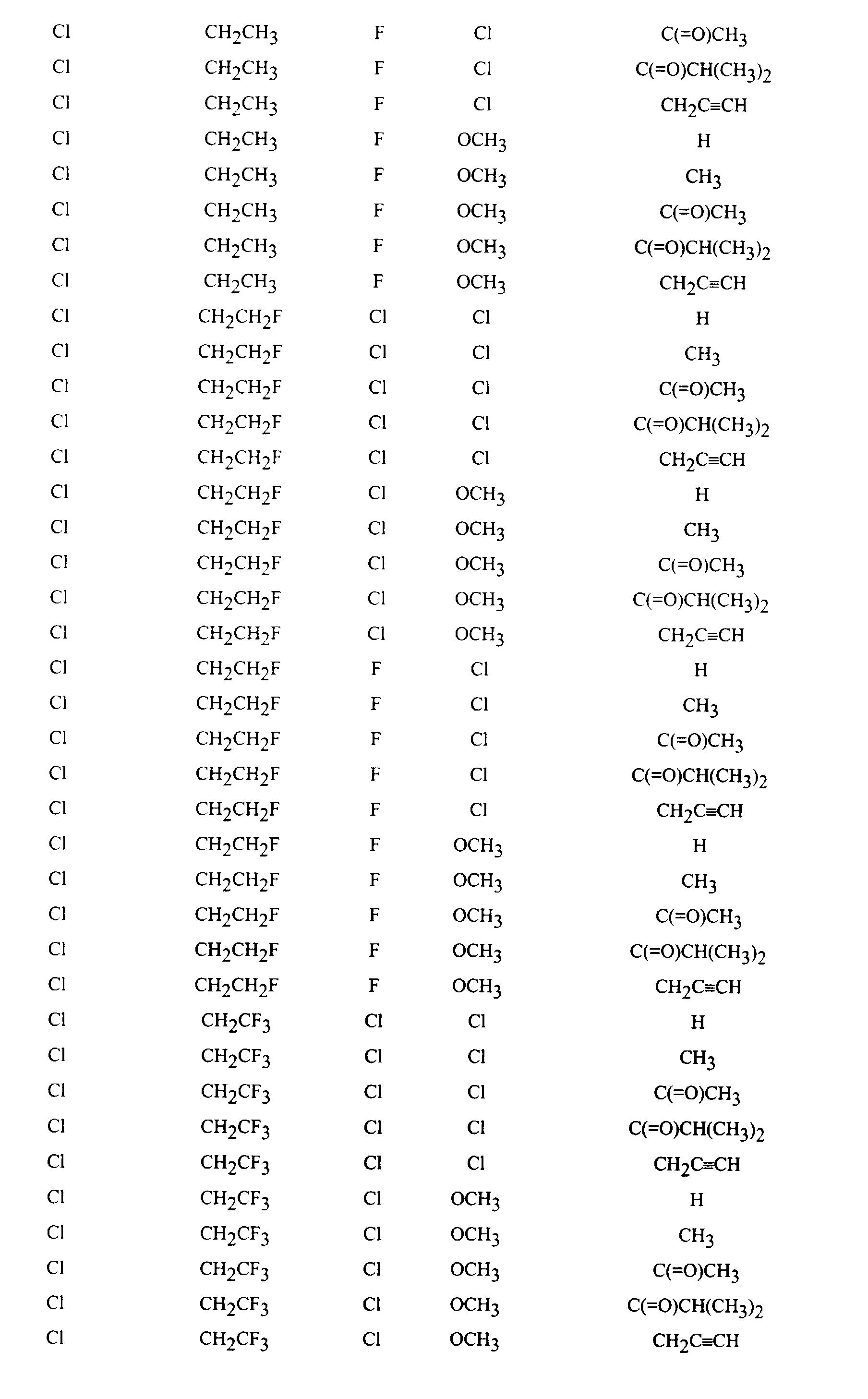

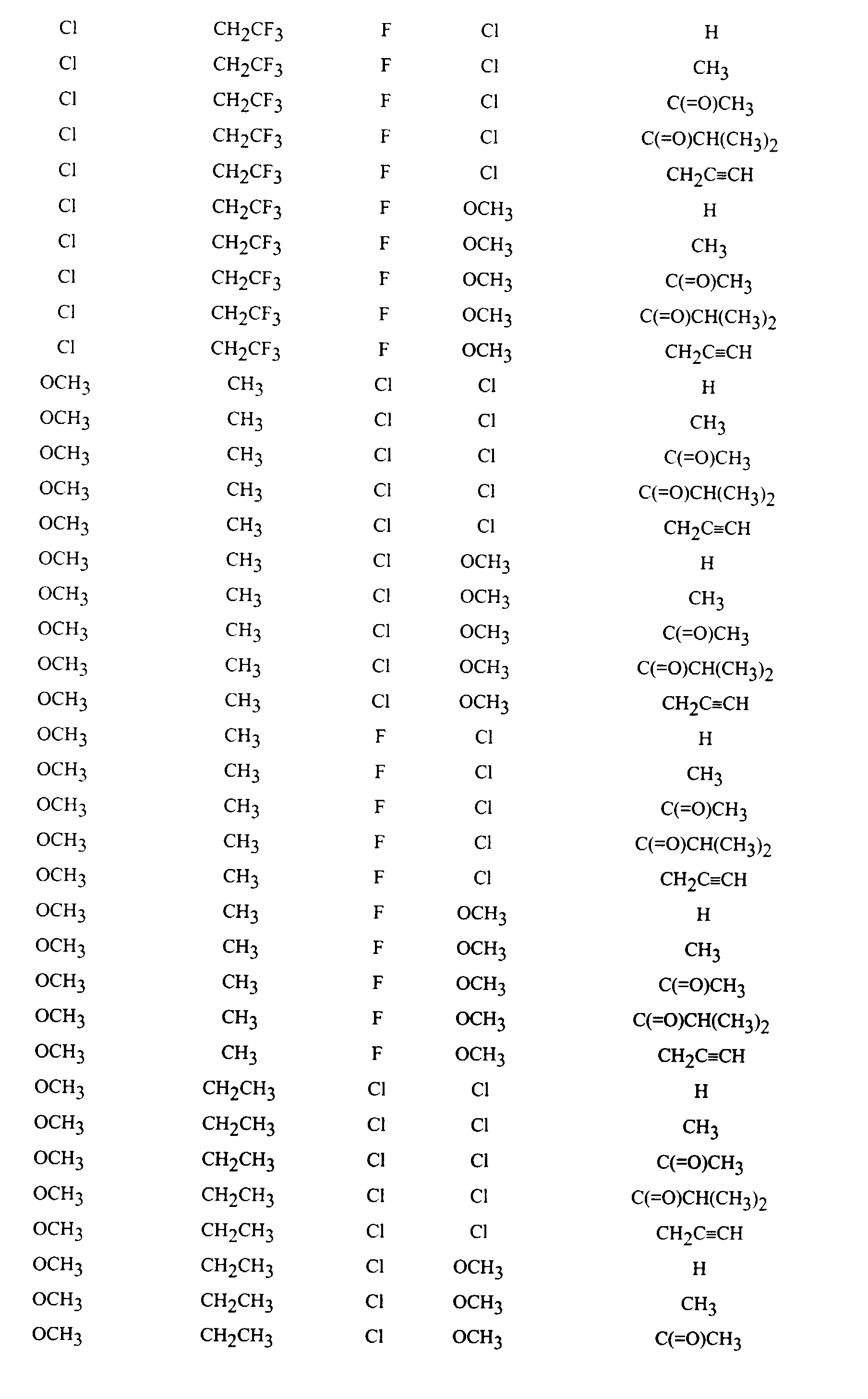

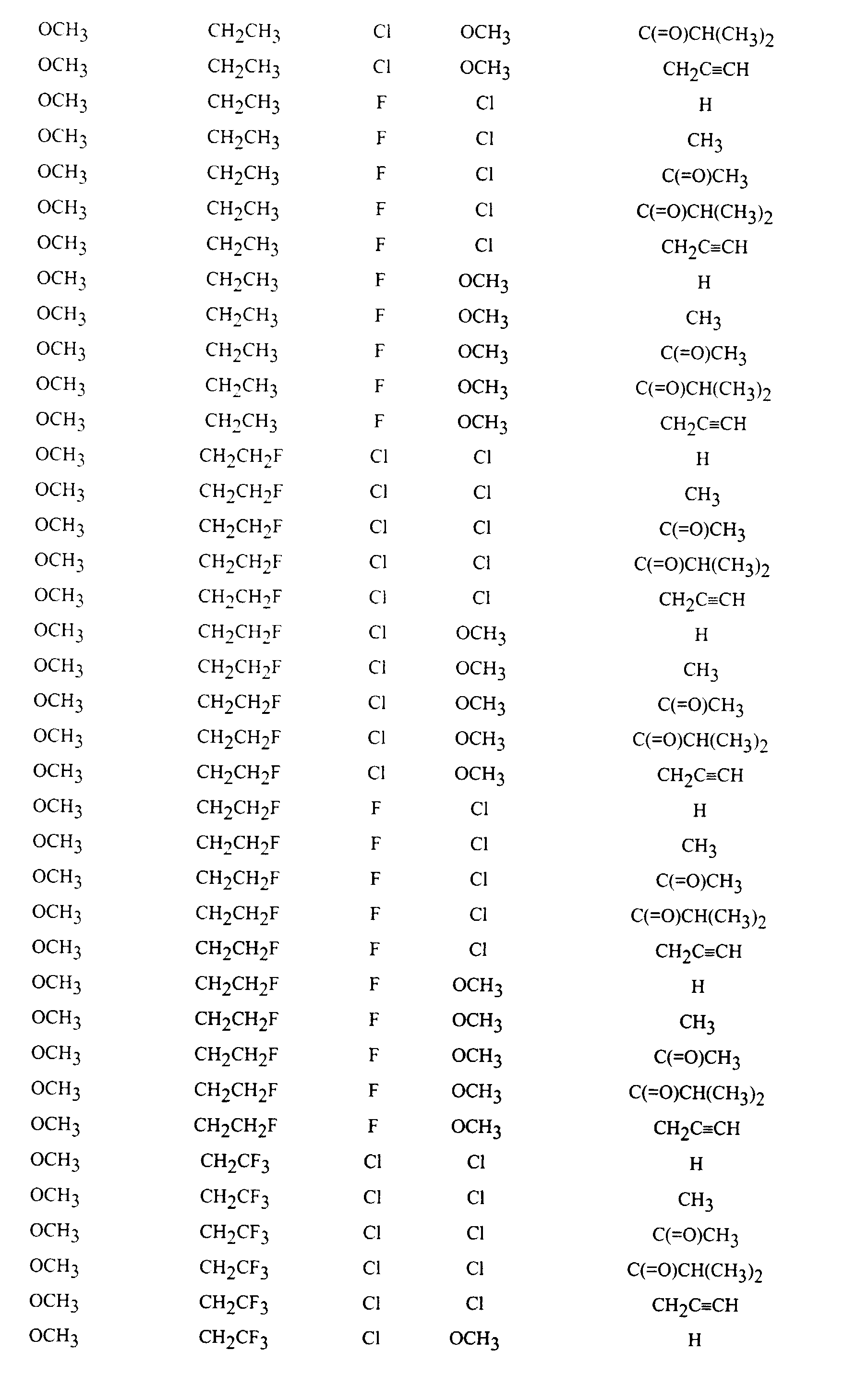

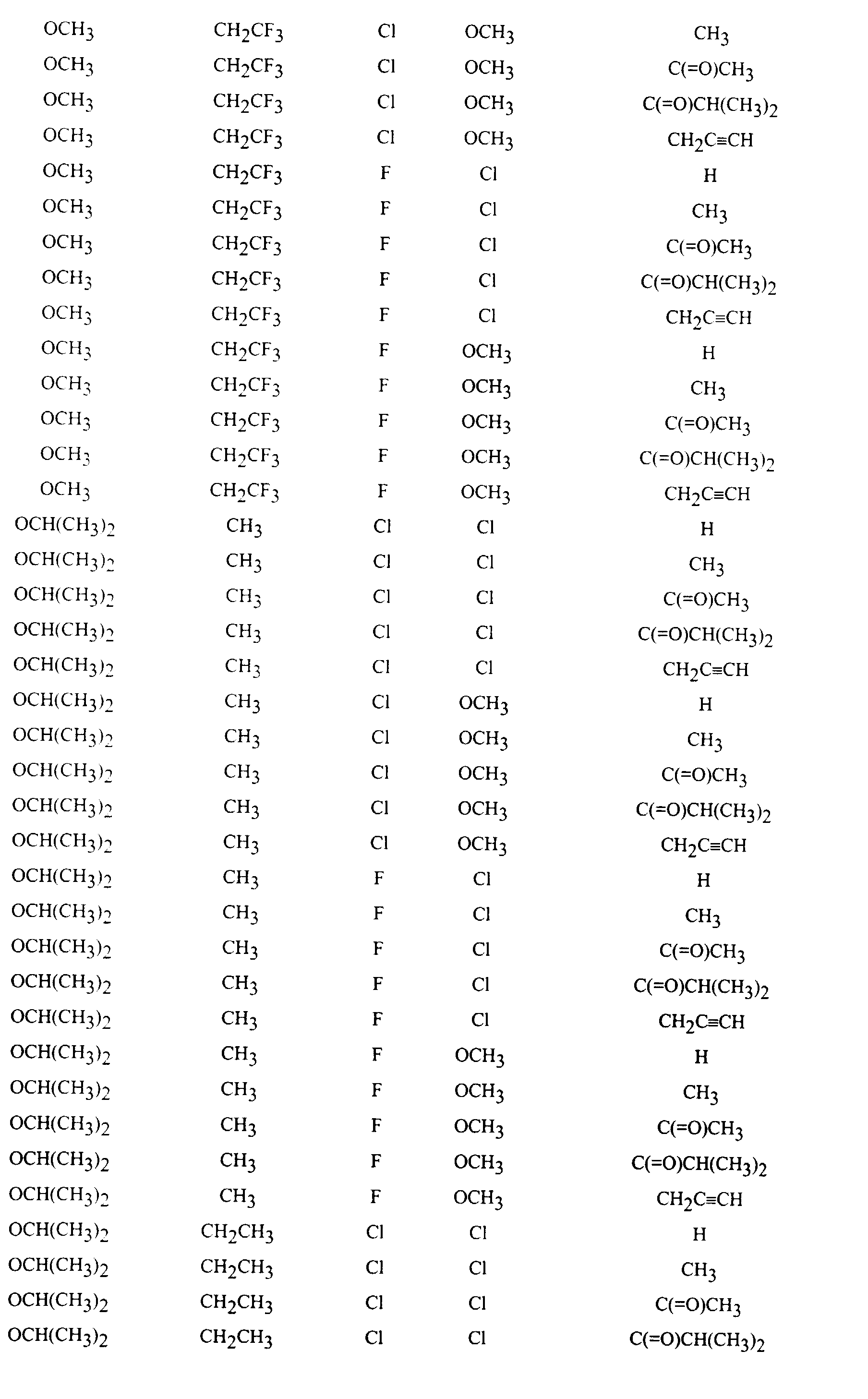

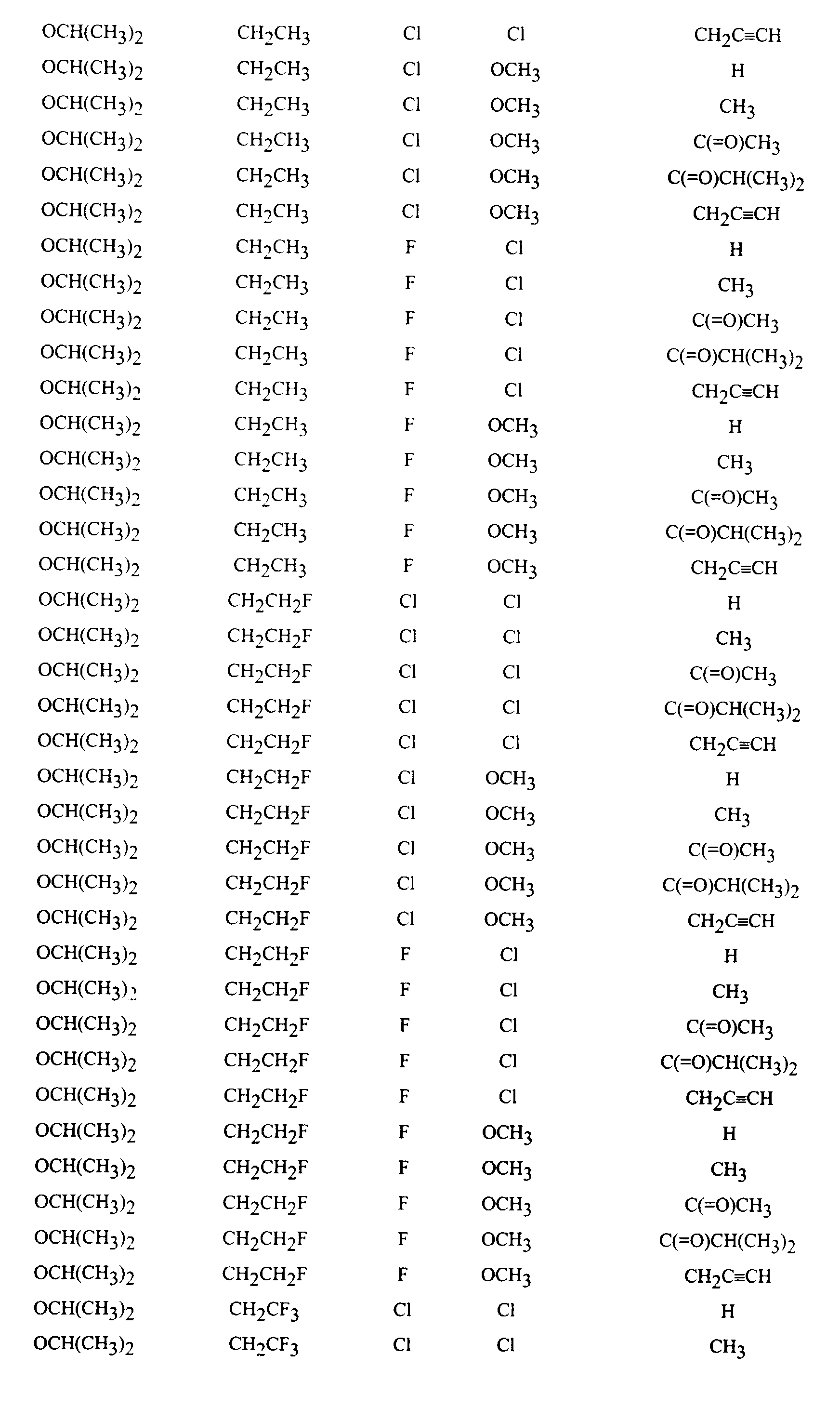

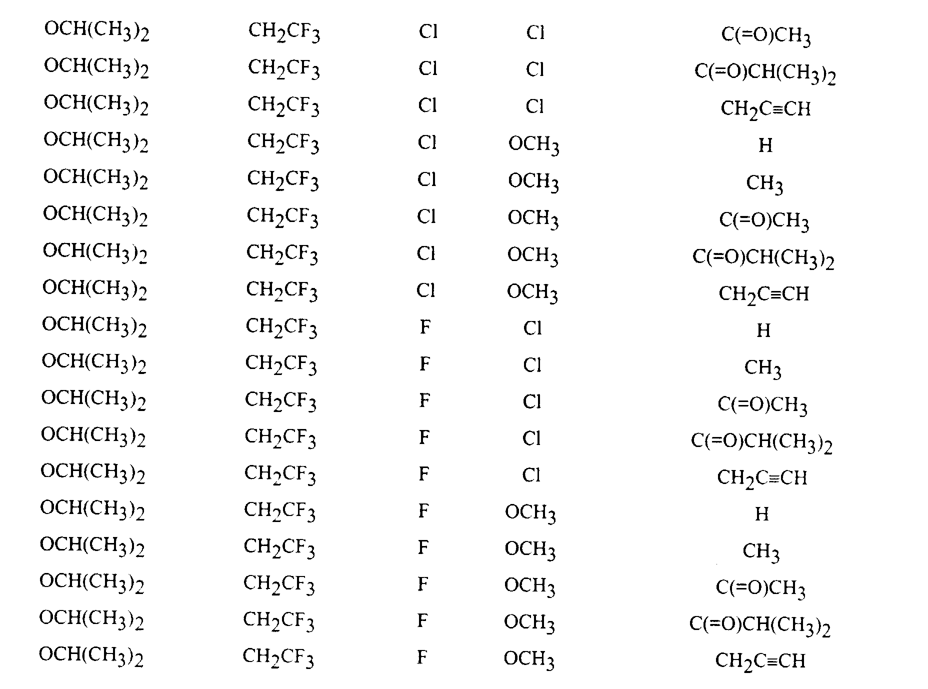

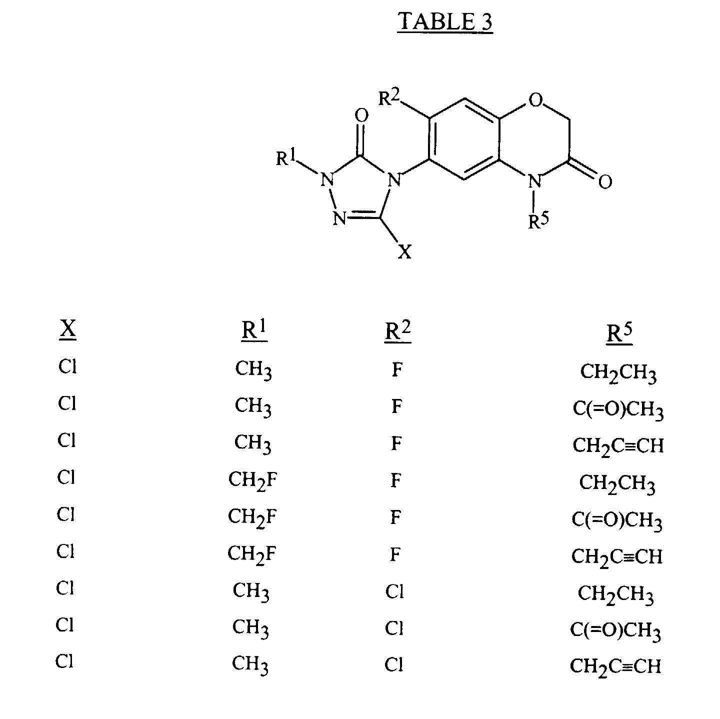

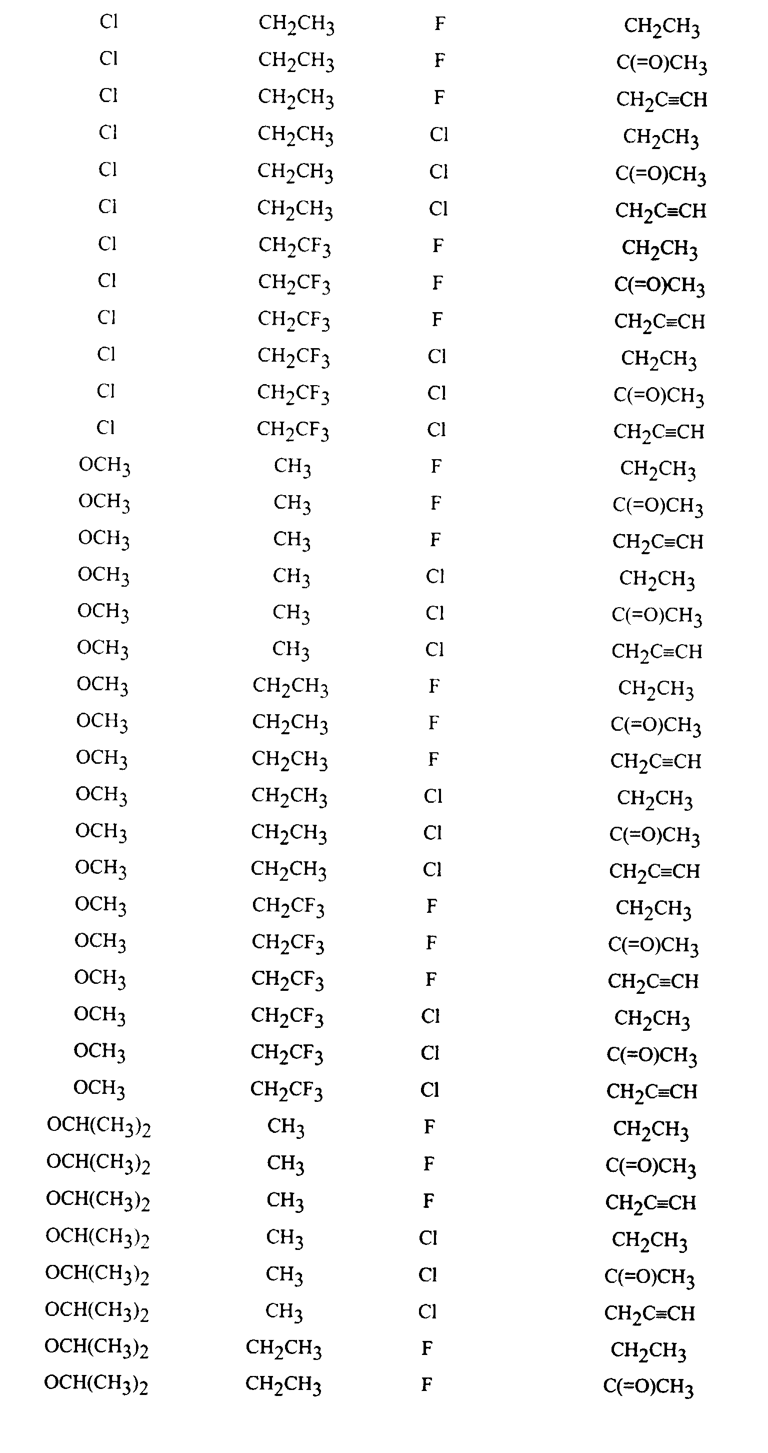

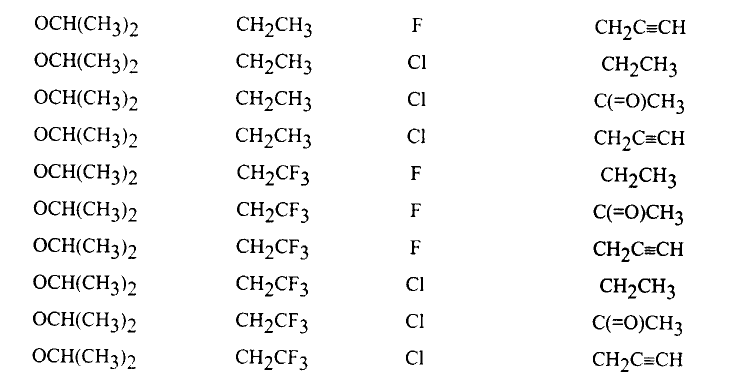

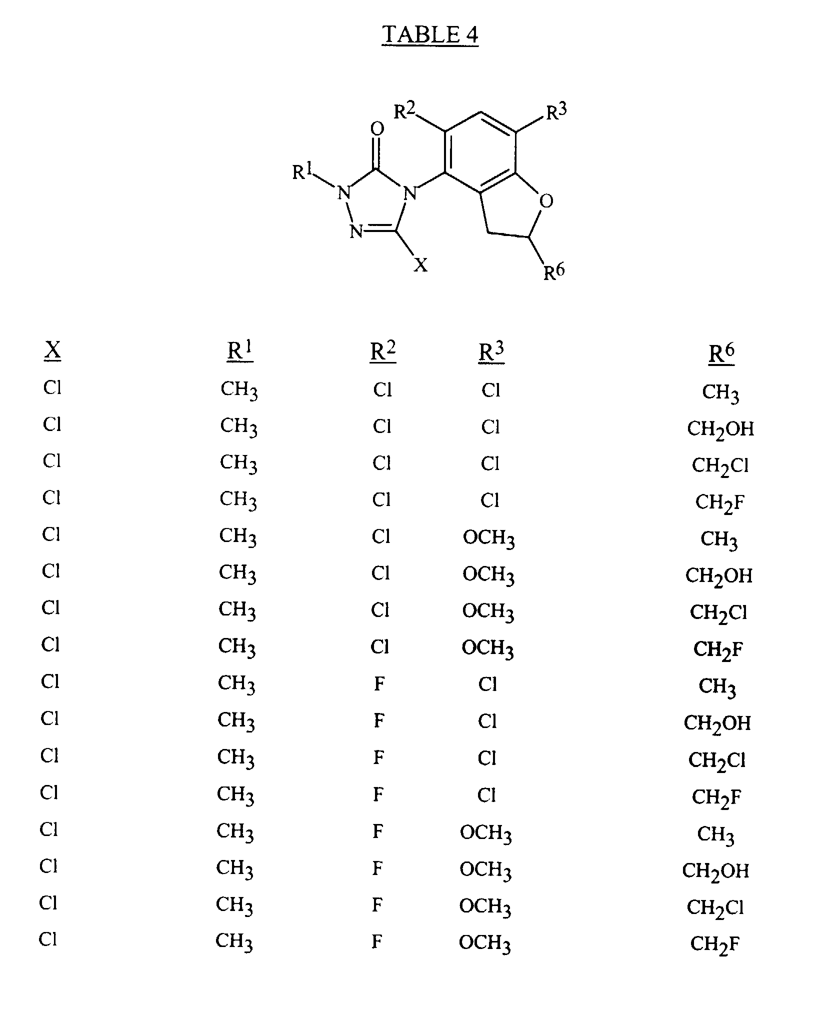

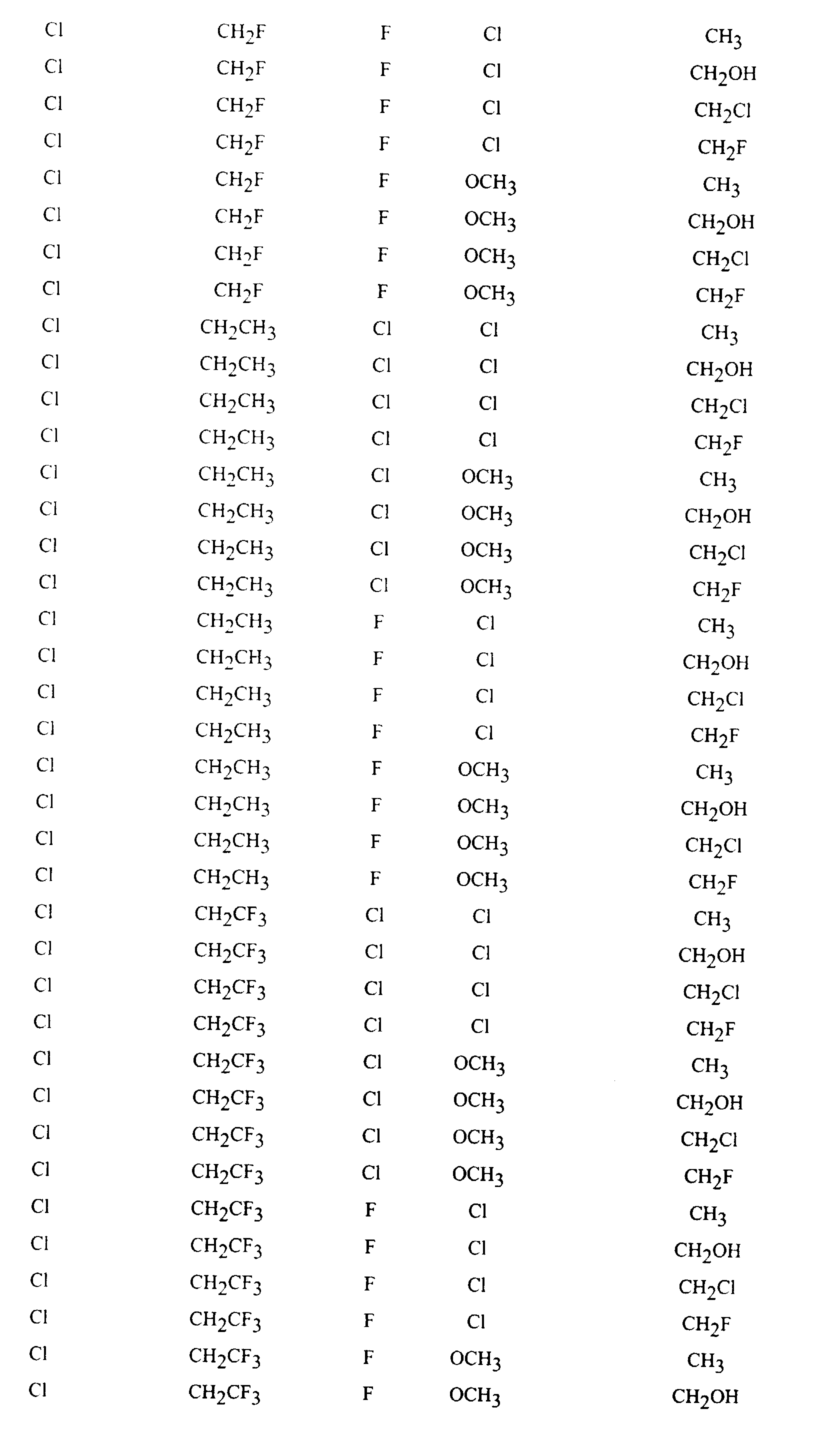

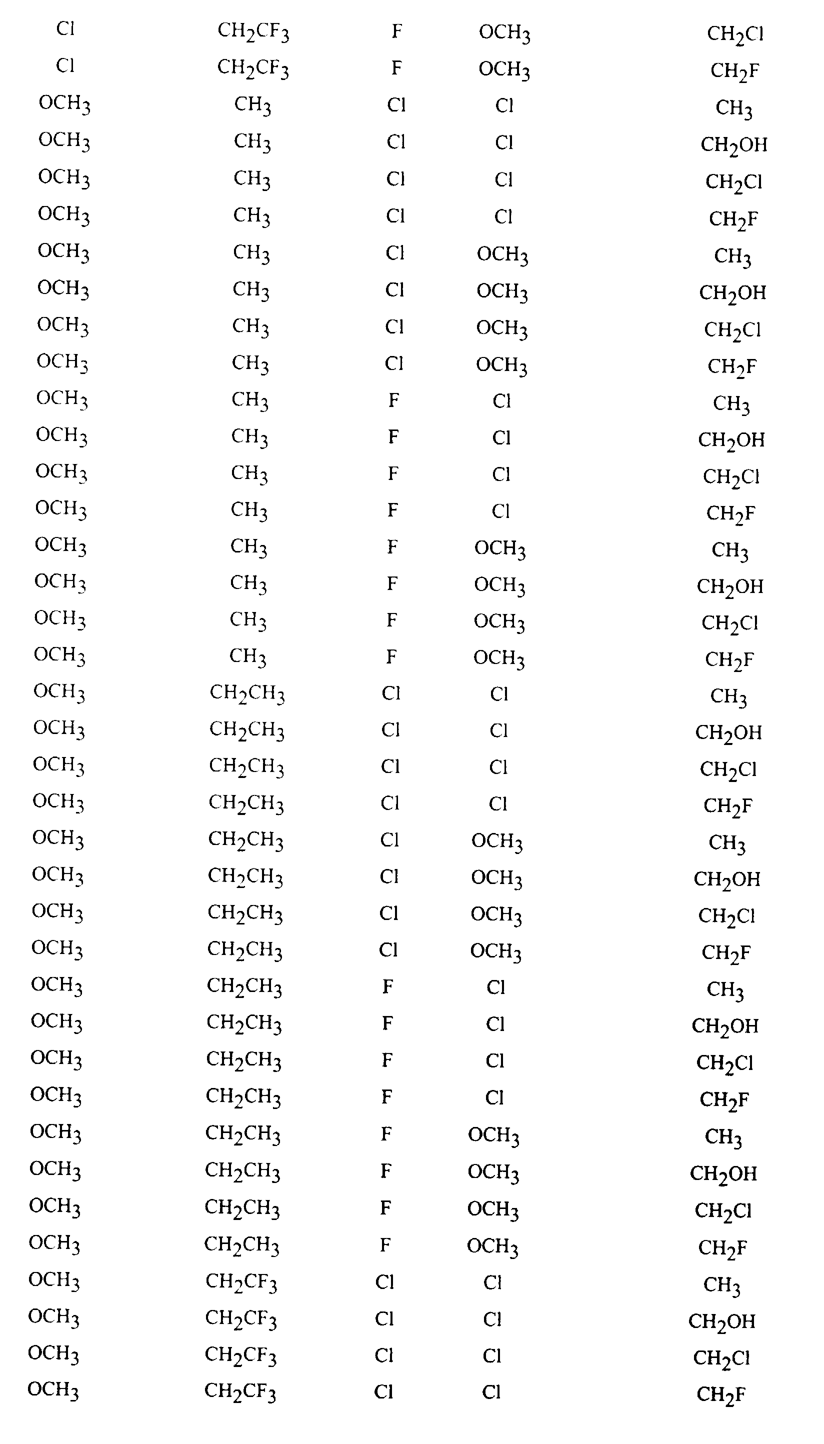

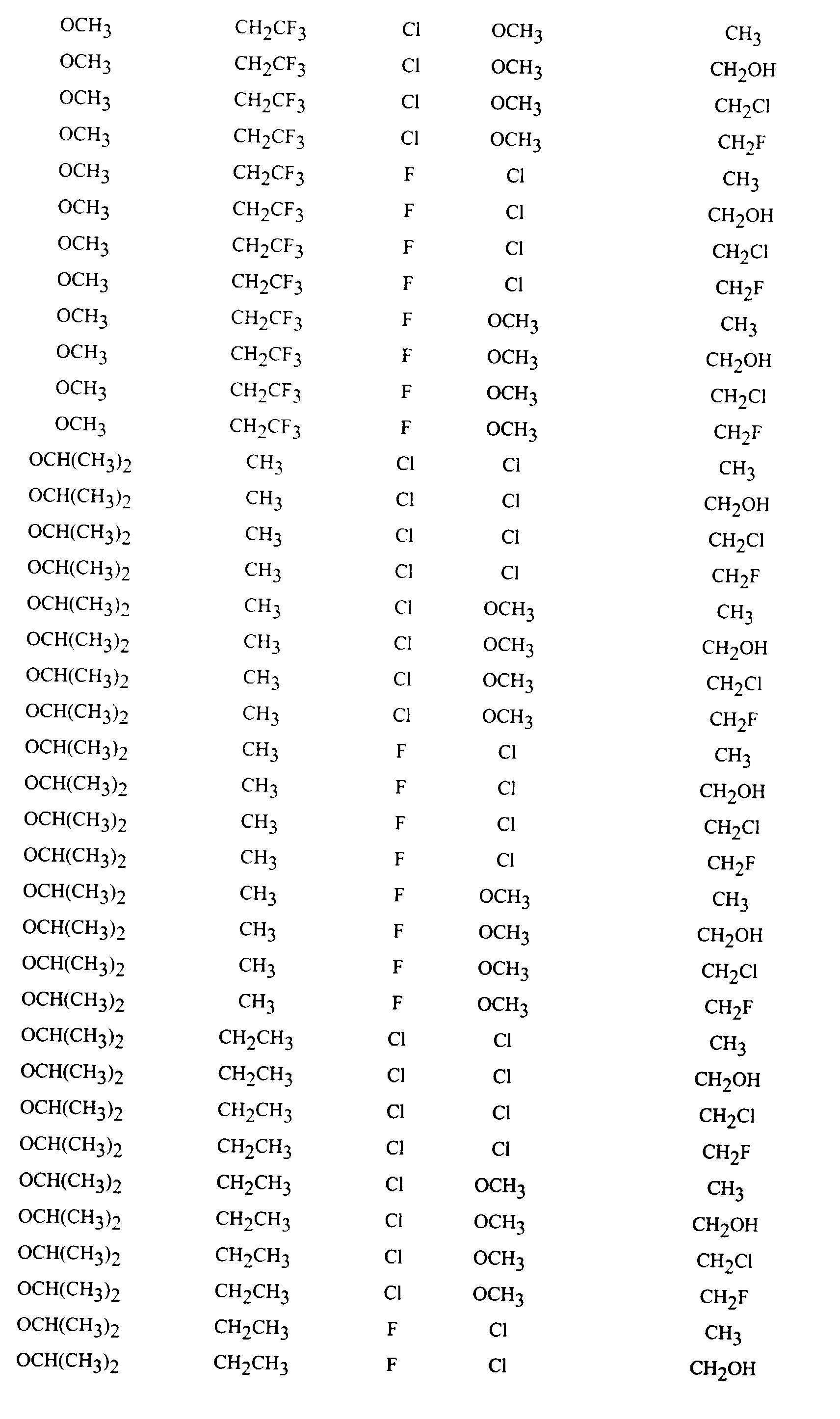

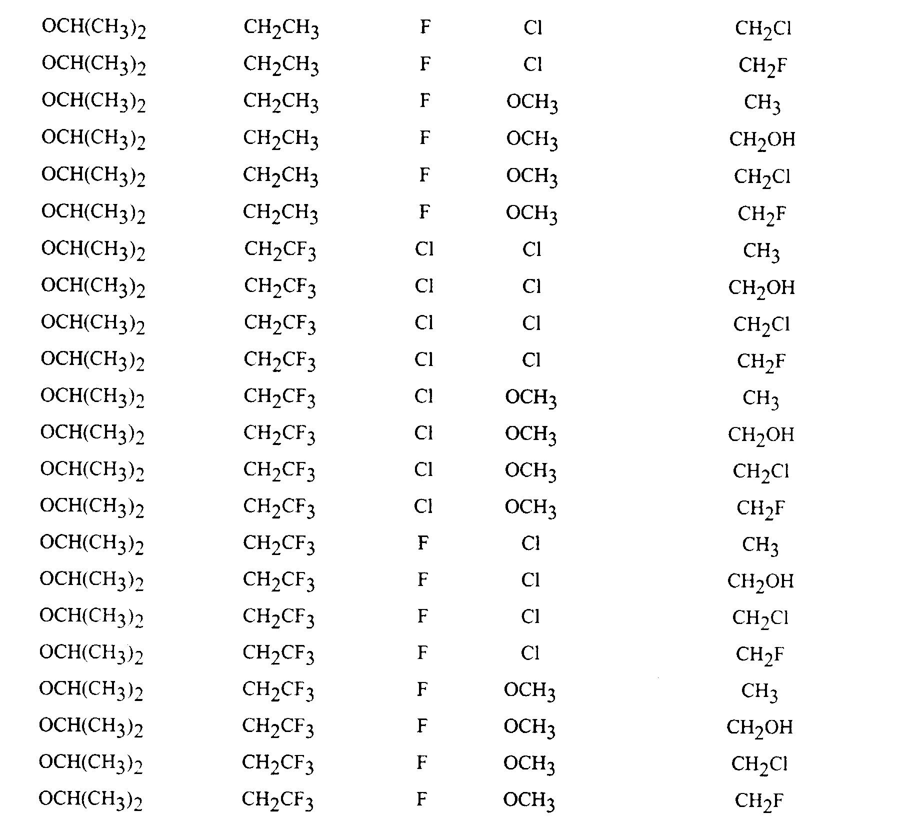

### Formulation/Utility

Compounds of this invention will generally be used as a formulation or composition with an agriculturally suitable carrier comprising at least one of a liquid diluent, a solid diluent or a surfactant. The formulation or composition ingredients are selected to be consistent with the physical properties of the active ingredient, mode of application and environmental factors such as soil type, moisture and temperature. Useful formulations include liquids such as solutions (including emulsifiable concentrates), suspensions, emulsions (including microemulsions and/or suspoemulsions) and the like which optionally can be thickened into gels. Useful formulations further include solids such as dusts, powders, granules, pellets, tablets, films, and the like which can be water-dispersible ("wettable") or water-soluble. Active ingredient can be (micro)encapsulated and further formed into a suspension or solid formulation; alternatively the entire formulation of active ingredient can be encapsulated (or "overcoated"). Encapsulation can control or delay release of the active ingredient. Sprayable formulations can be extended in suitable media and used at spray volumes from about one to several hundred liters per hectare. High-strength compositions are primarily used as intermediates for further formulation.

The formulations will typically contain effective amounts of active ingredient, diluent and surfactant within the following approximate ranges which add up to 100 percent by weight.

| | Weight Percent | | |
|---|---|---|---|
| | Active Ingredient | Diluent | Surfactant |
| Water-Dispersible and Water-soluble | 5-90 | 0-94 | 1-15 |
| Granules, Tablets and Powders. | | | |
| | | | |
| Suspensions. Emulsions. Solutions | 5-50 | 40-95 | 0-15 |
| (including Emulsifiable Concentrates) | | | |
| | | | |
| Dusts | 1-25 | 70-99 | 0-5 |
| Granules and Pellets | 0.01-99 | 5-99.99 | 0-15 |
| | | | |
| High Strength Compositions | 90-99 | 0-10 | 0-2 |

Typical solid diluents are described in Watkins, et al., *Handbook of Insecticide Dust Diluents and Carriers,* 2nd Ed., Dorland Books, Caldwell, New Jersey. Typical liquid diluents are described in Marsden, *Solvents Guide,* 2nd Ed., Interscience, New York, 1950. *McCutcheon 's Detergents and Emulsifiers Annual,* Allured Publ. Corp., Ridgewood, New Jersey, as well as Sisely and Wood, *Encyclopedia of Surface Active Agents,* Chemical Publ. Co., Inc., New York, 1964, list surfactants and recommended uses. All formulations can contain minor amounts of additives to reduce foam, caking, corrosion, microbiological growth and the like, or thickeners to increase viscosity.

Surfactants include, for example, polyethoxylated alcohols, polyethoxylated alkylphenols, polyethoxylated sorbitan fatty acid esters, dialkyl sulfosuccinates, alkyl sulfates, alkylbenzene sulfonates, organosilicones, *N,N*-dialkyltaurates, lignin sulfonates, naphthalene sulfonate formaldehyde condensates, polycarboxylates, and polyoxyethylene/polyoxypropylene block copolymers. Solid diluents include, for example, clays such as bentonite, montmorillonite, attapulgite and kaolin, starch, sugar, silica, talc, diatomaceous earth, urea, calcium carbonate, sodium carbonate and bicarbonate, and sodium sulfate. Liquid diluents include, for example, water, *N,N*-dimethylformamide, dimethyl sulfoxide, *N*-alkylpyrrolidone, ethylene glycol, polypropylene glycol, paraffins, alkylbenzenes, alkylnaphthalenes, oils of olive, castor, linseed, tung, sesame, corn, peanut, cotton-seed, soybean, rape-seed and coconut, fatty acid esters, ketones such as cyclohexanone, 2-heptanone, isophorone and 4-hydroxy-4-methyl-2-pentanone, and alcohols such as methanol, cyclohexanol, decanol and tetrahydrofurfuryl alcohol.

Solutions, including emulsifiable concentrates, can be prepared by simply mixing the ingredients. Dusts and powders can be prepared by blending and, usually, grinding as in a hammer mill or fluid-energy mill. Suspensions are usually prepared by wet-milling; see, for example, U.S. 3,060,084. Granules and pellets can be prepared by spraying the active material upon preformed granular carriers or by agglomeration techniques. See Browning, "Agglomeration", *Chemical Engineering,* December 4, 1967, pp 147-48, *Perry's Chemical Engineer's Handbook,* 4th Ed., McGraw-Hill, New York, 1963, pages 8-57 and following, and WO 91/13546. Pellets can be prepared as described in U.S. 4,172,714.
Water-dispersible and water-soluble granules can be prepared as taught in U.S. 4,144,050, U.S. 3,920,442 and DE 3,246,493. Tablets can be prepared as taught in U.S. 5,180,587, U.S. 5,232,701 and U.S. 5,208,030. Films can be prepared as taught in GB 2,095,558 and U.S. 3,299,566.

For further information regarding the art of formulation, see U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, *Weed Control as a Science,* John Wiley and Sons, Inc., New York, 1961, pp 81-96; and Hance et al., *Weed Control Handbook,* 8th Ed., Blackwell Scientific Publications, Oxford, 1989.

In the following Examples, all percentages are by weight and all formulations are prepared in conventional ways. Compound numbers refer to compounds in Index Tables A-C.

| Example A | |
|---|---|
| High Strength Concentrate | |
| Compound 3 | 98.5% |
| silica aerogel | 0.5% |
| synthetic amorphous fine silica | 1.0%. |

| Example B | |
|---|---|
| Wettable Powder | |
| Compound 5 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0%. |

| Example C | |
|---|---|
| Granule | |
| Compound 7 | 10.0% |
| attapulgite granules (low volatile matter, 0.71/0.30 mm; U.S.S. No. 25-50 sieves) | 90.0%. |

| Example D | |
|---|---|
| Extruded Pellet | |
| Compound 17 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0%. |

Test results indicate that the compounds of the present invention are highly active preemergent and postemergent herbicides or plant growth regulants. Many of them have utility for broad-spectrum pre- and/or postemergence weed control in areas where complete control of all vegetation is desired such as around fuel storage tanks, industrial storage areas, parking lots, drive-in theaters, air fields, river banks, irrigation and other waterways, around billboards and highway and railroad structures. Some of the compounds are useful for the control of selected grass and broadleaf weeds with tolerance to important agronomic crops which include but are not limited to alfalfa, barley, cotton, wheat, rape, sugar beets, corn (maize), sorghum, soybeans, rice, oats, peanuts, vegetables, tomato, potato, perennial plantation crops including coffee, cocoa, oil palm, rubber, sugarcane, citrus, grapes, fruit trees, nut trees, banana, plantain, pineapple, hops, tea and forests such as eucalyptus and conifers (e.g., loblolly pine), and turf species (e.g., Kentucky bluegrass, St. Augustine grass, Kentucky fescue and Bermuda grass). Those skilled in the art will appreciate that not all compounds are equally effective against all weeds. Alternatively, the subject compounds are useful to modify plant growth.

A herbicidally effective amount of the compounds of this invention is determined by a number of factors. These factors include: formulation selected, method of application, amount and type of vegetation present, growing conditions, etc. In general, a herbicidally effective amount of compounds of this invention is 0.001 to 20 kg/ha with a preferred range of 0.004 to 1.0 kg/ha. One skilled in the art can easily determine the herbicidally effective amount necessary for the desired level of weed control.

Compounds of this invention can be used alone or in combination with other commercial herbicides, insecticides or fungicides. Compounds of this invention can also be used in combination with commercial herbicide safeners such as benoxacor, dichlormid and furilazole to increase safety to certain crops. A mixture of one or more of the following herbicides with a compound of this invention may be particularly useful for weed control: acetochlor, acifluorfen and its sodium salt, aclonifen, acrolein (2-propenal), alachlor, ametryn, amidosulfuron, amitrole, ammonium sulfamate, anilofos, asulam, atrazine, azafenidin, azimsulfuron, benazolin, benazolin-ethyl, benfluralin, benfuresate, bensulfuron-methyl, bensulide, bentazone, bifenox, bispyribac and its sodium salt, bromacil, bromoxynil, bromoxynil octanoate, butachlor, butralin, butroxydim (ICIA0500), butylate, caloxydim (BAS 620H), carfentrazone-ethyl, chlomethoxyfen, chloramben, chlorbromuron, chloridazon, chlorimuron-ethyl, chlomitrofen, chlorotoluron, chlorpropham, chlorsulfuron, chlorthal-dimethyl, cinmethylin, cinosulfuron, clethodim, clomazone, clopyralid, clopyralid-olamine, cyanazine, cycloate, cyclosulfamuron, 2,4-D and its butotyl, butyl, isoctyl and isopropyl esters and its dimethylammonium, diolamine and trolamine salts, daimuron, dalapon, dalapon-sodium, dazomet, 2,4-DB and its dimethylammonium, potassium and sodium salts, desmedipham, desmetryn, dicamba and its diglycolammonium, dimethylammonium, potassium and sodium salts, dichlobenil, dichlorprop, diclofop-methyl, 2-[4,5-dihydro-4-methyl-4-(1-methylethyl)-5-oxo-1*H*-imidazol-2-yl]-5-methyl-3-pyridinecarboxylic acid (AC 263,222), difenzoquat metilsulfate, diflufenican, dimepiperate, dimethenamid, dimethylarsinic acid and its sodium salt, dinitramine, diphenamid, diquat dibromide, dithiopyr, diuron, DNOC, endothal, EPTC, esprocarb, ethalfluralin, ethametsulfuron-methyl, ethofumesate, ethoxysulfuron, fenoxaprop-ethyl, fenoxaprop-P-ethyl, fenuron, fenuron-TCA, flamprop-methyl, flamprop-M-isopropyl, flamprop-M-methyl, flazasulfuron, fluazifop-butyl, fluazifop-P-butyl, fluchloralin, flumetsulam, flumiclorac-pentyl, flumioxazin, fluometuron, fluoroglycofen-ethyl, flupoxam, flupyrsulfuron-methyl and its sodium salt, fluridone, flurochloridone, fluroxypyr, fluthiacet-methyl, fomesafen, fosamine-ammonium, glufosinate, glufosinate-ammonium, glyphosate, glyphosate-isopropylammonium, glyphosate-sesquisodium, glyphosate-trimesium, halosulfuron-methyl, haloxyfop-etotyl, haloxyfop-methyl, hexazinone, imazamethabenz-methyl, imazamox, imazapyr, imazaquin, imazaquin-ammonium, imazethapyr, imazethapyr-ammonium, imazosulfuron, ioxynil, ioxynil octanoate, ioxynil-sodium, isoproturon, isouron, isoxaben, isoxaflutole, lactofen, lenacil, linuron, maleic hydrazide, MCPA and its dimethylammonium, potassium and sodium salts, MCPA-isoctyl, mecoprop, mecoprop-P, mefenacet, mefluidide, metam-sodium, methabenzthiazuron, methylarsonic acid and its calcium, monoammonium, monosodium and disodium salts, methyl [[[1-[5-[2-chloro-4-(trifluoromethyl)phenoxy]-2-nitrophenyl]-2-methoxyethylidene]amino]oxy]acetate (AKH-7088), methyl 5-[[[[(4,6-dimethyl-2-pyrimidinyl)amino]carbonyl]amino]sulfonyl]-1-(2-pyridinyl)-1*H*-pyrazole-4-carboxylate (NC-330), metobenzuron, metolachlor, metosulam, metoxuron, metribuzin, metsulfuron-methyl, molinate, monolinuron, napropamide, naptalam, neburon, nicosulfuron, norflurazon, oryzalin, oxadiazon, oxasulfuron, oxyfluorfen, paraquat dichloride, pebulate, pendimethalin, pentoxazone (KPP-314), perfluidone, phenmedipham, picloram, picloram-potassium, pretilachlor, primisulfuron-methyl, prometon, prometryn, propachlor, propanil, propaquizafop, propazine, propham, propyzamide, prosulfuron, pyrazolynate, pyrazosulfuron-ethyl, pyridate, pyriminobac-methyl, pyrithiobac, pyrithiobac-sodium, quinclorac, quizalofop-ethyl, quizalofop-P-ethyl, quizalofop-P-tefuryl, rimsulfuron, sethoxydim, siduron, simazine, sulcotrione (ICIA0051), sulfentrazone, sulfometuron-methyl, TCA, TCA-sodium, tebuthiuron, terbacil, terbuthylazine, terbutryn, thenylchlor, thiafluamide (BAY 11390), thifensulfuron-methyl, thiobencarb, tralkoxydim, tri-allate, triasulfuron, triaziflam, tribenuron-methyl, triclopyr, triclopyr-butotyl, triclopyr-triethylammonium, tridiphane, trifluralin, triflusulfuron-methyl, and vernolate.

In certain instances, combinations with other herbicides having a similar spectrum of control but a different mode of action will be particularly advantageous for preventing the development of resistant weeds.

The following Tests demonstrate the control efficacy of the compounds of this invention against specific weeds. The weed control afforded by the compounds is not limited, however, to these species. See Index Tables A-D for compound descriptions. The following abbreviation is used in the Index Tables which follow: Ph = phenyl. The abbreviation "dec" indicates that the compound appeared to decompose on melting. The abbreviation "Ex." stands for "Example" and is followed by a number indicating in which example the compound is prepared.

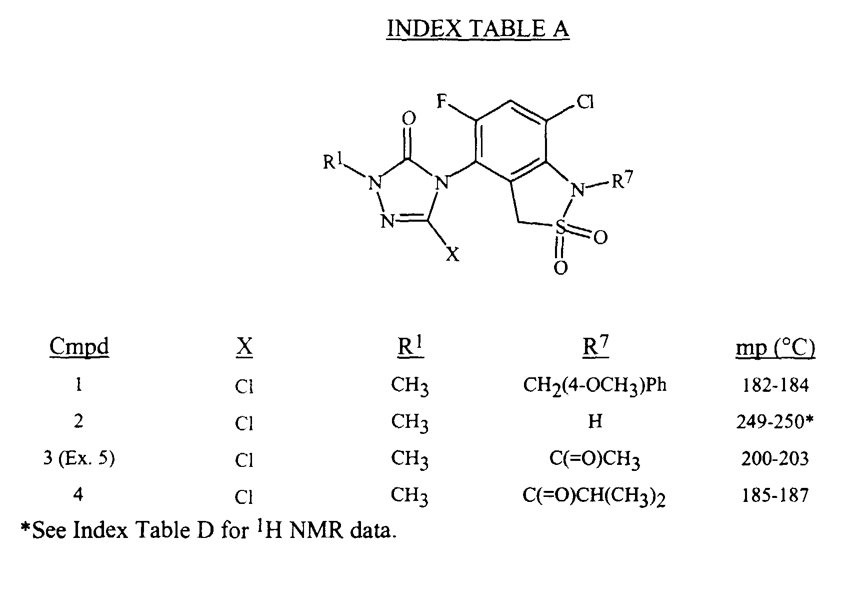

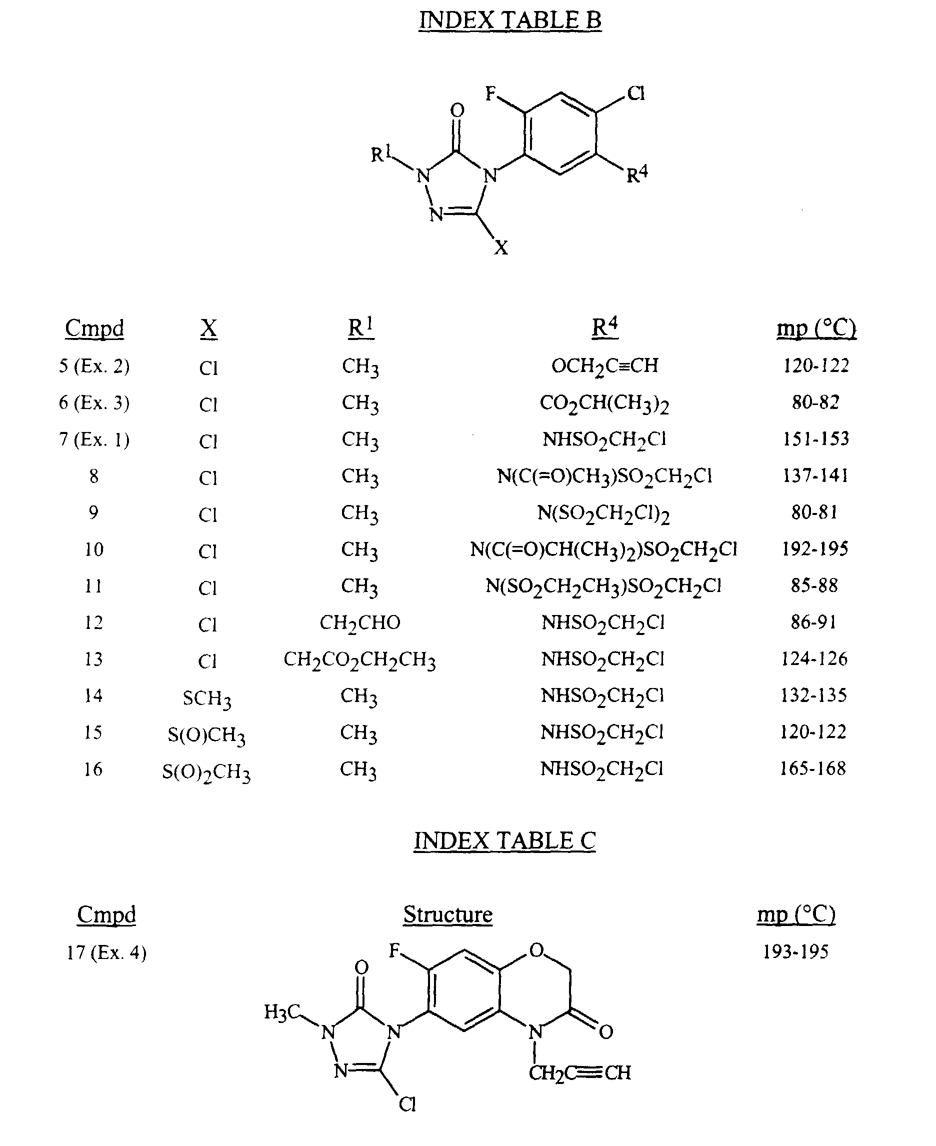

| INDEX TABLE D | |
|---|---|
| Cmpd No. | ¹H NMR Data (((CD₃)₂SO) solution unless indicated otherwise)^{a} |
| 2 | δ 7.70 (s, 1H), 7.38 (d, 1H), 4.62 (q, 2H), 4.04 (q, 2H), 3.48 (s,3H). |

| | |
|---|---|
| a ¹H NMR data are in ppm downfield from tetramethylsilane. Couplings are designated by (s)-singlet, (d)-doublet, (q)-quartet. | |

### BIOLOGICAL EXAMPLES OF THE INVENTION

### Test A

Seeds of barley (*Hordeum vulgare*), barnyardgrass (*Echinochloa crus-galli*), bedstraw (*Galium aparine*), blackgrass (*Alopecurus myosuroides*), chickweed (*Stellaria media*), cocklebur (*Xanthium strumarium*), corn (*Zea mays*), cotton (*Gossypium hirsutum*), crabgrass (*Digitaria sanguinalis*), downy brome (*Bromus tectorum*), giant foxtail (*Setaria faberii*), lambsquarters (*Chenopodium album*), morningglory (*Ipomoea hederacea*), rape *(Brassica napus),* rice *(Oryza sativa),* sorghum *(Sorghum bicolor),* soybean *(Glycine max),* sugar beet *(Beta vulgaris), velvetleaf(Abutilon theophrasti),* wheat *(Triticum aestivum),* wild buckwheat *(Polygonum convolvulus),* wild oat *(Avena fatua)* and purple nutsedge *(Cyperus rotundus)* tubers were planted and treated preemergence with test chemicals formulated in a non-phytotoxic solvent mixture which included a surfactant.

At the same time, these crop and weed species were also treated with postemergence applications of test chemicals formulated in the same manner. Plants ranged in height from 2 to 18 cm (1- to 4-leaf stage) for postemergence treatments. Treated plants and controls were maintained in a greenhouse for twelve to sixteen days, after which all species were compared to controls and visually evaluated. Plant response ratings, summarized in Table A, are based on a scale of 0 to 10 where 0 is no effect and 10 is complete control. A dash (-) response means no test result.

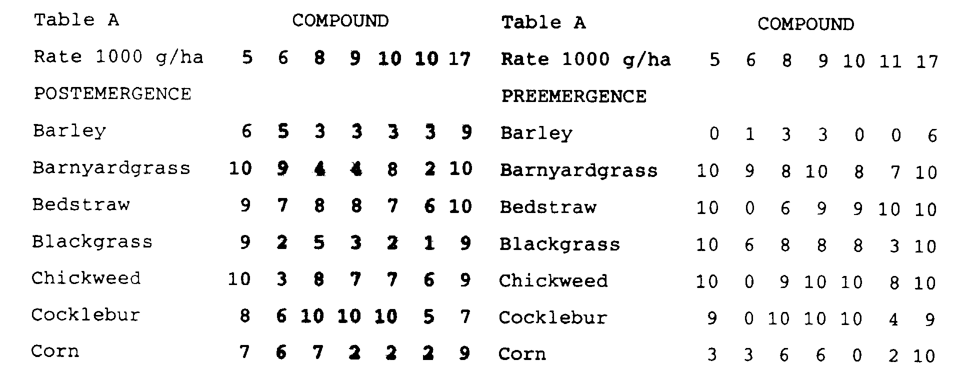

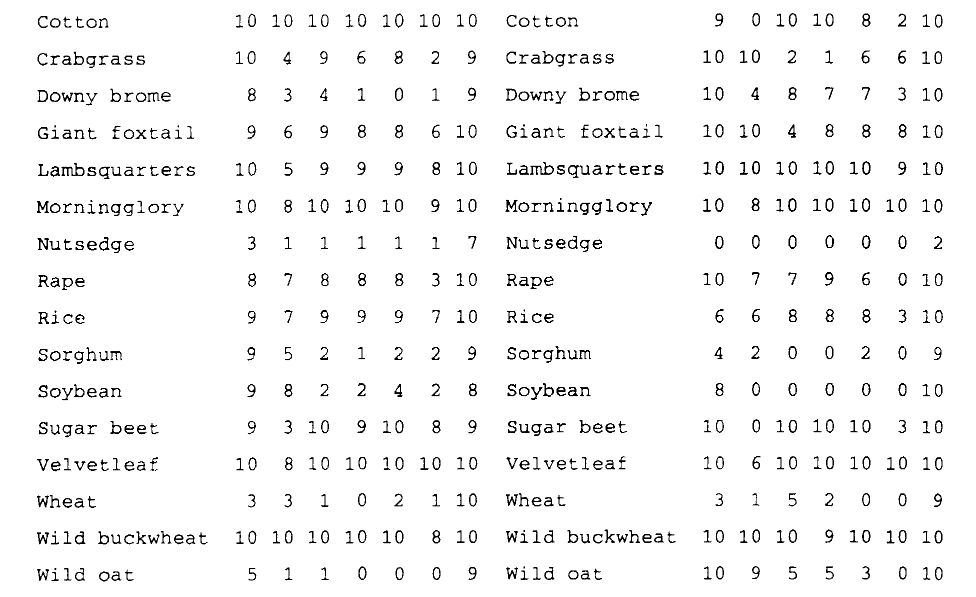

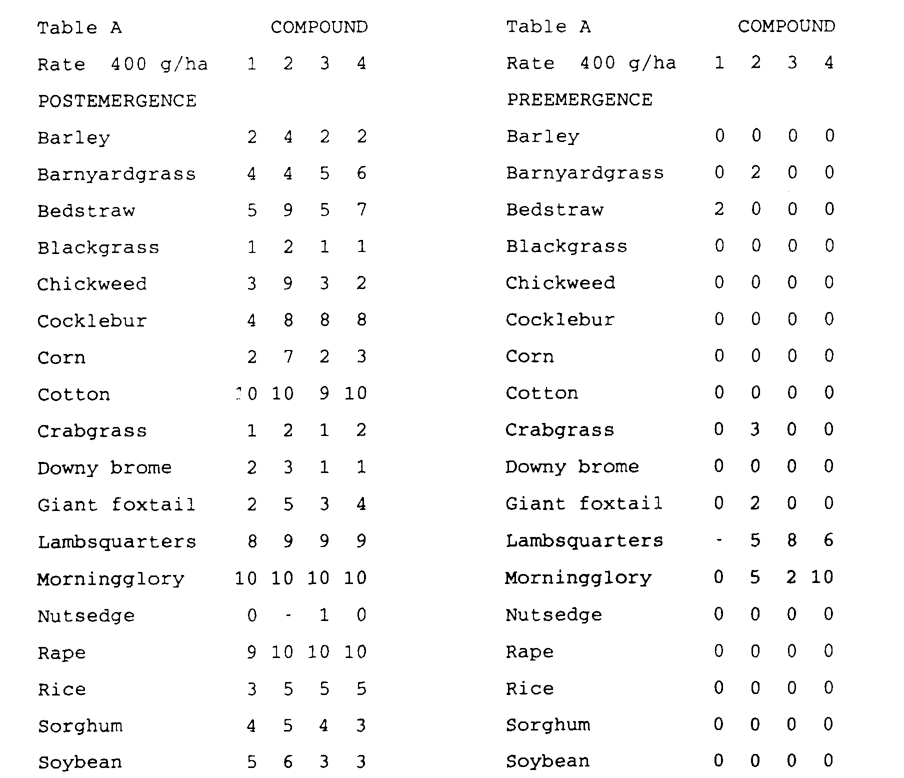

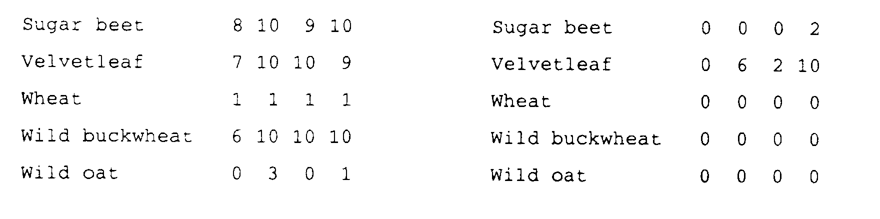

| Table A | COMPOUND | | | | | | | |
|---|---|---|---|---|---|---|---|---|
| Rate 200 g/ha | 5 | 6 | 7 | 8 | 9 | 10 | 11 | 17 |
| POSTEMERGENCE | | | | | | | | |
| Barley | 5 | 3 | 2 | 2 | 1 | 3 | 2 | 5 |
| Barnyardgrass | 6 | 6 | 6 | 2 | 4 | 3 | 2 | 8 |
| Bedstraw | 9 | 6 | 6 | 7 | 6 | 4 | 4 | 8 |
| Blackgrass | 4 | 2 | 2 | 3 | 2 | 1 | 0 | 9 |
| Chickweed | 7 | 2 | 7 | 7 | 6 | 6 | 4 | 9 |
| Cocklebur | 7 | 5 | 10 | 8 | 10 | 10 | 4 | 7 |
| Corn | 3 | 2 | 2 | 2 | 1 | 2 | 1 | 5 |
| Cotton | 10 | 10 | 10 | 10 | 10 | 9 | 10 | 10 |
| Crabgrass | 7 | 3 | 1 | 1 | 3 | 2 | 2 | 6 |
| Downy brome | 4 | 2 | 1 | 4 | 1 | 1 | 1 | 4 |
| Giant foxtail | 7 | 4 | 4 | 5 | 7 | 3 | 3 | 8 |
| Lambsquarters | 10 | 1 | 8 | 8 | 8 | 9 | 8 | - |
| Morningglory | 9 | 6 | 10 | 10 | 10 | 10 | 10 | 10 |
| Nutsedge | 0 | 0 | - | 0 | 0 | 0 | 0 | 2 |
| Rape | 7 | 5 | 8 | 7 | 3 | 3 | 2 | 8 |
| Rice | 5 | 4 | 4 | 8 | 8 | 3 | 2 | 9 |
| Sorghum | 4 | 4 | 2 | 2 | 1 | 2 | 2 | 7 |
| Soybean | 8 | 4 | 2 | 1 | 2 | 2 | 1 | 8 |
| Sugar beet | 8 | 2 | 10 | 9 | 9 | 7 | 7 | 8 |
| Velvetleaf | 8 | 8 | 10 | 10 | 10 | 10 | 9 | 8 |
| Wheat | 3 | 2 | 0 | 1 | 0 | 1 | 0 | 6 |
| Wild buckwheat | 10 | 8 | 10 | 10 | 10 | 10 | 7 | 10 |
| Wild oat | 4 | 1 | 0 | 0 | 0 | 0 | 0 | 3 |

| Table A | COMPOUND | | | | | | | |
|---|---|---|---|---|---|---|---|---|
| Rate 200 g/ha | 5 | 6 | 7 | 8 | 9 | 10 | 11 | 17 |
| PREEMERGENCE | | | | | | | | |
| Barley | 0 | 0 | 0 | 2 | 0 | 0 | 0 | 2 |
| Barnyardgrass | 9 | 0 | 7 | 3 | 6 | 2 | 0 | 7 |
| Bedstraw | 4 | 0 | 0 | 0 | 9 | 0 | 0 | 5 |
| Blackgrass | 5 | 0 | 7 | 2 | 2 | 2 | 1 | 7 |
| Chickweed | 10 | 0 | 6 | 6 | 7 | 3 | 6 | 8 |
| Cocklebur | 0 | 0 | 6 | 8 | 7 | 3 | - | 2 |
| Corn | 0 | 0 | 0 | 0 | 0 | 0 | 0 | 4 |
| Cotton | 0 | 0 | 0 | 5 | 6 | - | 0 | 8 |
| Crabgrass | 9 | 8 | 5 | 0 | 0 | 0 | 0 | 10 |
| Downy brome | 4 | 2 | 0 | 0 | 3 | 0 | 0 | 4 |
| Giant foxtail | 9 | 5 | 3 | 3 | 0 | 2 | 0 | 9 |
| Lambsquarters | 10 | 3 | 9 | 9 | 9 | 8 | 4 | 10 |
| Morningglory | 8 | 0 | 10 | 10 | 6 | 10 | 8 | 8 |
| Nutsedge | 0 | 0 | 0 | 0 | 0 | 0 | 0 | 0 |
| Rape | 7 | 0 | 0 | 0 | 1 | 0 | 0 | 10 |
| Rice | 3 | 0 | 2 | 4 | 6 | 7 | 0 | 6 |
| Sorghum | 2 | 0 | 0 | 0 | 0 | 0 | 0 | 2 |
| Soybean | 3 | 0 | 0 | 0 | 0 | 0 | 0 | 9 |
| Sugar beet | 9 | 0 | 7 | 8 | 9 | 7 | 0 | 9 |
| Velvetleaf | 10 | 0 | 3 | 8 | 6 | 10 | 3 | 10 |
| Wheat | 0 | 0 | 0 | 2 | 0 | 0 | 0 | 2 |
| Wild buckwheat | 10 | 0 | 0 | 7 | 7 | 3 | 0 | 10 |
| Wild oat | 6 | 2 | 0 | 4 | 2 | 0 | 0 | 5 |

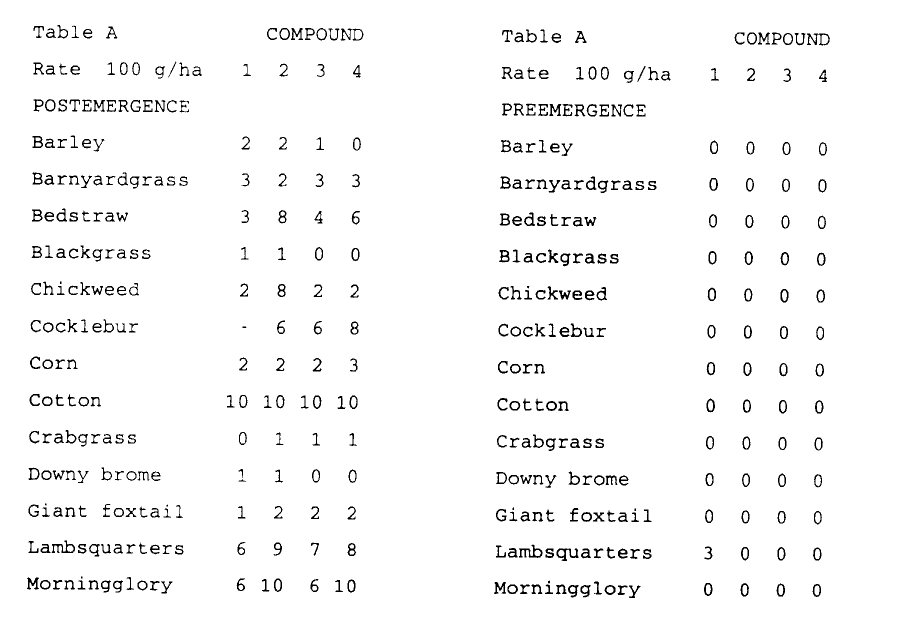

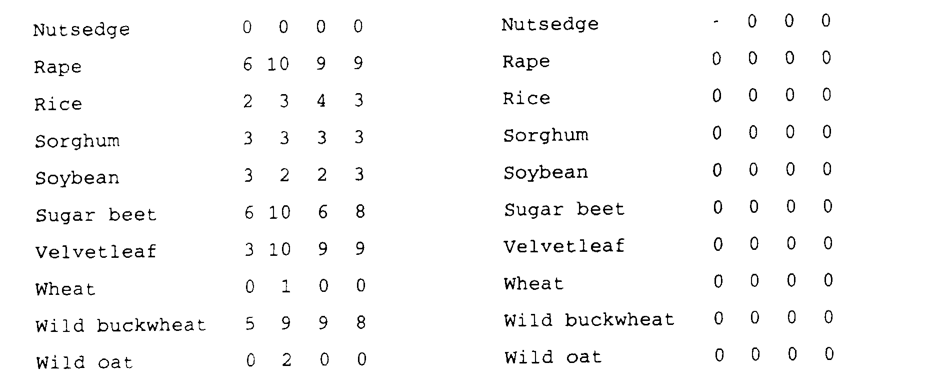

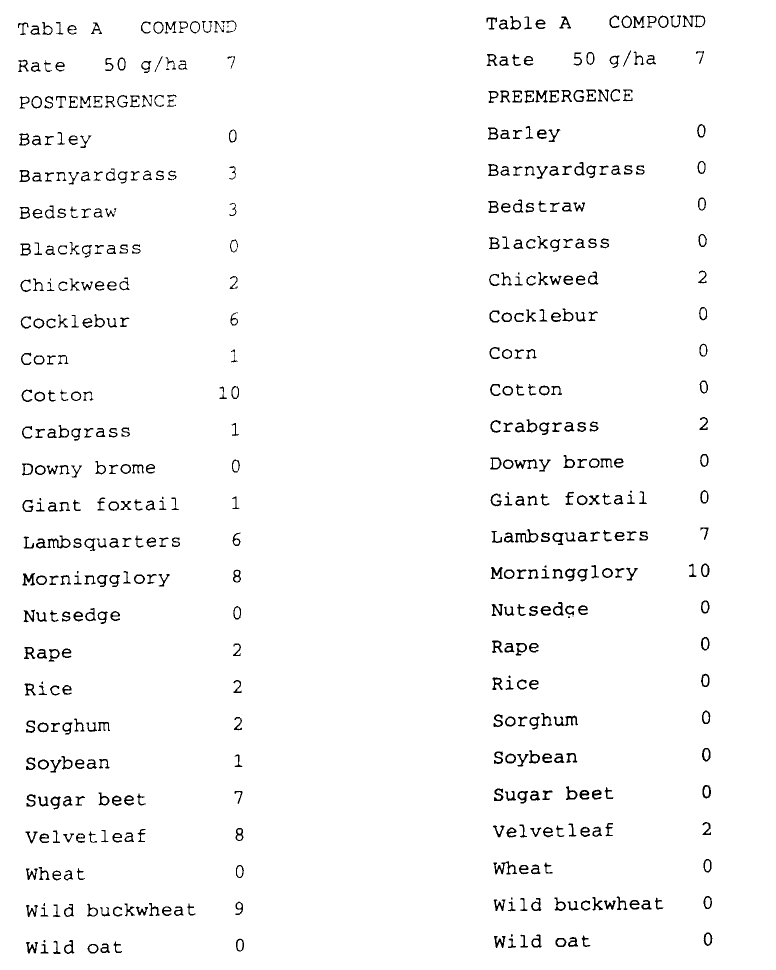

### Test B

Seeds of bedstraw *(Galium aparine),* blackgrass *(Alopecurus myosuroides),* broadleaf signalgrass *(Brachiaria decumbens),* cocklebur *(Xanthium strumarium),* corn *(Zea mays),* crabgrass *(Digitaria sanguinalis),* giant foxtail *(Setaria faberii),* lambsquarters *(Chenopodium album),* morningglory *(Ipomoea hederacea),* pigweed *(Amaranthus retroflexus),* rape *(Brassica napus),* soybean *(Glycine max),* sugar beet *(Beta vulgaris),* velvetleaf *(Abutilon theophrasti),* wheat *(Triticum aestivum),* wild oat *(Avena fatua)* and purple nutsedge *(Cyperus rotundus)* tubers were planted and treated preemergence with test chemicals formulated in a non-phytotoxic solvent mixture which included a surfactant.

At the same time, these crop and weed species were also treated with postemergence applications of test chemicals formulated in the same manner. Plants ranged in height from 2 to 18 cm (1- to 4-leaf stage) for postemergence treatments. Plant species in the flood test consisted of rice *(Oryza sativa),* smallflower flatsedge *(Cyperus difformis),* duck salad *(Heteranthera limosa)* and barnyardgrass *(Echinochloa crus-galli)* grown to the 2-leaf stage for testing. Treated plants and controls were maintained in a greenhouse for twelve to sixteen days, after which all species were compared to controls and visually evaluated. Plant response ratings, summarized in Table B, are based on a scale of 0 to 10 where 0 is no effect and 10 is complete control. A dash (-) response means no test result.

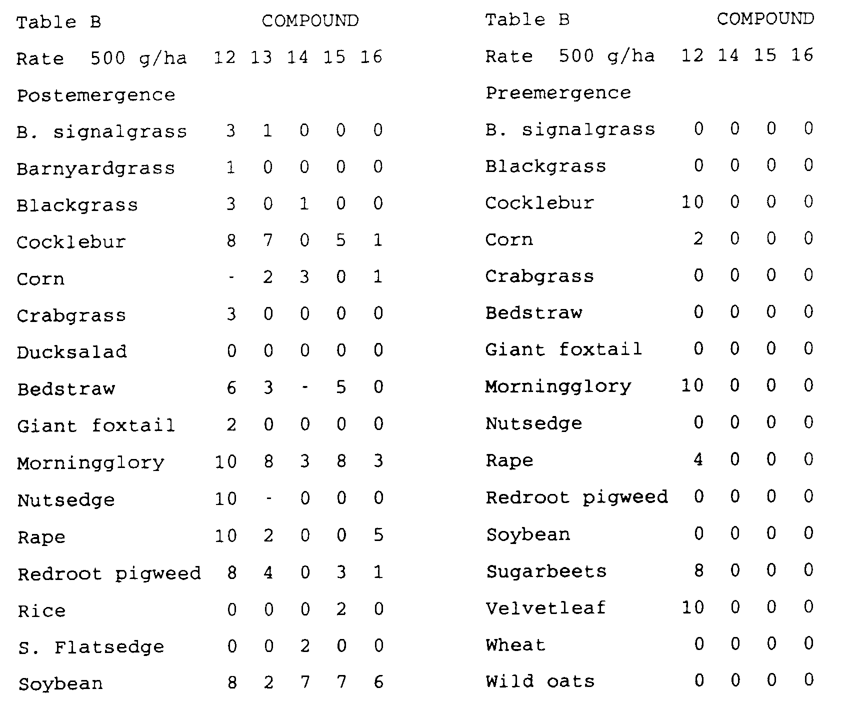

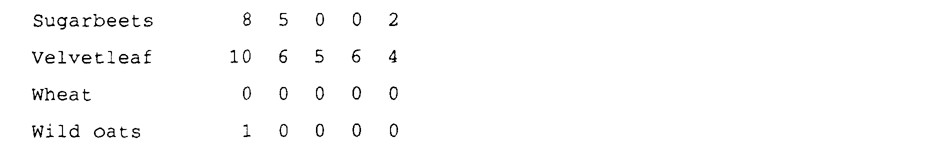

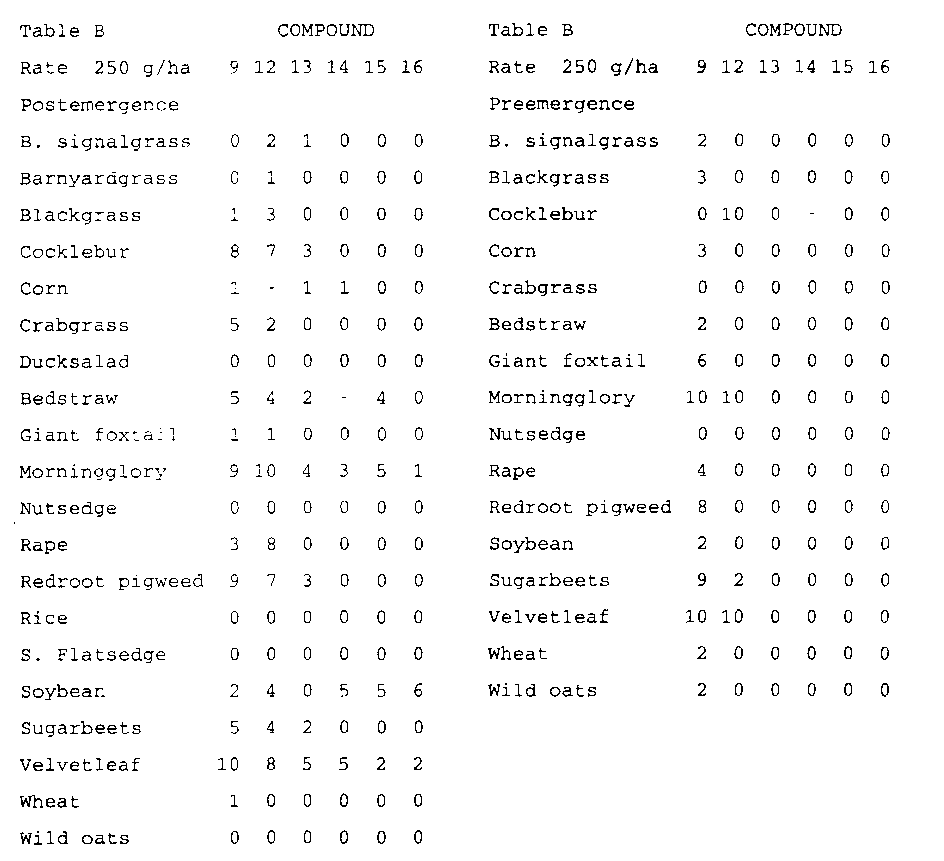

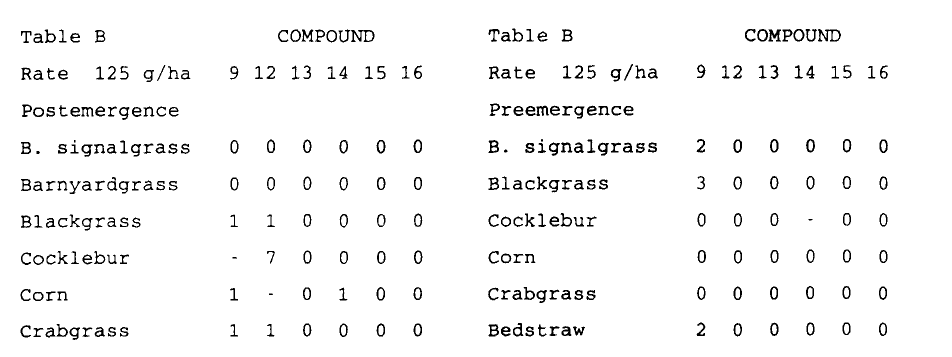

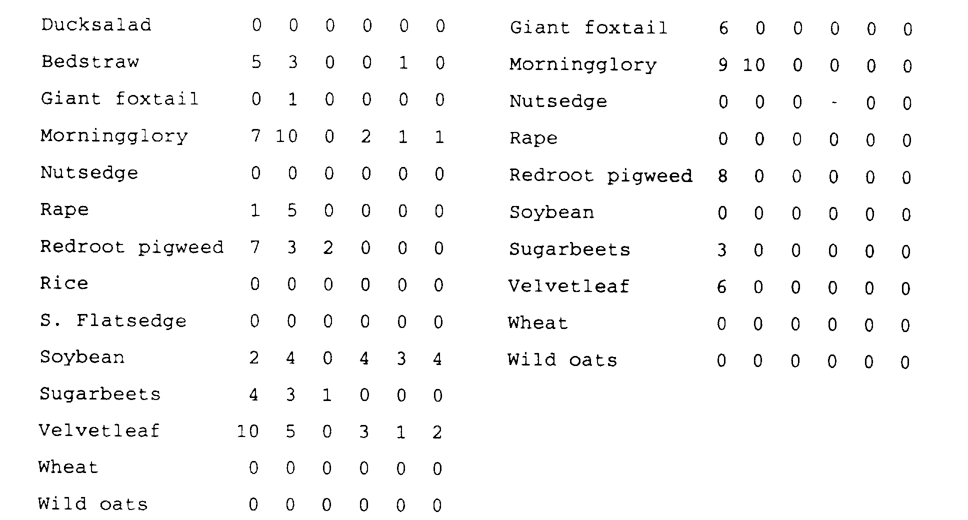

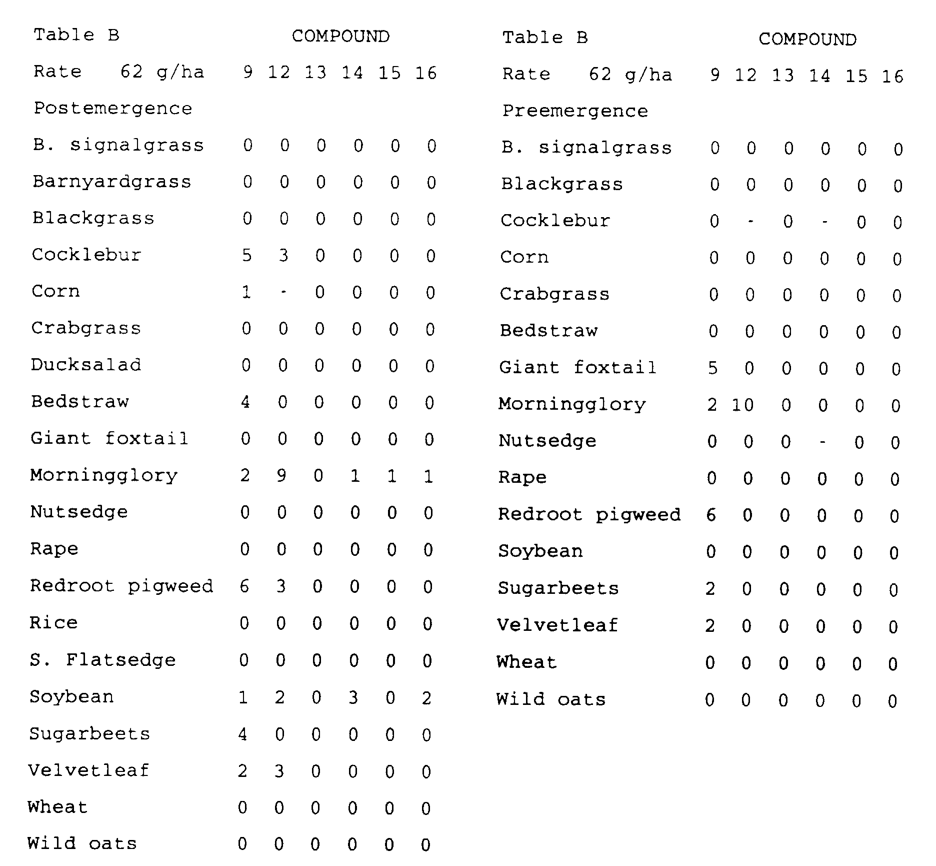

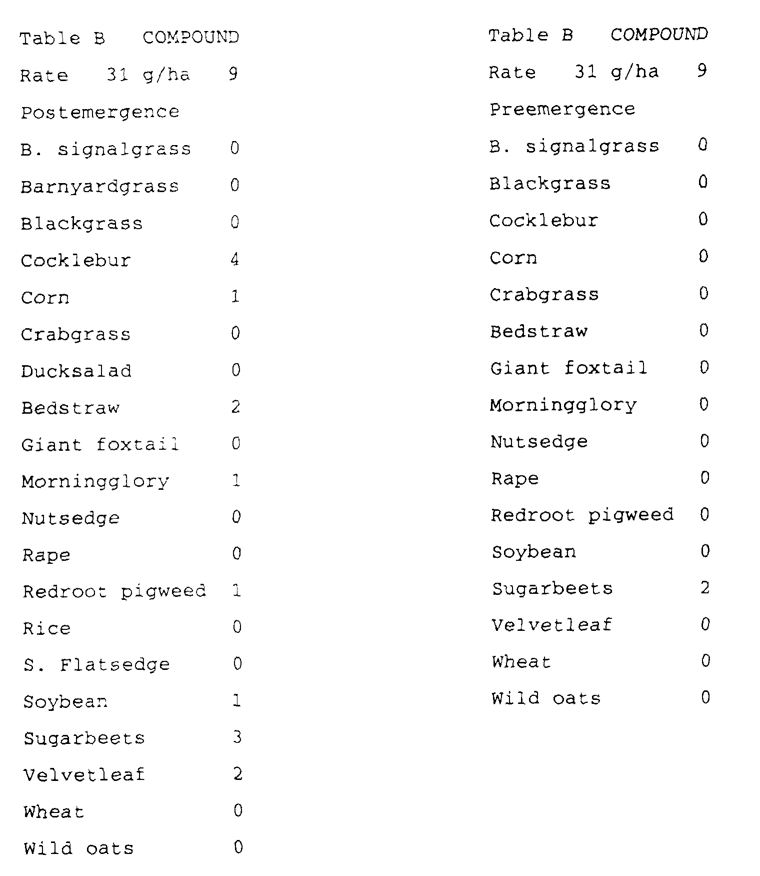

### Test C

The compounds evaluated in this test were formulated in a non-phytotoxic solvent mixture which included a surfactant and applied to the soil surface before plant seedlings emerged (preemergence application), to water that covered the soil surface (flood application), and to plants that were in the one-to-four leaf stage (postemergence application). A sandy loam soil was used for the preemergence and postemergence tests, while a silt loam soil was used in the flood application. Water depth was approximately 2.5 cm for the flood application and was maintained at this level for the duration of the test.

Plant species in the preemergence and postemergence tests consisted of barnyardgrass *(Echinochloa crus-galli),* winter barley *(Hordeum vulgare),* bedstraw *(Galium aparine),* blackgrass *(Alopecurus myosuroides),* chickweed *(Stellaria media),* cocklebur *(Xanthium strumarium),* corn 1 *(Zea mays),* cotton *(Gossypium hirsutum), crabgrass (Digitaria sanguinalis),* downy brome *(Bromus tectorum),* giant foxtail *(Setaria faberii),* johnsongrass *(Sorghum halepense),* lambsquarters *(Chenopodium album),* morningglory *(Ipomoea hederacea).* redroot pigweed *(Amaranthus retroflexus),* rape *(Brassica napus),* Italian ryegrass *(Lolium multiflorum),* soybean *(Glycine max),* speedwell *(Veronica persica),* sugar beet *(Beta vulgaris),* velvetleaf *(Abutilon theophrasti),* wheat *(Triticum aestivum),* wild buckwheat *(Polygonum convolvulus),* and wild oat *(Avena fatua).* All plant species were planted one day before application of the compound for the preemergence portion of this test. Plantings of these species were adjusted to produce plants of appropriate size for the posternergence portion of the test. Plant species in the flood test consisted of rice *(Oryza sativa),* umbrella sedge *(Cyperus difformis),* duck salad *(Heteranthera limosa)* and barnyardgrass 1 *(Echinochloa crus-galli).*

All plant species were grown using normal greenhouse practices. Visual evaluations of injury expressed on treated plants, when compared to untreated controls, were recorded approximately fourteen to twenty one days after application of the test compound. Plant response to the test compound is summarized in Table C, recorded on a 0 to 100 scale where 0 is no effect and 100 is complete control. A dash (-) response means no test result.

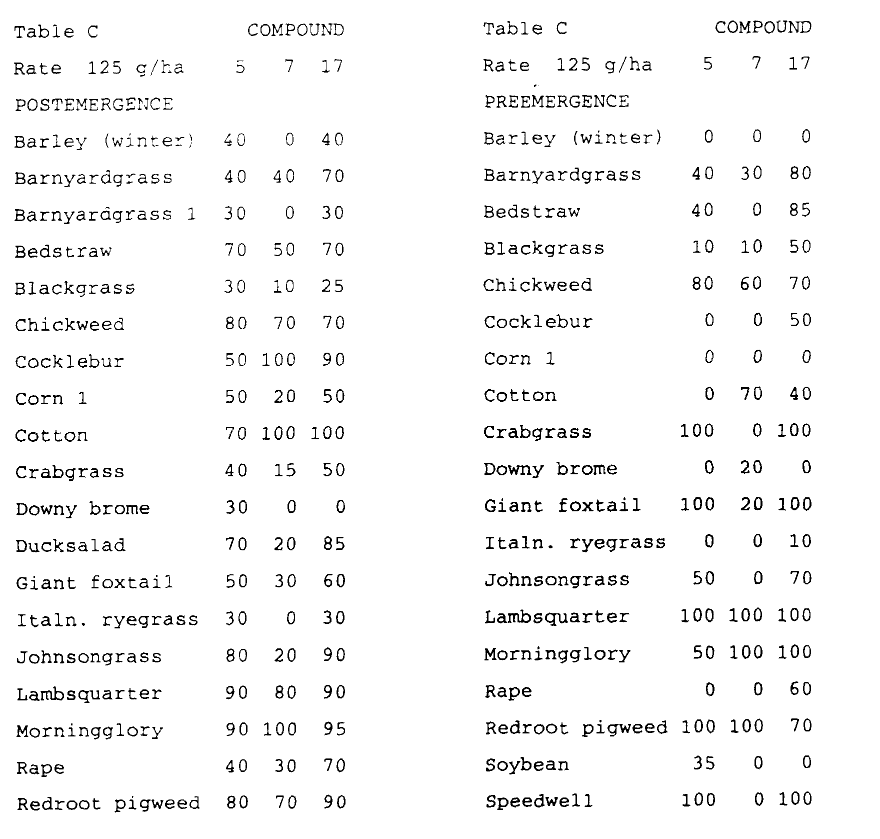

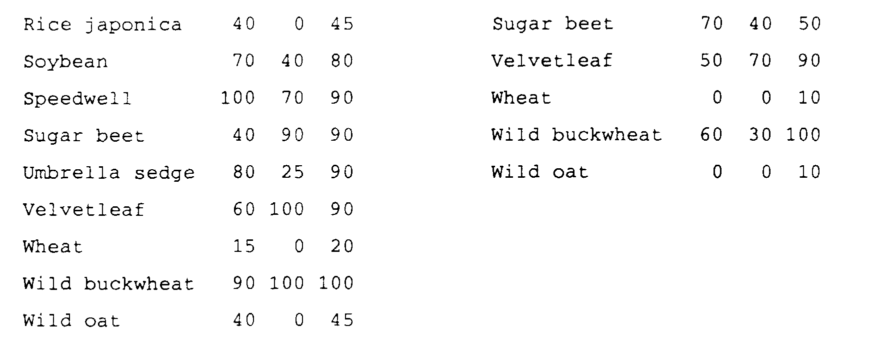

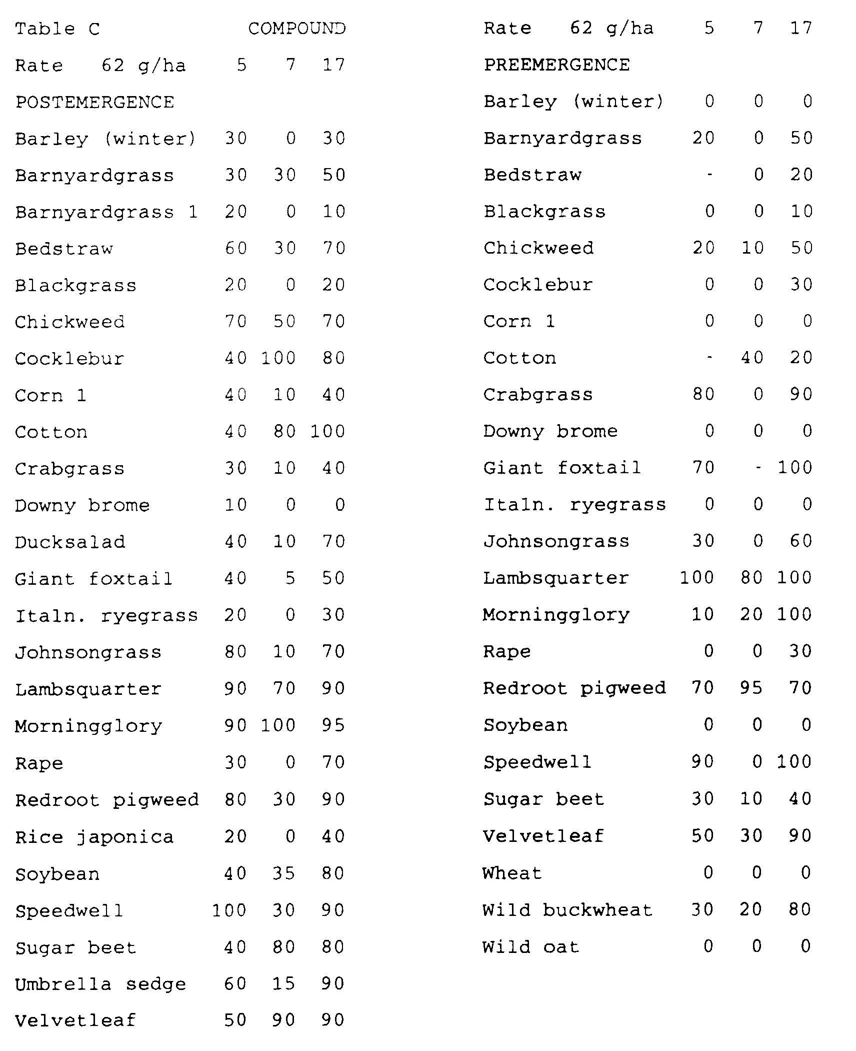

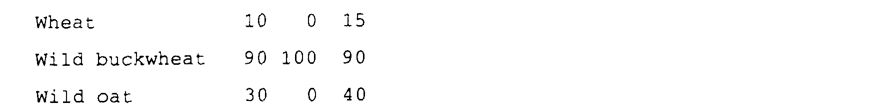

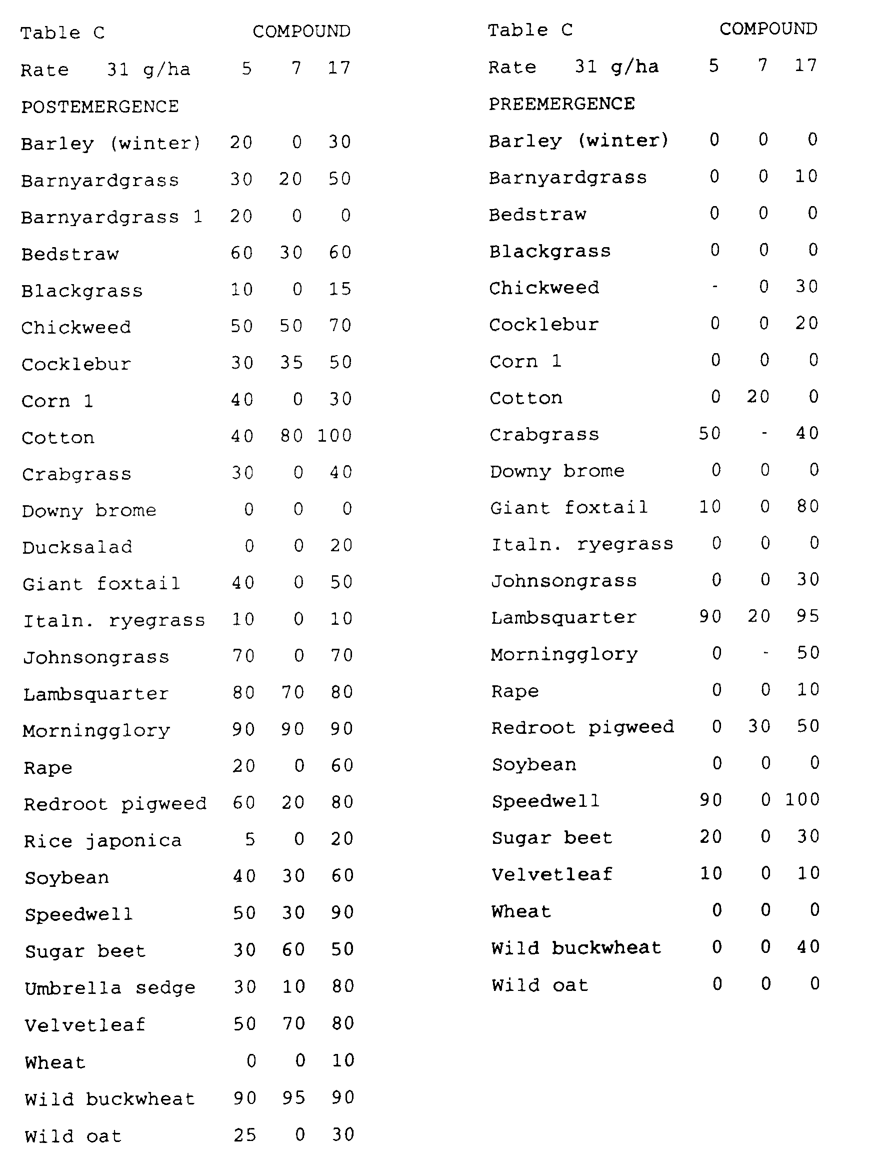

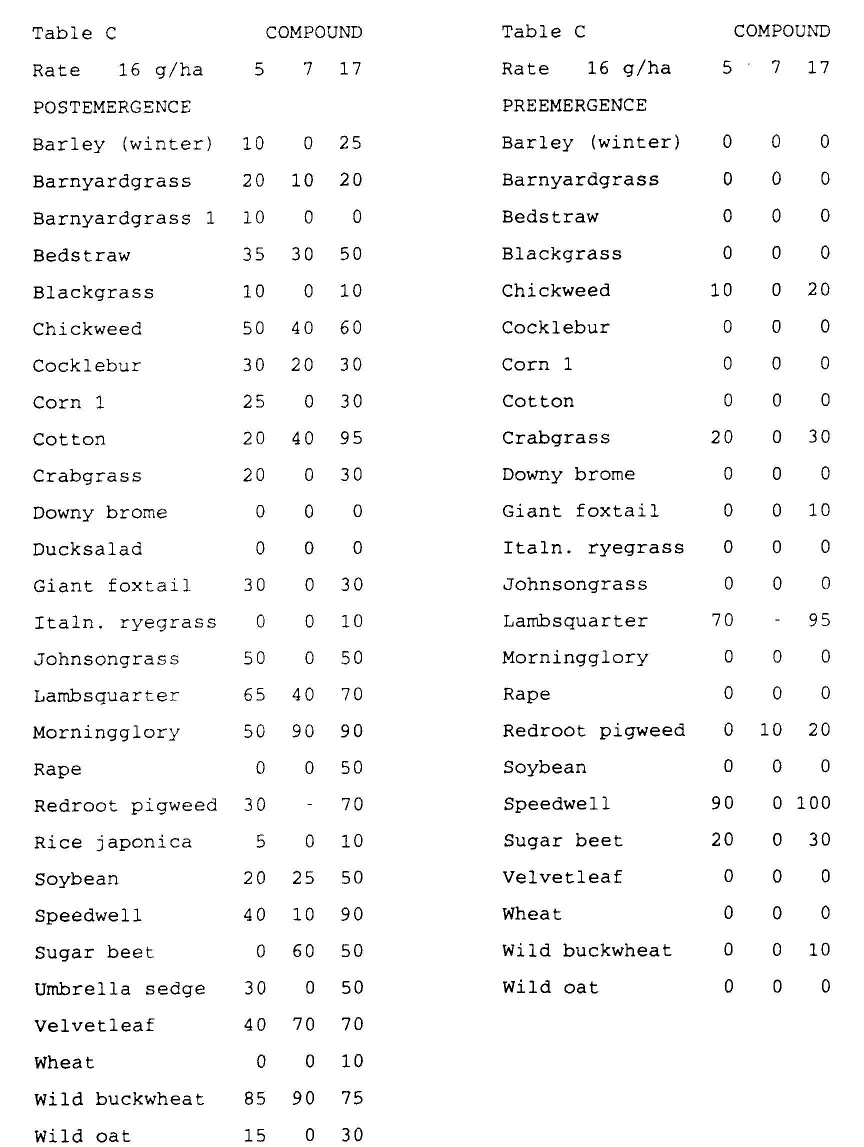

### Test D

Seeds of barnyardgrass *(Echinochloa crus-galli),* bindweed *(Concolculus arvensis),* black nightshade *(Solanum ptycanthum dunal),* cassia *(Cassia obtusifolia),* cocklebur *(Xanthium strumarium),* common ragweed *(Ambrosia artemisiifolia),* corn *(Zea mays* designated `Corn 2' and `Corn 5'), cotton *(Gossypium hirsutum),* crabgrass *(Digitaria spp.),* fall panicum *(Panicum dichotomiflorum),* giant foxtail *(Setaria faberii),* green foxtail *(Setaria viridis),* jimsonweed *(Datura stramonium),* johnsongrass *(Sorghum halepense),* lambsquarters *(Chenopodium album),* morningglory *(Ipomoea spp.),* pigweed *(Amaranthus retroflexus),* prickly sida *(Sida spinosa),* shattercane *(Sorghum vulgare),* signalgrass *(Brachiaria platyphylla),* soybean *(Glycine max* designated `Soybean 1' and `Soybean 2'), sunflower *(Helianthus annuus),* velvetleaf *(Abutilon theophrasti), wild* proso *(Pancium miliaceum),* woolly cupgrass *(Eriochloa villosa),* yellow foxtail *(Setaria lutescens)* and purple nutsedge *(Cyperus rotundus)* tubers were planted into a clay loam soil. These crops and weeds were grown in the greenhouse until the plants ranged in height from two to eighteen cm (one- to four-leaf stage), then treated postemergence with the test chemicals formulated in a non-phytotoxic solvent mixture which included a surfactant. Pots receiving preemergence treatments were planted immediately prior to test chemical application. Pots treated in this fashion were placed in the greenhouse and maintained according to routine greenhouse procedures.

Treated plants and untreated controls were maintained in the greenhouse approximately 14-21 days after application of the test compound. Visual evaluations of plant injury responses were then recorded. Plant response ratings, summarized in Table D, are reported on a 0 to 100 scale where 0 is no effect and 100 is complete control.

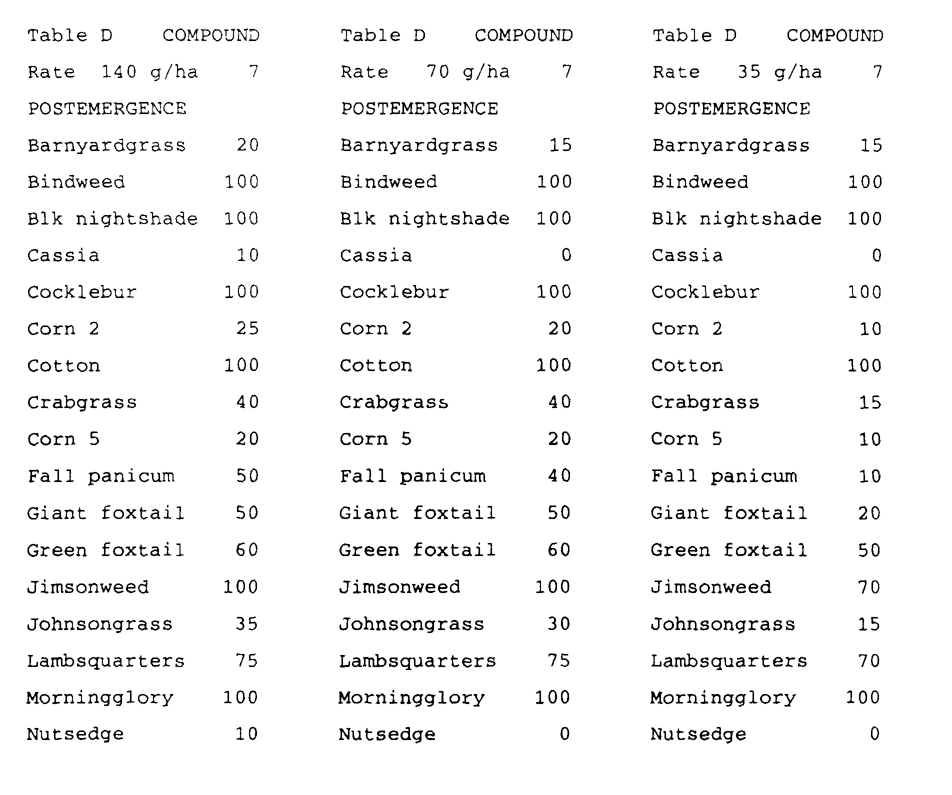

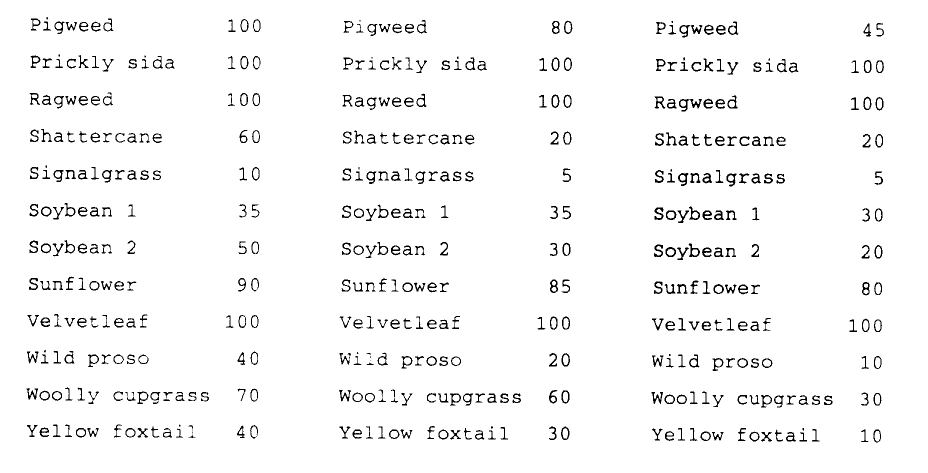

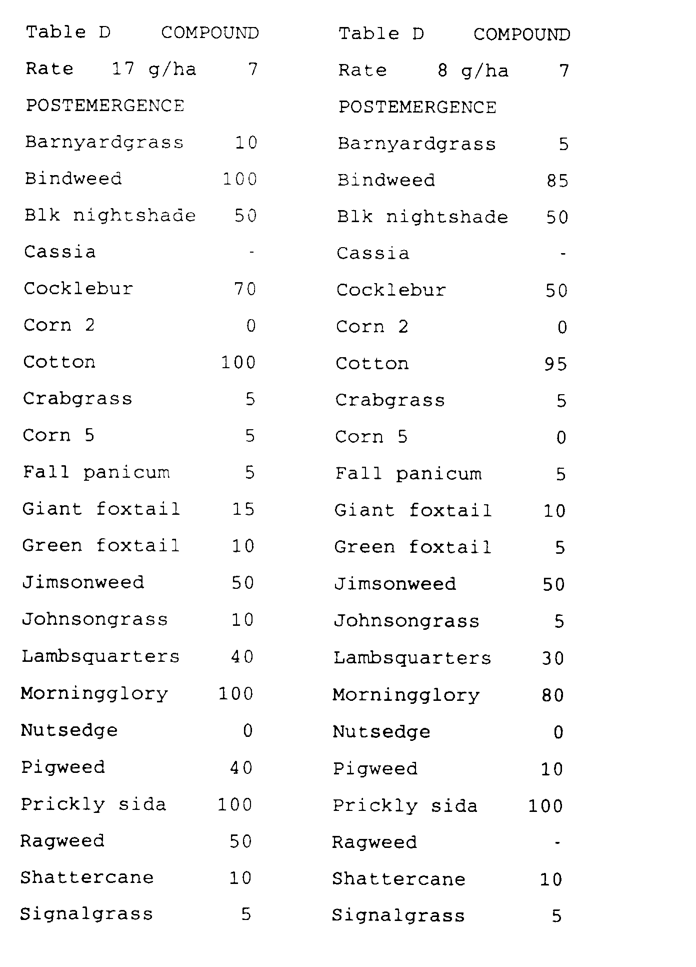

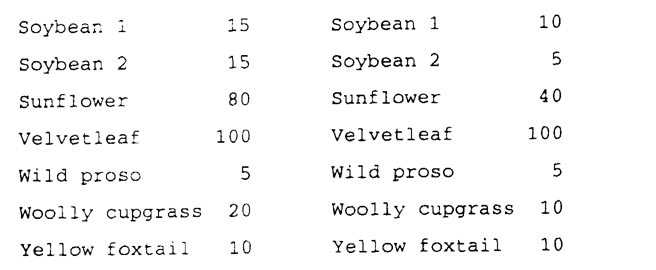

### Test E

Compounds evaluated in this test were formulated in a non-phytotoxic solvent mixture which included a surfactant and applied to plants that were grown for various periods of time before treatment (postemergence application). A mixture of sandy loam soil and greenhouse potting mix in a 60:40 ratio was used for the postemergence test.

Plantings of these crops and weed species were adjusted to produce plants of appropriate size for the postemergence test. All plant species were grown using normal greenhouse practices. Crop and weed species include arrowleaf sida *(Sida rhombifolia),* barnyardgrass *(Echinochloa crus-galli),* cocklebur *(Xanthium strumarium),* common ragweed *(Ambrosia elatior),* corn 1 (*Zea mays*), cotton (*Gossypium hirsutum*), eastern black nightshade *(Solanum ptycanthum),* fall panicum *(Panicum dichotomiflorum),* field bindweed *(Convolvulus arvensis),* giant foxtail *(Setaria faberii),* hairy beggarticks *(Bidens pilosa),* ivyleaf morningglory *(Ipomoea hederacea),* johnsongrass *(Sorghum halepense),* ladysthumb smartweed *(Polygonum persicaria),* lambsquarters *(Chenopodium album),* large crabgrass *(Digitaria sanguinalis),* purple nutsedge *(Cyperus rotundus),* redroot pigweed *(Amaranthus retroflexus),* soybean 1(*Glycine max*), surinam grass *(Brachiaria decumbens),* velvetleaf *(Abutilon theophrasti)* and wild poinsettia *(Euphorbia heterophylla).*

Treated plants and untreated controls were maintained in a greenhouse for approximately 14 to 21 days, after which all treated plants were compared to untreated controls and visually evaluated. Plant response ratings, summarized in Table E, were based upon a 0 to 100 scale where 0 was no effect and 100 was complete control. A dash response (-) means no test result.

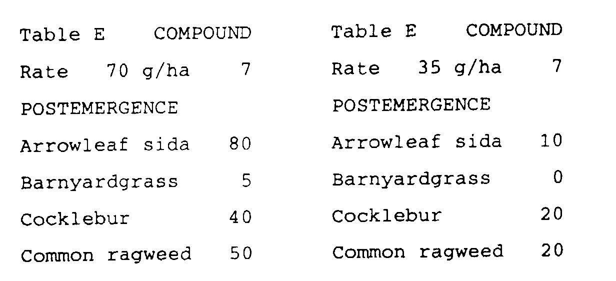

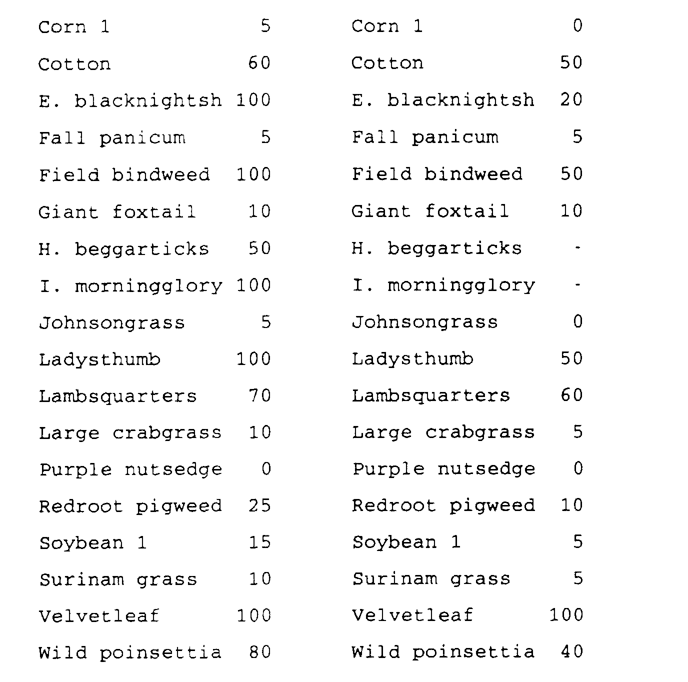

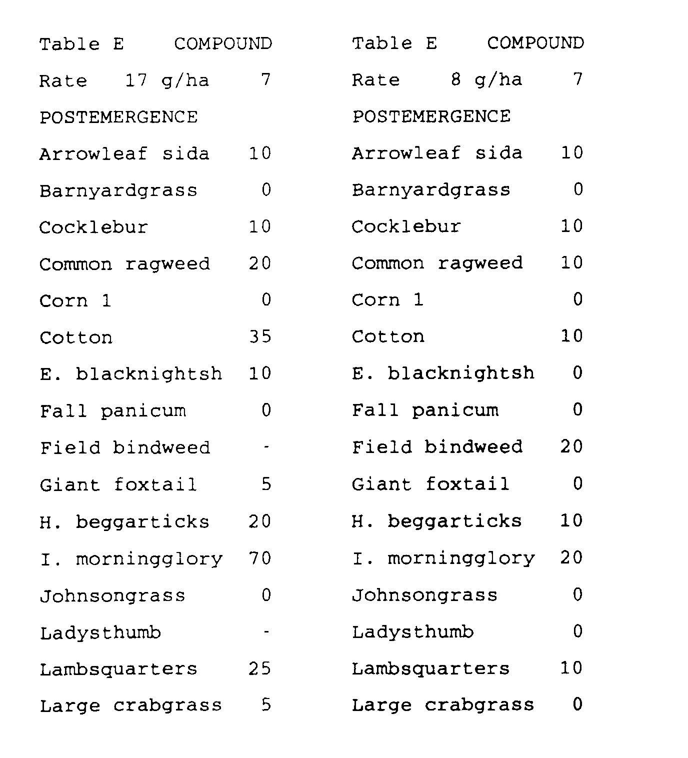

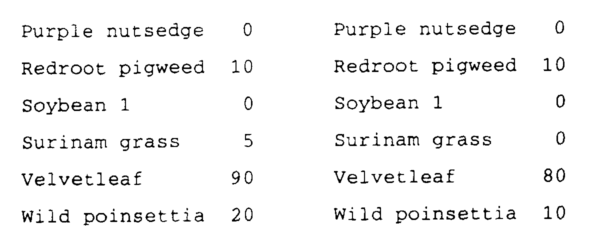

### Test F

Compounds evaluated in this test were formulated in a non-phytotoxic solvent mixture which included a surfactant and applied to plants that were in the 1- to 4-leaf stage (postemergence application). A mixture of sandy loam soil and greenhouse potting mix in a 60:40 ratio was used for the postemergence test.

Plantings of these crops and weed species were adjusted to produce plants of appropriate size for the postemergence test. All plant species were grown using normal greenhouse practices. Crop and weed species include annual bluegrass *(Poa annua),* blackgrass 2 *(Alopecurus myosuroides),* black nightshade *(Solanum nigra),* chickweed *(Stellaria media),* common poppy *(Papaver rhoeas),* deadnettle *(Lamium amplexicaule),* downy brome *(Bromus tectorum),* field violet *(Viola arvensis),* galium 2 *(Galium aparine),* green foxtail *(Setaria viridis),* Italian ryegrass *(Lolium multiflorum),* jointed goatgrass *(Aegilops cylindrica),* kochia *(Kochia scoparia),* lambsquarters *(Chenopodium album),* littleseed canarygrass *(Phalaris minor),* rape 1 *(Brassica napus),* redroot pigweed *(Amaranthus retroflexus),* Russian thistle *(Salsola kali),* scentless chamomile *(Matricaria inodora),* spring barley *(Hordeum vulgare),* sugar beet *(Beta vulgaris),* sunflower *(Helianthus annuus),* ivyleaf speedwell *(Veronica hederaefolia)*, spring wheat *(Triticum aestivum),* winter wheat *(Triticum aestivum),* wild buckwheat *(Polygonum convolvulus),* wild mustard *(Sinapsis arvensis),* wild oat 1 *(Avena fatua),* windgrass *(Apera spica-venti)* and winter barley *(Hordeum vulgare).*

Treated plants and untreated controls were maintained in a greenhouse for approximately 21 to 28 days, after which all treated plants were compared to untreated controls and visually evaluated. Plant response ratings, summarized in Table F, are based upon a 0 to 100 scale where 0 is no effect and 100 is complete control. A dash response (-) means no test result.

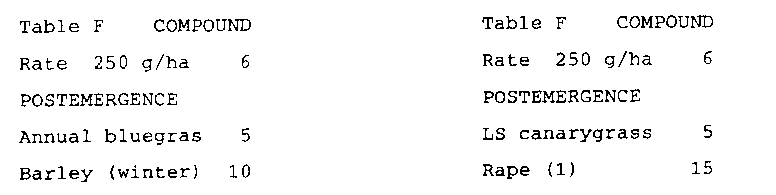

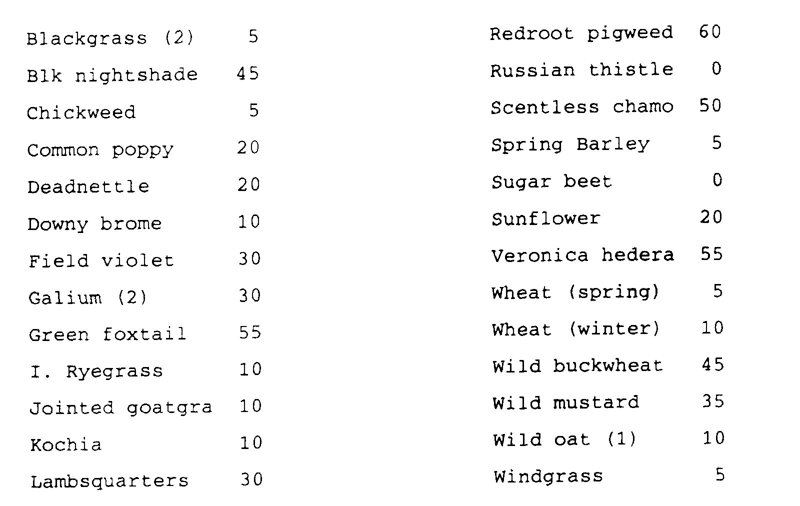

| Table F | COMPOUND | | | | | | | | | | | | |
|---|---|---|---|---|---|---|---|---|---|---|---|---|---|
| Rate 125 g/ha | 2 | 3 | 4 | 5 | 6 | 7 | 8 | 9 | 10 | 11 | 14 | 16 | 17 |
| POSTEMERGENCE | | | | | | | | | | | | | |
| Annual bluegras | 10 | 0 | 20 | 20 | 50 | 10 | 20 | 10 | 10 | 10 | 0 | 0 | 50 |
| Barley (winter) | 10 | 10 | 10 | 25 | 20 | 10 | 10 | 20 | 10 | 10 | 5 | 0 | 20 |
| Blackgrass (2) | 10 | 2 | 5 | 20 | 30 | 10 | 10 | 5 | 10 | 10 | 0 | 0 | 30 |
| Blk nightshade | 100 | 100 | 100 | 100 | 85 | 100 | 100 | 100 | 100 | 75 | 20 | 0 | 100 |
| Chickweed | 100 | 80 | 80 | 75 | 60 | 100 | 85 | 80 | 85 | 50 | 10 | 40 | 100 |
| Common poppy | 20 | 15 | 40 | 50 | - | 70 | 70 | 50 | 70 | 60 | 10 | 50 | 100 |
| Deadnettle | 85 | 75 | 100 | 100 | 75 | 100 | 100 | 65 | 60 | 30 | - | - | 100 |
| Downy brome | 30 | 5 | 5 | 40 | 30 | 5 | 5 | 10 | 5 | 2 | 0 | 0 | 60 |
| Field violet | 80 | 85 | 75 | 100 | 75 | 90 | 85 | 80 | 75 | 60 | 30 | 0 | 80 |
| Galium (2) | 60 | 65 | 95 | 60 | 50 | 65 | 50 | 55 | 55 | 20 | 20 | 5 | 85 |
| Green foxtail | 50 | 60 | 30 | 100 | 80 | 30 | 15 | 20 | 50 | 20 | 0 | 2 | 100 |
| I. Ryegrass | 10 | 10 | 5 | 30 | 10 | 10 | 10 | 5 | 5 | 2 | 10 | 0 | 30 |
| Jointed goatgra | 10 | 5 | 10 | 20 | 20 | 10 | 5 | 0 | 5 | 5 | 0 | 0 | 20 |
| Kochia | 70 | 80 | 100 | 100 | 90 | 90 | - | - | - | - | 0 | 30 | 100 |
| Lambsquarters | 100 | - | 100 | 100 | 10 | 70 | 85 | 100 | 100 | 60 | 20 | 30 | 100 |
| LS canarygrass | 15 | 10 | 15 | 50 | 30 | 30 | 10 | 10 | 10 | 5 | 0 | 0 | 40 |
| Rape (1) | 100 | 100 | 100 | 60 | 30 | 70 | 75 | 35 | 45 | 25 | 20 | 20 | 100 |
| Redroot pigweed | 100 | 100 | 100 | 100 | 100 | 100 | 100 | 100 | 100 | 85 | 0 | 0 | 100 |
| Russian thistle | 100 | 100 | 100 | 70 | 20 | - | 100 | 100 | 100 | 80 | 0 | 40 | 100 |
| Scentless chamo | 65 | 70 | 75 | 50 | 50 | 70 | 65 | 55 | 50 | 50 | 0 | 10 | 100 |
| Spring Barley | 10 | 5 | 10 | 20 | 10 | 5 | 10 | 10 | 10 | 10 | 5 | 0 | 10 |
| Sugar beet | 100 | 100 | 100 | 80 | 10 | 100 | 100 | 100 | 100 | 70 | 20 | 50 | 100 |
| Sunflower | 80 | 75 | 80 | 30 | 30 | 90 | 100 | 90 | 98 | 60 | 10 | 10 | 100 |
| Veronica hedera | 50 | 50 | 50 | 65 | 85 | 100 | 60 | 60 | 65 | 40 | 0 | 0 | 100 |
| Wheat (spring) | 10 | 5 | 10 | 20 | 15 | 10 | 10 | 10 | 10 | 10 | 5 | 0 | 20 |
| Wheat (winter) | 10 | 5 | 5 | 10 | 10 | 5 | 5 | 10 | 10 | 2 | 0 | 0 | 15 |
| Wild buckwheat | 100 | 100 | 100 | 100 | 75 | 100 | 100 | 100 | 100 | 65 | 30 | 10 | 100 |
| Wild mustard | 100 | 100 | 100 | 70 | 55 | 55 | 55 | 55 | 55 | 30 | 10 | 0 | 100 |
| Wild oat (1) | 25 | 20 | 10 | 50 | 20 | - | 10 | 10 | 10 | 5 | 5 | 0 | 45 |
| Windgrass | 10 | 10 | 20 | 50 | 45 | 5 | 2 | 0 | 10 | 2 | 0 | 0 | 60 |

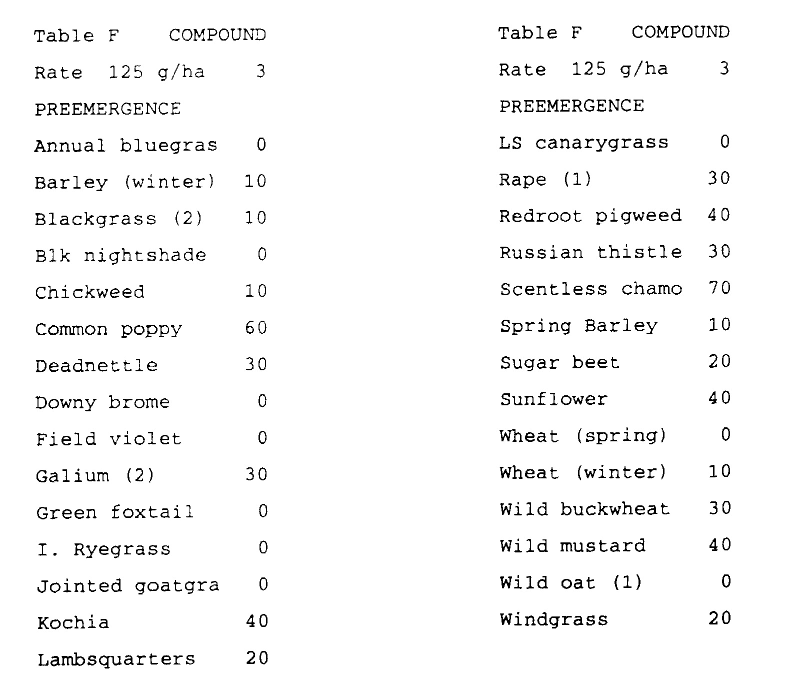

| Table F | COMPOUND | | | | | | | | | | | | |
|---|---|---|---|---|---|---|---|---|---|---|---|---|---|
| Rate 62 g/ha | 1 | 2 | 3 | 4 | 5 | 6 | 7 | 8 | 9 | 10 | 11 | 13 | 17 |
| POSTEMERGENCE | | | | | | | | | | | | | |
| Annual bluegras | 10 | 0 | 10 | 10 | 20 | 10 | 10 | 10 | 0 | 10 | 0 | 0 | 20 |
| Barley (winter) | 5 | 10 | 10 | 10 | 10 | 20 | 5 | 10 | 30 | 10 | 5 | 0 | 10 |
| Blackgrass (2) | 10 | 5 | 10 | 10 | 20 | 10 | 10 | 10 | 5 | 10 | 5 | 10 | 20 |
| Blk nightshade | 70 | 75 | 100 | 100 | 80 | 70 | 100 | 100 | 100 | 100 | 75 | 35 | 85 |
| Chickweed | 10 | 30 | 50 | 65 | 40 | 10 | 50 | 90 | 65 | 70 | 45 | 10 | 70 |
| Common poppy | 30 | 30 | 20 | 45 | 30 | 5 | 65 | 60 | 60 | 55 | 55 | - | 10 |
| Deadnettle | 10 | 100 | 100 | 75 | 100 | 30 | 100 | 60 | 50 | 40 | 40 | 10 | 65 |
| Downy brome | 0 | 5 | 10 | 5 | 30 | 10 | 10 | 5 | 20 | 0 | 0 | 20 | 10 |
| Field violet | 60 | 75 | 70 | 75 | 75 | 50 | 65 | 65 | 60 | 60 | 55 | 50 | 100 |
| Galium (2) | 10 | 50 | 60 | 65 | 50 | 25 | 30 | 60 | 50 | 40 | 20 | 0 | 55 |
| Green foxtail | 10 | 10 | 15 | 50 | 85 | 45 | 10 | 15 | 10 | 20 | 20 | 10 | 100 |
| I. Ryegrass | 5 | 5 | 2 | 5 | 10 | 5 | 0 | 5 | 5 | 10 | 0 | 10 | 10 |
| Jointed goatgra | 5 | 5 | 10 | 5 | 10 | 10 | 0 | 5 | 10 | 5 | 0 | 20 | 20 |
| Kochia | 55 | 65 | 70 | 70 | 100 | 65 | 70 | - | - | - | - | 20 | 100 |
| Lambsquarters | 100 | 100 | 100 | 100 | 80 | 10 | 100 | 60 | 70 | 80 | 55 | 40 | 75 |
| LS canarygrass | 10 | 10 | 10 | 20 | 30 | 30 | 5 | 10 | 5 | 10 | 0 | 10 | 30 |
| Rape (1) | 50 | 100 | 100 | 100 | 40 | 40 | 10 | 65 | 20 | 15 | 20 | 0 | 60 |
| Redroot pigweed | 100 | 30 | 45 | 100 | 100 | 30 | 85 | 100 | 100 | 80 | 60 | 50 | 60 |
| Russian thistle | 50 | 100 | 100 | 90 | 90 | 10 | 100 | 100 | 100 | 100 | 85 | - | 85 |
| Scentless chamo | 50 | 60 | 60 | 70 | 50 | 50 | 60 | 65 | 65 | 60 | 30 | 40 | 70 |
| Spring Barley | 5 | 5 | 5 | 5 | 15 | 10 | 5 | 10 | 10 | 10 | 5 | 5 | 10 |
| Sugar beet | 60 | 100 | 100 | 100 | 80 | 0 | 100 | 100 | 100 | 100 | 10 | 0 | 85 |
| Sunflower | 10 | 70 | 70 | 80 | 20 | 20 | 70 | 90 | 80 | 80 | 55 | 0 | 45 |
| Veronica hedera | 40 | 30 | 55 | 70 | 55 | 60 | 70 | 90 | 60 | 45 | 50 | 10 | 50 |
| Wheat (spring) | 5 | 10 | 5 | 5 | 10 | 15 | 10 | 5 | 10 | 10 | 5 | 0 | 15 |
| Wheat (winter) | 10 | 2 | 0 | 0 | 15 | 10 | 0 | 2 | 10 | 5 | 0 | 0 | 10 |
| Wild buckwheat | 60 | 95 | 100 | 100 | 75 | 60 | 100 | 100 | 100 | 100 | 65 | 30 | 100 |
| Wild mustard | 70 | 60 | 100 | 100 | 60 | 30 | 10 | 45 | 40 | 30 | 20 | 10 | 75 |
| Wild oat (1) | 5 | 10 | 10 | 20 | 30 | 10 | 10 | 10 | 10 | 10 | 0 | 20 | 20 |
| Windgrass | 0 | 5 | 0 | 20 | 10 | 10 | 0 | 0 | 0 | 10 | 2 | 10 | 20 |

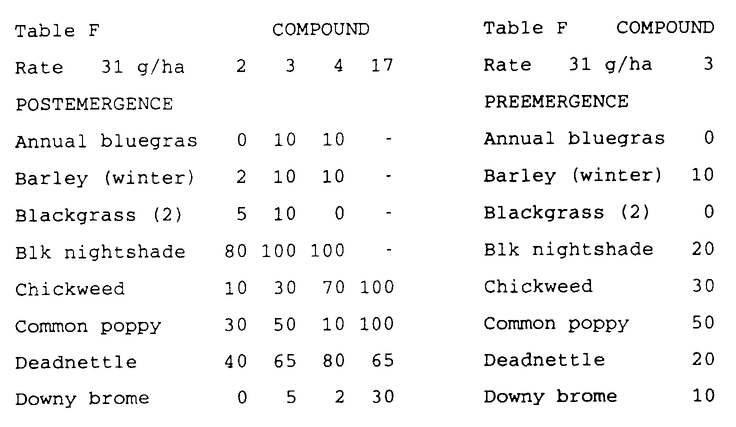

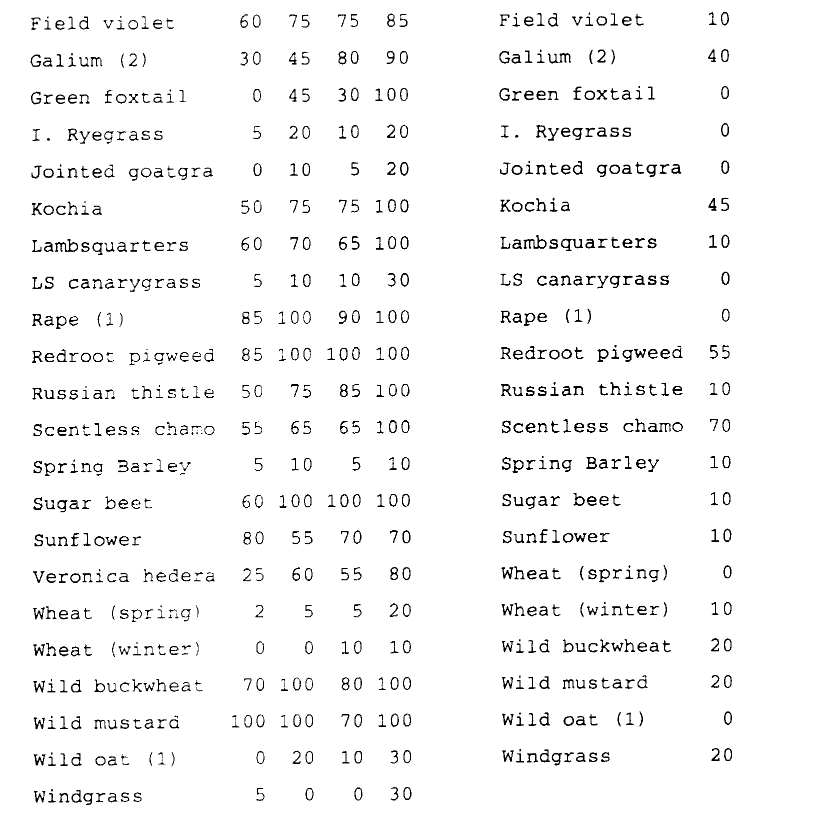

| Table F | COMPOUND | | | | | | |
|---|---|---|---|---|---|---|---|
| Rate 16 g/ha | 1 | 2 | 3 | 5 | 6 | 7 | 17 |
| POSTEMERGENCE | | | | | | | |
| Annual bluegras | 5 | 0 | 10 | 10 | 20 | 10 | 10 |
| Barley (winter) | 5 | 0 | 5 | 10 | 10 | 10 | 20 |
| Blackgrass (2) | 0 | 0 | 0 | 10 | 10 | 0 | 15 |
| Blk nightshade | 30 | 55 | 75 | 75 | 65 | 50 | 80 |
| Chickweed | 20 | 20 | 5 | 30 | 10 | 30 | 30 |
| Common poppy | 50 | 10 | 0 | 10 | 50 | 20 | 20 |
| Deadnettle | 10 | 20 | 55 | 50 | 30 | 35 | 50 |
| Downy brome | 0 | 0 | 0 | 20 | 10 | 0 | 25 |
| Field violet | 40 | 50 | 70 | 75 | 10 | 30 | 75 |
| Galium (2) | 10 | 15 | 30 | 30 | 10 | 30 | 50 |
| Green foxtail | 5 | 30 | 20 | 50 | 25 | 5 | 85 |
| I. Ryegrass | 2 | 0 | 0 | 10 | 0 | 0 | 20 |
| Jointed goatgra | 5 | 0 | 0 | 10 | 5 | 0 | 10 |
| Kochia | 60 | 25 | 15 | 70 | 40 | 60 | 85 |
| Lambsquarters | 60 | 70 | 60 | 60 | 0 | 60 | 65 |
| LS canarygrass | 5 | 0 | 0 | 20 | 20 | 0 | 20 |
| Rape (1) | 30 | 60 | 55 | 15 | 10 | 15 | 40 |
| Redroot pigweed | 100 | 0 | 40 | 100 | 10 | 50 | 100 |
| Russian thistle | 20 | 20 | 30 | 80 | 0 | 50 | 50 |
| Scentless chamo | 55 | 20 | 50 | 30 | 30 | 30 | 40 |
| Spring Barley | 5 | 0 | 0 | 10 | 10 | 0 | 15 |
| Sugar beet | 45 | 60 | 65 | 65 | 0 | 85 | 65 |
| Sunflower | 0 | 20 | 20 | 20 | 20 | 40 | 30 |
| Veronica hedera | - | 10 | 20 | 50 | 20 | 30 | 60 |
| Wheat (spring) | 5 | 0 | 0 | 10 | 10 | 0 | 20 |
| Wheat (winter) | 5 | 0 | 0 | 10 | 10 | 0 | 10 |
| Wild buckwheat | 40 | 40 | 70 | 60 | 40 | 75 | 70 |
| Wild mustard | 30 | 25 | 20 | 25 | 20 | 5 | 40 |
| Wild oat (1) | 10 | 5 | 5 | 20 | 10 | 5 | 20 |
| Windgrass | 2 | 0 | 0 | 30 | 10 | 0 | 20 |

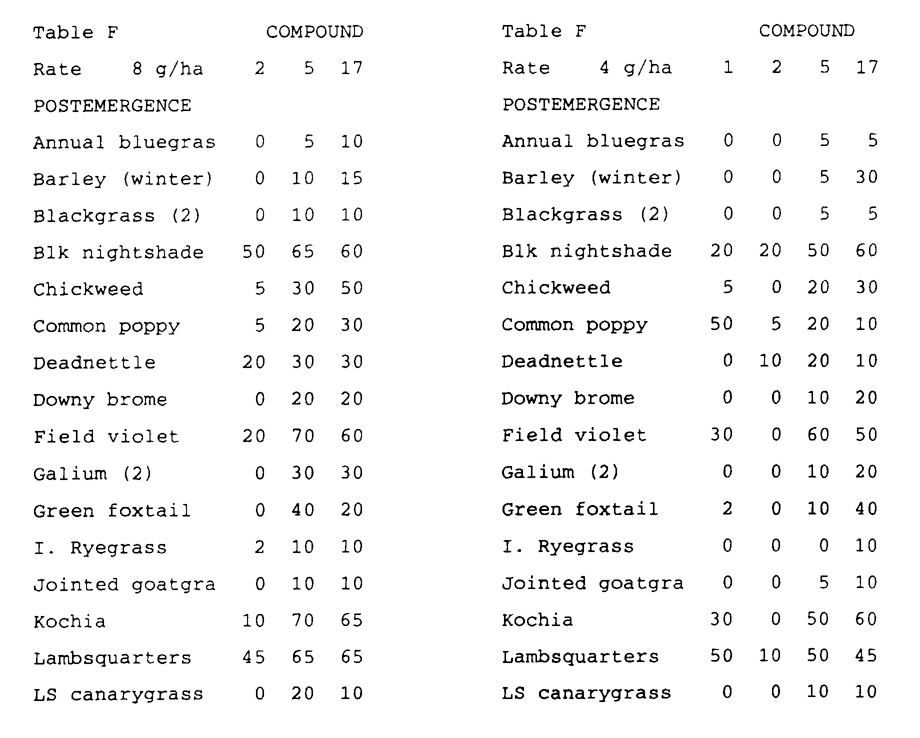

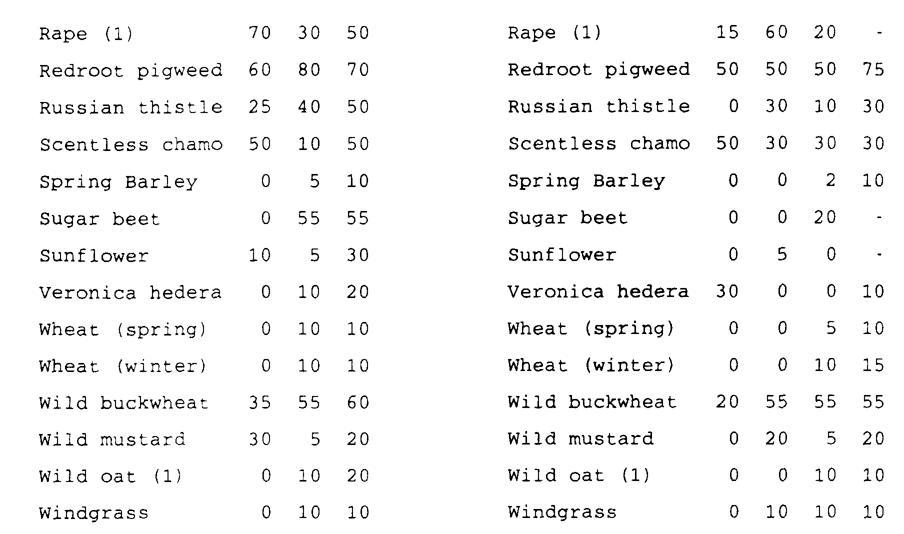

### Test G

Seeds, tubers, or plant parts of alexandergrass *(Brachiaria plantaginea),* broadleaf signalgrass *(Brachiaria decumbens),* bermudagrass *(Cynodon dactylon),* common purslane *(Portulaca oleracea),* common ragweed *(Ambrosia elatior),* cotton *(Gossypium hirsutum),* dallisgrass *(Paspalum dilatatum),* goosegrass *(Eleusine indica),* guineagrass *(Panicum maximum),* itchgrass *(Rottboellia exaltata),* johnson grass *(Sorghum halepense),* large crabgrass *(Digitaria sanguinalis),* pitted morningglory *(Ipomoea lacunosa),* peanuts *(Arachis hypogaea),* purple nutsedge *(Cyperus rotundus),* sandbur *(Cenchrus echinatus),* sourgrass *(Trichachne insularis)* and surinam grass *(Brachiaria decumbens)* were planted into greenhouse pots of flats containing greenhouse planting medium. Plant species were grown in separate pots or individual compartments. Preemergence applications were made within one day of planting the seed or plant part. Postemergence applications were applied when the plants were in the two- to four-leaf stage (three to twenty cm).

Test chemicals were formulated in a non-phytotoxic solvent mixture which included a surfactant and applied preemergence and postemergence to the plants. Untreated control plants and treated plants were placed in the greenhouse and visually evaluated for injury 13 to 21 days after herbicide application. Plant response ratings, summarized in Table G, are based on a 0 to 100 scale where 0 is no injury and 100 is complete control. A dash (-) response means no test result.

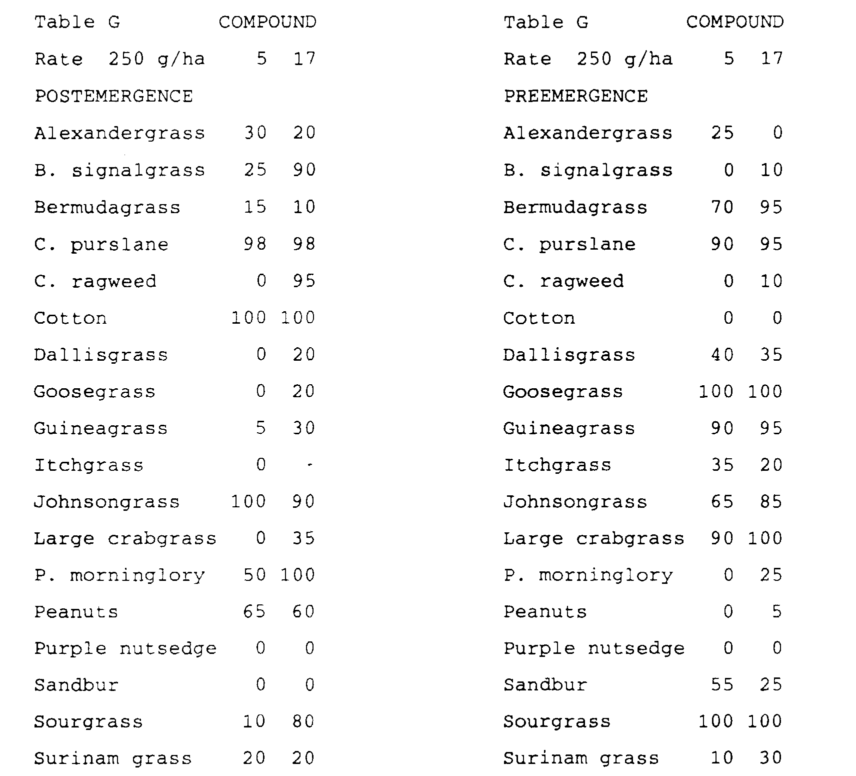

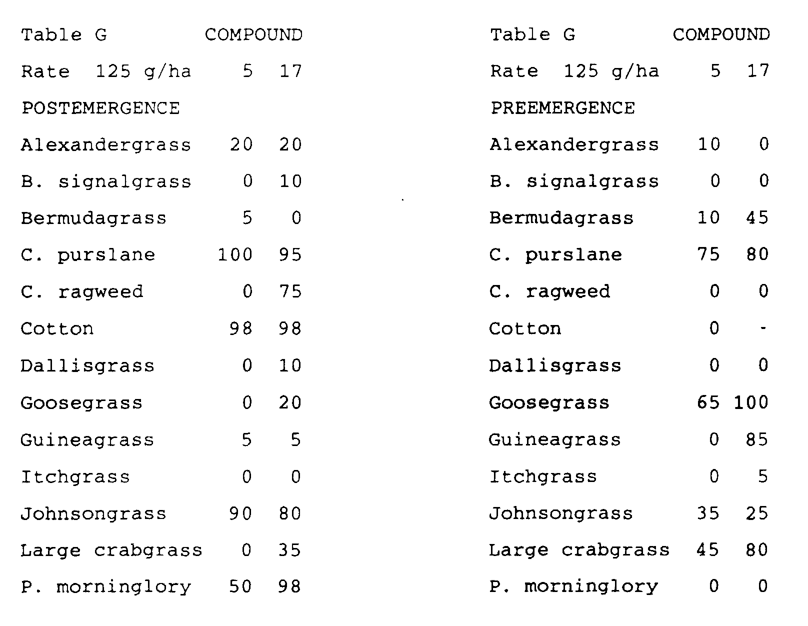

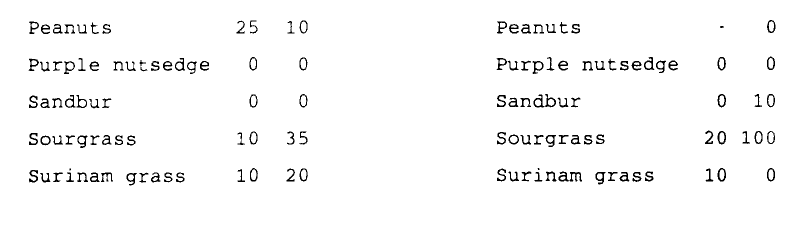

## Claims

1. A compound selected from the formula 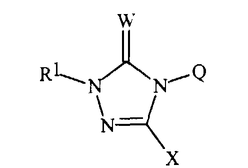 and *N*-oxides and agriculturally suitable salts thereof, wherein
Q is 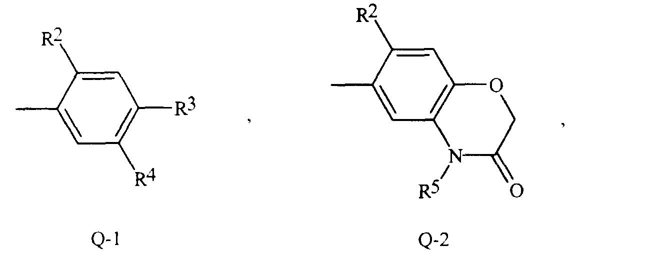 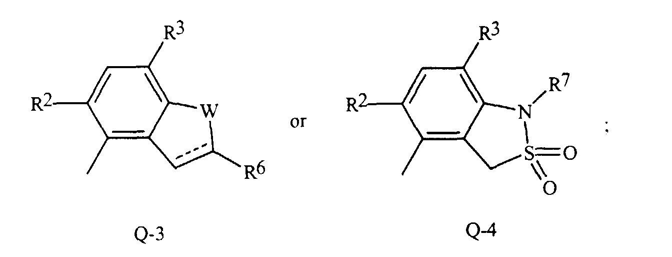
each W is independently O or S;
X is halogen, OR⁸, SR⁸ or S(O)ₙR⁹;
R¹ is C₁-C₄ alkyl, C₁-C₄ haloalkyl, C₂-C₄ alkoxyalkyl, C₃-C₄ alkenyl, C₃-C₄ haloalkenyl, C₃-C₄ alkynyl, CH₂CHO or CH₂CO₂R¹⁰;
R² is H or halogen;
R³ is C₁-C₂ alkyl, C₁-C₂ haloalkyl, OCH₃, SCH₃, OCHF₂, halogen, cyano or nitro;
R⁴ is OR¹¹, SR¹¹, CO₂R¹⁰, CH₂CO₂R¹⁰, WCH₂CO₂R^{10a}, OCH₂CN, CH₂CHClCO₂R¹⁰ or NR¹²SO₂R¹³;
R⁵ is C₁-C₄ alkyl, C₁-C₄ haloalkyl, C₂-C₄ cyanoalkyl, C₃-C₄ alkenyl, C₃-C₄ haloalkenyl, C₃-C₄ alkynyl, S(O)₂R⁹, CO₂R⁸ or C(=O)R⁸;
R⁶ is H, C₁-C₂ alkyl, C₁-C₂ haloalkyl, CO₂R¹⁰, CH₂OR¹⁰ or C(=O)R⁸;
R⁷ is H, C₁-C₄ alkyl, C₁-C₄ haloalkyl, C₃-C₄ alkenyl, C₃-C₄ haloalkenyl, C₃-C₄ alkynyl, S(O)₂R⁹, CO₂R⁸, CH₂CO₂R¹⁰, C(=O)R⁸ or CH₂C(=O)R⁸; or R⁷ is benzyl optionally substituted on the phenyl ring with C₁-C₂ alkyl, C₁-C₂ haloalkyl, C₁-C₂ alkoxy, halogen, nitro or cyano;
each R⁸ is independently C₁-C₄ alkyl, C₁-C₄ haloalkyl, C₃-C₄ alkenyl, C₃-C₄ haloalkenyl or C₃-C₄ alkynyl;
each R⁹ is independently C₁-C₄ alkyl or C₁-C₄ haloalkyl;
each R¹⁰ is independently H, C₁-C₄ alkyl, C₁-C₄ haloalkyl, C₃-C₄ alkenyl, C₃-C₄ haloalkenyl or C₃-C₄ alkynyl;
R^{10a} is H, C₁-C₄ alkyl, C₁-C₄ haloalkyl, C₃-C₄ alkenyl, C₃-C₄ haloalkenyl, C₃-C₄ alkynyl or 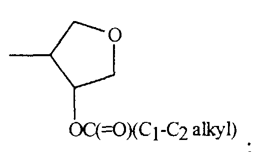
R¹¹ is C₁-C₄ alkyl, C₁-C₄ haloalkyl, C₃-C₇ cycloalkyl, C₃-C₇ halocycloalkyl, C₂-C₆ alkoxyalkyl, C₃-C₄ alkenyl, C₃-C₄ haloalkenyl or C₃-C₄ alkynyl;
R¹² is H, C₁-C₄ alkyl, C₁-C₄ haloalkyl, C₃-C₆ cycloalkyl, C₃-C₆ halocycloalkyl, C₃-C₄ alkenyl, C₃-C₄ haloalkenyl, C₃-C₄ alkynyl, C₂-C₆ alkoxyalkyl, C₂-C₆ haloalkoxyalkyl, CO₂R⁸, C(=O)R⁸ or S(O)₂R⁹;
R¹³ is C₁-C₄ alkyl, C₁-C₄ haloalkyl, C₁-C₄ alkoxy, C₁-C₄ haloalkoxy, C₂-C₆ alkoxyalkyl, C₂-C₄ cyanoalkyl, C₂-C₆ alkenyl, C₂-C₆ haloalkenyl or C₂-C₆ alkynyl; and
n is 1 or 2; and
wherein the dashed line in Q-3 indicates that the bond may be a carbon-carbon single bond or a carbon-carbon double bond.

2. A compound of Claim 1 wherein
Q is Q-1.

3. A compound of Claim 2 wherein
R¹ is C₁-C₄ alkyl or C₁-C₄ haloalkyl;
X is F, Cl, Br, OR⁸ or SR⁸;
R² is F or Cl;
R³ is halogen, OCH₃ or cyano;
R⁴ is OR¹¹, NR¹²SO₂R¹³ or CO₂R¹⁰;
each R⁸ is independenlty C₁-C₃ alkyl or C₁-C₂ haloalkyl;
each R¹⁰ is independently C₁-C₃ alkyl or C₁-C₃ haloalkyl;
R¹¹ is C₁-C₃ alkyl, C₃-C₄ alkenyl or C₃-C₄ alkynyl;
R¹² is H, CO₂R⁸ or C(=O)R⁸; and
R¹³ is C₁-C₃ alkyl or C₁-C₃ haloalkyl.

4. A compound of Claim 1 wherein
Q is Q-2.

5. A compound of Claim 4 wherein
R¹ is C₁-C₄ alkyl or C₁-C₄ haloalkyl;
X is F, Cl, Br, OR⁸ or SR⁸;
R² is F or Cl;
R⁵ is C₁-C₂ alkyl or C₃-C₄ alkynyl; and
each R⁸ is independently C₁-C₃ alkyl or C₁-C₂ haloalkyl.

6. A compound of Claim 1 wherein
Q is Q-3 or Q-4.

7. A compound of Claim 6 wherein
R¹ is C₁-C₄ alkyl or C₁-C₄ haloalkyl;
X is F, Cl, Br, OR⁸ or SR⁸;
R² is F or Cl;
R³ is halogen, OCH₃ or cyano;
W is O;
R⁶ is C₁-C₂ alkyl or C₁-C₂ haloalkyl;
R⁷ is H, C₁-C₃ alkyl, C₃-C₄ alkynyl, CO₂R⁸ or C(=O)R⁸;
each R⁸ is independently C₁-C₃ alkyl or C₁-C₂ haloalkyl; and
each R¹⁰ is independently C₁-C₂ alkyl.

8. The compound of Claim 1 which is selected from the group:
1-acetyl-7-chloro-4-(3-chloro-1,5-dihydro-1-methyl-5-oxo-4*H*-1,2,4-triazol-4-yl)-5-fluoro-1,3-dihydro-2,1-benzisothiazole 2,2-dioxide; and
1-chloro-*N*-[2-chloro-5-(3-chloro-1,5-dihydro-1-methyl-5-oxo-4*H*-1,2,4-triazol-4-yl)-4-fluorophenyl]methanesulfonamide.

9. A herbicidal composition comprising a herbicidally effective amount of a compound of Claim 1 and at least one of a surfactant, a solid diluent or a liquid diluent.

10. A method for controlling the growth of undesired vegetation comprising contacting the vegetation or its environment with a herbicidally effective amount of a compound of Claim 1.
